# EUROPEAN PATENT APPLICATION

(11) **EP 2 420 832 A1**
(43) Date of publication of application: **22.02.2012**
(21) Application number: 11075142.7
(22) Date of filing: 03.05.2007
(51) Int. Cl.: G01N 33/53

(54) **Cross-species and multi-species display systems**

(30) Priority: 04.05.2006 US 746489 P
(62) Divisional of application: 07756198.3
(71) Applicant: Abmaxis, Inc., Whitehouse Station NJ 08889-0100 (US)
(72) Inventor: Luo, Peter Peizhi, New Jersey 08889-0100 (US); Wang, Kevin Caili, New Jersey 08889-0100 (US); Zhong, Pingyu, New Jersey 08889-0100 (US)
(74) Representative: Horgan, James Michael Frederic

(57) **Abstract**

The present invention provides expression vectors and helper display vectors which can be used in various combinations as vector sets for multi-species and cross-species display of polypeptides on the outer surface of prokaryotic genetic packages and/or eukaryotic host cells. The multi-species and cross-species display systems can be practiced using the vector sets of the invention without having to change or reengineer the display vectors. The display systems of the invention are particularly useful for displaying a genetically diverse repertoire or library of polypeptides on the surface of phage, bacterial host cells, yeast cells, and mammalian cells.

## Description

### CROSS-REFERENCE TO RELATED APPLICATIONS

This application claims benefit under the provision of 35 USC 119(e) of US Provisional Application No. 60/746,489, filed May 4, 2006 entitled "Multi-species Display System." The disclosure of this provisional application is incorporated herein by reference in its entirety.

### TECHNICAL FIELD OF THE INVENTION

This invention relates to the field of protein display and provides display systems which allow for sequential multi-species display or cross-species display of protein libraries on the surface of prokaryotic and/or eukaryotic host cells without any molecular manipulations of the expression vectors.

### BACKGROUND OF THE INVENTION

Phage display systems are regarded as a core technology platform for the construction and screening of polypeptide libraries (e.g., antibody libraries). This is attributed to numerous practical considerations including, the availability of various genetic tools, the convenience of manipulation, and the high transformation efficiency of *.E coli* cells (typically, 10⁹ to 10¹⁰ cfu/ug pUC18 DNA). Today, naïve antibody libraries displayed on phage are routinely used for antibody discovery, thereby obviating the need for animal immunizations and the use of traditional hybridoma technology. The complexity of phage display librares have reached the size of 10¹¹. However, despite the successful use of phage display in antibody discovery and engineering, there are a number of drawbacks associated with the expression and display of eukaryotic proteins in prokaryotic systems. For example, some eukaryotic proteins can not be functionally expressed in prokaryotic cells, and prokaryotic host cells are not able to accomplish the full range of post-translational modifications that are characteristic of eukaryotic host cells.

Some of the limitations associated with the use of a prokaryotic display system can be overcome by the use of a eukaryotic display system. For example, eukaryotic host cells generally can accommodate the display of relatively large proteins, and are capable of post-translation modifications including complex glycosylation. A unique advantage associated with the use of a yeast display system include the fact that advantageous feature that yeast cells can be cultivated to high density in relatively simple and inexpensive culture medium. In addition, since eukaryotic cells are larger in size, libraries can be screened for single cells expressing proteins with desired properties by flow cytometry.

However, in practice the large scale use of eukaryotic host cells for protein display is limited by some fundamental issues such as the efficiency of host cell transformation. For example, the efficiency of yeast cell transformation is typically 10⁴-10⁵ cfu/ ug DNA , which imposes a significant limitation on the ability to display large size libraries in systems based on yeast display. Today, the largest of yeast display library reported is around size of 10⁹.

A multispecies display system that allows an investigator to exploit the strengths of prokaryotic and eukaryotic display systems for antibody discovery and molecular evolution applications would represent a significance advancement in the area of protein display. Prior efforts to develop multispecies or cross-species display systems are hindered by the fact that display strategies which are based on fusing a library of protein sequences of interest with a specific outer surface protein are limited to the single species of host cell from which the surface protein is derived. For example, a yeast display system that is premised on a display strategy in which the proteins of interest are displayed as fusion proteins which comprise a yeast cell wall protein, are by definition limited to yeast and cannot be practiced with phage and mammalian host cells.

As demonstrated in Hufton *et al* (US patent application 20030186374 A1) prior art display systems typically use vectors in which additional steps are required to transfer the coding sequences from prokaryotic (phage) display vectors to eukaryotic (yeast) display vectors. Generally speaking, in order to move from a first display system to a second display system using a different genetic package the vectors have to be modified for use in the second host cell (e.g., species) by digestion and ligation using a cloning strategy which was designed to use the same set of restriction sites to shuttle the expression cassette from a first vectors to a second vector. In practice, the process of having to perform DNA digestion and ligation in order to adapt a library of coding sequence for display in a second species is inefficient and introduces the possibility that potentially desirable members with unique properties may be lost during the process thereby having a negative impact on the diversity of the library.

Therefore, there is an unmet need for an ideal protein display system which can shuttle display vectors between alternative different species of prokaryotic (phage, or bacteria) genetic packages or between prokaryotic genetic packages and eukaryotic (yeast, mammalian cells) systems, or between two alternative eukaryotic host cells (yeast and mammalian cells), without any molecular manipulation of the display vector or library of display vectors.

### SUMMARY OF THE INVENTION

This invention provides protein display systems that is capable of both cross-species multi-species display of diverse libraries of polypeptides. The cross- and multi-species display methods can be practiced using the same expression vector without the need to perform any molecular manipulations (i.e., DNA digestions and ligations). The compositions and methods of the invention can be used to display the protein products encoded by a diverse repertoire of coding sequences on the surface of multiple species of genetic packages such as phage and bacterial cells, or phage and yeast cells, or phage and mammalian cells. The compositions and methods of the invention are particularly useful the display of collections of proteins in the context of discovery (i.e. screening) or molecular evolution protocols.

More specifically, the invention provides a protein display system which allows an investigator to shuttle expression/display vectors between both prokaryotic (e.g. phage, or bacteria) and eukaryotic (yeast, mammalian cells) host cells, or between different species of eukaryotic hot cells (e.g., yeast and mammalian cells), without any molecular manipulation of the display vector or library of display vectors.

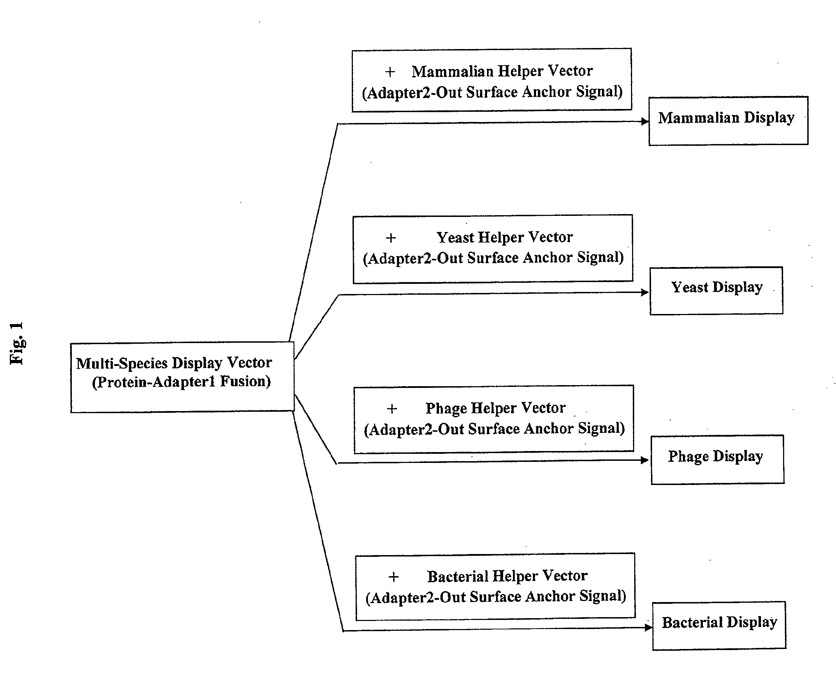

As depicted in Figure 1 the disclosed display systems allows for the display of exogenous sequence on the outer surface of various species of genetic packages or host cells. Each of the display systems generally has two components: 1) a multi-species or cross-species expression vector and 2) a corresponding helper vector for each species. In one embodiment the invention provides the cross- or multi-species expression vectors pMAG1, pMAG9, pMAT4, pMAT2, pMAT6 and PMAT5. In the pMAG1 and pMAG9 vectors, the expression of adapter 1 fusion protein is under control of a mammalian promoter and a bacterial promoter. In vectors pMAT2, pMAT4, pMAT5 and pMAT6 vectors, the expression of the adapter 1 fusion protein is under control of a yeast promoter and a bacterial promoter.

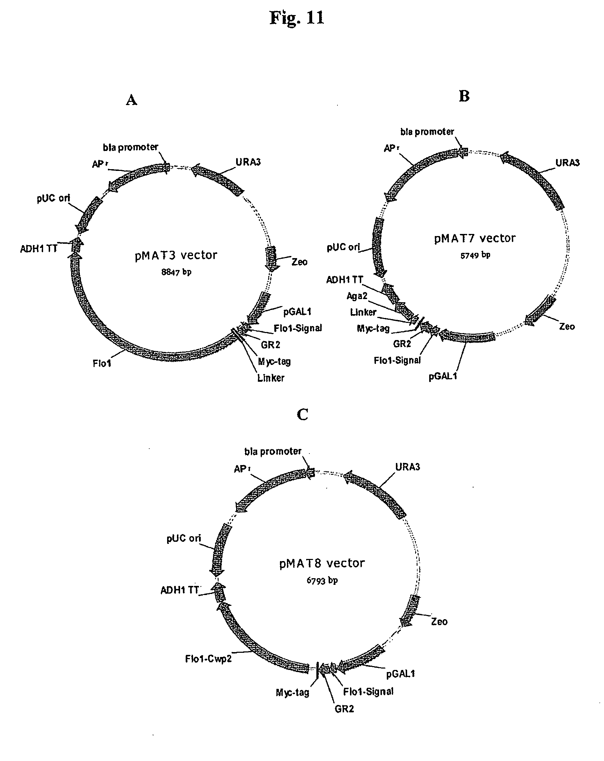

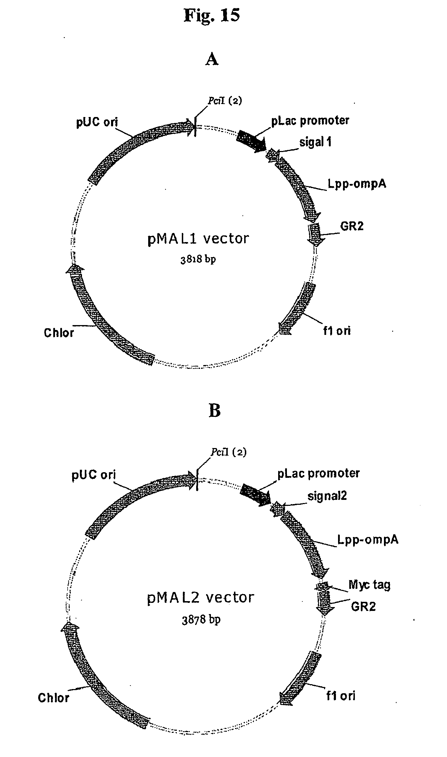

In an alternative embodiment the invention provides the helper display vectors GMCT, pMAG2, pMAL1, pMAL2, pMAT3, pMAT7 and pMAT8. The bacterial helper dispaly vector pMAL1 (see Figure 15A) and pMAL2 (see Figure 15 B) comprises an expression cassette which expresses a fusion protein comprising adapter 2 fused to the bacterial outer membrane domain of Lpp-OmpA. Each of the yeast helper display vectors directs the expression of a fusion protein comprising adapter 2 fused to different yeast cell outer wall proteins (see Figure 11). More specifically, pMAT 3 directs the expression of adapter 2 fused to the C-terminal domain of Flol, pMAT7 expresses adapter 2 Aga2 fusion proteins; and pMAT8 expresses adapater 2 Cwp2 fusion proteins.

Introduction of a cross- or multi-species expression vector alone into the corresponding host cells, such as *E. coli,* or yeast cells, or mammalian cells, leads to expression and secretion of exogenous polypeptides that are fused in-frame with a first adapter element, referred to herein as "adapter 1." Co-expression of an expression vector of the invention in combination with a corresponding helper vector of a particular display vector set directs the display of the exogenous polypeptide members of the protein library on the surface of the genetic package or host cell. Surface display results from the fact that the helper vector component of a particular vector set of comprises a second adapter element, referred to here in as "adapter 2, "which direct via pairwise interaction between the first and second adapters fused to an outer surface anchor protein that is functional the specific genetic package or host cells which is being used to display the library of protein sequences.

For example, coexpression of a helper vector comprising adapter 2 fused to a phage coat protein, in combination with a multi- or cross-species display vector which directs the expression of exogenous polypeptide-adapter 1 fusion proteins in E. *coli* cells results in a library of exogenous polypeptides displayed on phage particles. Similarly, coexpression of a bacterial helper vector comprising adapter 2 fused to bacterial out surface anchor protein in combination with a multi-species display vector which directs expression of exogenous polypeptide-adapter 1 fusion proteins results in a bacterial display library. Alternatively, the use a yeast helper vector comprising adapter 2 fused to a yeast outer surface anchor protein in combination with a multi-species display vector which directs expression of a library of polypeptide-adapter 1 fusion proteins will results in a yeast display library. Mammalian display libraries can be prepared by coexpressing a mammalian helper vector comprising adapter 2 fused to mammalian outer surface protein in combination with a multi-species display vector of the invention.

In one embodiment, this invention provides a cross-species display system that is suitable for shuttling a library of proteins of interest, between prokaryotic and eukaryotic display systems. More specifically, the invention provides across-species display system for the sequential display of a repertoire of polypeptide sequences of interest in a prokaryotic host and a eukaryotic host cell without having to manipulate the expression vectors encoding the polypeptide sequences of interest.

In general terms, each of the cross-species display vector sets of the invention comprise 1) a display vector comprising one or more promoters and a cross-species expression cassettes encoding proteins of interest fused in frame to a first adapter sequence, 2) a first helper vector encoding a fusion protein consisting of a second adapter sequence which interacts in a pair-wise manner with the first adapter sequence of the display vector fused to an outer surface protein expressed by the prokaryotic host, and 3) a second helper vector encoding a fusion protein consisting of the second adapter sequence fused to an outer surface protein expressed by the eukaryotic host cell. The disclosed bacterial/yeast cross-species display vector comprise the following functional elements: a yeast promoter and a bacterial promoter behind; a bacterial/yeast dual functional signal sequence; a gene of interest; adapter1 coding sequence; and yeast transcription termination sequences. The bacterial helper vectors comprise at least one copy of gene fusion encoding for adapter2 and bacterial out surface protein, Co-expression of adater1 and adter2 fusions in *E coli* cells will cause the cell surface display of protein of interest on E coli cells. Accordingly, co-expression of adater1 and adter2 fusions in yeast cells will cause the surface display of protein of interest on yeast cells.

A particular embodiment of this aspect of the invention provides a method for the display of a protein of interest, or a library of proteins, sequentially on phage viral particles (prokaryotic host cells) and either yeast or mammalian cells (eukaryotic host cells). Generally speaking, the phage/mammalian cross-species display vectors comprise the following functional elements: (1) a mammalian promoter and a bacterial promoter behind; (2) a bacterial/mammalian dual functional signal sequence; (3) a gene of interest; (4) adapter1 coding sequence; and (5) mammalian polyA sequences. The helper vectors for phage comprise all the phage genes necessary for assembly of viral particles, and at least one copy of gene fusion encoding for adapter2 and out surface protein, as described in US patent 7,175,983. The helper vectors for mammalian cells comprise expression cassettes to express the adapter2 fusion with mammalian surface anchor signal. Co-expression of adater1 and adter2 fusions in E coli cells will cause the surface display of protein of interest on phage particles. Accordingly, Co-expression of adater1 and adter2 fusions in mammalian cells will cause the surface display of protein of interest on mammalian cells.

One embodiment of the cross-species display systems of the invention provides a system for the sequential display of a library of protein sequences on the surface of *E coli* and mammalian cells. Using the disclosed phage/mammalian cross display vector plus bacterial helper vector described above, co-expression of adater1 and adter2 fusions in *E coli* cells will cause the surface display of protein of interest on bacterial cells. Accordingly, co-expression of adater1 and adter2 fusions in mammalian cells will cause the surface display of protein of interest on mammalian cells, by using phage/mammalian cross display vector plus mammalian helper vector.

In another embodiment, the present invention provides a method for cross species display on more than two species of recipient host cells. For example, the invention can be used to sequentially display a library of protein sequences on phage, yeast, or mammalian host cells in any combination or order. A phage/yeast/mammalian cross display vector comprises: (1) a mammalian promoter, a yeast promoter inside a intron sequence, and a short bacterial promoter after the intron; (2) a signal sequence that is functional in E. coli, yeast and mammalian cells; (3) a gene of interest; (4) adapter1 coding sequence; and (5) transcription termination sequences. The helper vectors for individual specie are described above. Accordingly, co-expression of adater1 and adter2 fusions in corresponding species will cause the surface display of protein of interest on phage, or yeast cell, or mammalian cells, by using phage/yeast/mammalian cross display vector plus corresponding helper vector for each species.

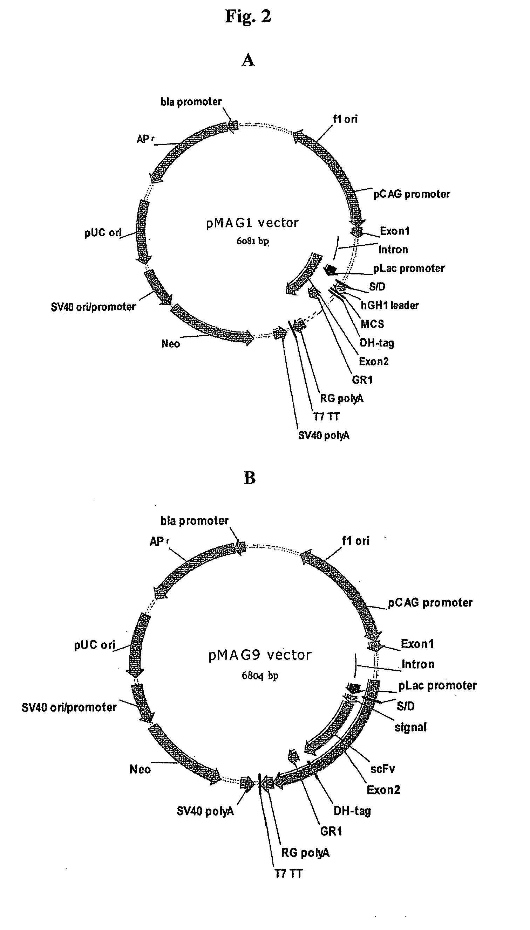

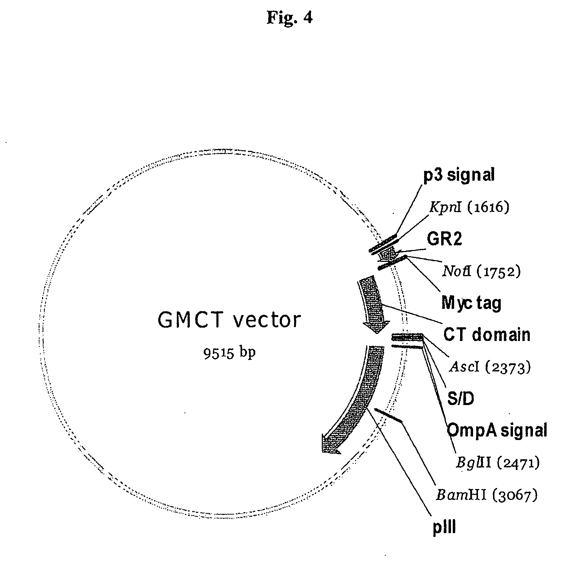

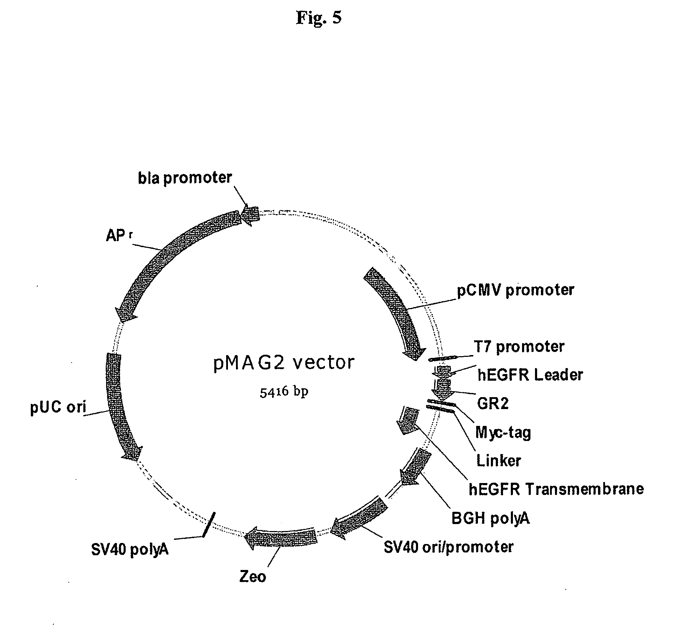

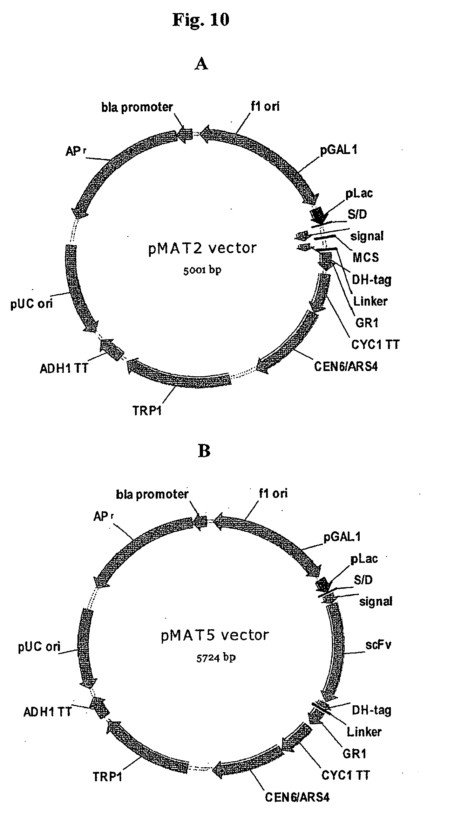

The invention also provides kits comprising display vector sets of the invention, including particular sets of cross-species and multi-species expression vectors in combination with helper display vectors which are suitable for directing the surface display of proteins in various prokaryotic and eukaryotic host cells. For example, a cross-species display kit suitable for use to direct the sequential display of a protein expression library on the surface of phage and mammalian host cells could comprise the vector set pMAG9 (Fig 2) and GMCT (Fig 4) which facilitates phage display, in combination with the mammalian helper display vector pMAG2 ( Fig 5) which directs cell surface display in mammalian cells in suitable packaging. Alternatively, a multi-species display kit suitable for use to direct the sequential display of a protein expression library on yeast and mammalian cells could comprise a vector set including the vectors pMAG9 (Fig 2)and pMAG2 (Fig 5) for mammalian display, and vectors pMAT5 (Fig 10) and pMAT3 (Fig 11) for yeast display in suitable packaging.

An alternative embodiment of the invention includes host cells comprising the helper display vectors of the invention. Suitable prokaryotic hosts include E. *coli* cells or phage. Suitable eukaryotic hosts are yeast cells or mammalian cells. The preferable prokaryotic and eukaryotic hosts are filamentous phage virus and yeast *S.cerevisiae* cells.

The phage/yeast cross-species display vector sets of the invention generally comprise the following functional elements: (1) a yeast promoter and a bacterial promoter behind; (2) a bacterial/yeast dual functional signal sequence; (3) a gene of interest; (4) adapter1 coding sequence; and (5) yeast transcription termination sequence. The helper vectors for phage comprise all the phage genes necessary for assembly of viral particles, and at least one copy of gene fusion encoding for adapter2 and out surface protein, as described in US patent 7,175,983. The helper vectors for yeast S. *cerevisiae* comprise expression cassettes which direct the expression of a fusion protein comprising adapter2 fused with a yeast outer wall proteins. Co-expression of adater1 and adapter2 fusions in *E coli* cells will cause the surface display of protein of interest on phage particles. Accordingly, co-expression of adapter1 and adapter 2 fusions in yeast cells will cause the surface display of protein of interest on yeast cells.

The invention also provides a versatile method for expressing soluble proteins in alternative species of host cells. The use of the expression vectors of the invention in the absence of a helper vector which comprises a fusion protein comprising an outer surface anchor protein in combination with an adapter sequence which is capable of interacting in a pairwise maner with a compatible adapter sequence fused to the protein product of the cross-species expression cassette results in the soluble expression of proteins in multiple host cells including *E. coli,* yeast, and mammalian cells. Using the multi- and cross-species expression/display vectors of the invention proteins of interest can be produced sequentially in different host cells without having to perform any molecular manipulation of the vector.

In an alternative embodiment, this invention provides a multi-species display method which can be used to display proteins of interest, or a library of diverse proteins, on two different species of prokaryotice or eukaryotic host cells. Suitable prokaryotic cells include gram-negative bacteria cells. Suitable eukaryotic host include yeast cells such as, but not limited to *S.cerevisiae* cells or mammalian cells.

In a particular multi-species embodiment, the invention provides a method for the sequential cell surface display of a library of protein sequences of interest on yeast and mammalian cells. The yeast/mammalian multi-species display vector of the invention comprises the following functional elements: (1) a mammalian promoter and a yeast promoter inside a intron sequences; (2) a yeast/mammalian dual functional signal sequence; (3) a gene of interest; (4) adapter1 coding sequence; and (5) transcription termination sequences for yeast and mammalian. The helper vectors for yeast or mammalian comprise expression cassettes to express the adapter2 fusion with cell surface anchor signal of yeast or mammalian cells described above. Accordingly, co-expression of adater1 and adter2 fusions in corresponding species will cause the surface display of protein of interest on yeast cell, or mammalian cells.

Additionally, this invention provides methods which can be used for the isolation of proteins of interest characterized by desired protein specificities from a library of proteins of interest. A preferred method is to initially identify proteins with desired properties from libraries of protein sequences displayed on phage and to subsequently reevaluate the lead proteins using a yeast or mammalian display system. For example, in order to engineer a human protein, a large size of library (diversity > 10e9) will be made on phage/yeast/mammalian cross display vector. Taking the unique advantages of phage display technology, this large size of library can be made first on phage display format with phage helper vector, and a few round of library panning will be carried out. Thus, the DNAs of leads library from phage panning (diversity < 10e6) will be directly tranformed into yeast cells with yeast helper vector to make a small size of yeast display library. The leads isolated from FACS sorting of yeast display library will have desired eukaryotic properties such as folding and glycosylation, which will provide great benefits on downstream process such as function and production. Furthermore, if necessary, the leads isolated from yeast display can be directly displayed on mammalian cell surface for functional assay.

The invention also provides methods for the isolation of proteins characterized by a desired binding specificity from a yeast/mammalian cross-display library, if the size of library is appropriate for the construction of a yeast display library. Using a yeast helper display vector of the invention a library comprising proteins of interest can be initially displayed on yeast. Subsequently, the leads DNAs isolated from a yeast library selection assay can be directly transfected into mammalian cells for a second round of cell surface display and selection. The above-described cross-species (yeast to mammalian cells) can be performed without having to perform any molecular manipulation of the expression/display vector. The cross-species display capabilities of the invention allows an investigator to conduct a screening assay which simultaneously provides a functional confirmation of the leads identified in the first display system.

In another embodiment, this invention provides a method for isolation of signal sequences with desired properties such as functions of cross multiple species. A library of signal sequences can be constructed using corresponding cross-species display vectors. The signal sequences with functions in corresponding species can be isolated from shuttling selections of display library in corresponding species.

### BRIEF DESCRIPTION OF THE DRAWINGS

Figure 1 is a schematic representation of the experimental design of the subject cross-species and multi-species display systems of the invention.
Figures 2A and 2B provide a schematic representations of phage and mammalian cross- or multi-species display vectors pMAG1(Figure 2A) and pMAG9 (Figure 2B). In pMAG1 and pMAG9 vectors, the expression of adapter1 fusion protein is under control of a mammalian promoter and a bacterial promoter.
Figure 3 provides a graphic representation of the results of anti- phage ELISA screen for kanamycin-resistant phage-positive clones of K07Kpn vector. Clone # C2, B3, B7, B9, A12 represent K07kpn helper phage-positive clones. F1 and F2 represent two positive controls of parent M13KO7 phages.
Figure 4 provides a schematic representation of the phage helper vector GMCT. The vector contains nucleotide sequence encoding the additional copy of engineered gene III fused to adapter GR2 and Myc-tag in KO7kpn phage vector.
Figure 5 provides a schematic representation of the mammalian helper vector pMAG2, which produces a fusion protein comprising adapte 2 fused to the transmembrane domain of human EGF receptor.
Figures 6A and 6B provide a graphic representation of the results of a functional expression assay for soluble anti-VEGF scFv expressed in E. *coli* cells (Figure 6A) and in mammalian cells (Figure 6B) using cross-species display vector pMAG9. Those data showed the VEGF binding activity of scFv antibody from the culture supernatants, and demonstrated the cross-species expression function of the vector pMAG9.
Figures 7A and 7B provides results illustrating the expression of soluble scFv in mammalian cells using cross-species display vector pMAG9 and pMAT6. Figure 7A provides western blot results developed with anti-HA antibody showing a 35 KD scFv protein secreted in the culture supernatants from 293 and COS cells by pMAG9 and pMAT6 vectors. Figure 7B summarizes the result of an ELISA assay showing the VEGF binding activity of scFv expressed in COS cell supernatant by pMAT6 vector, and demonstrated the multi-species expression function of the vector pMAT6.
Figure 8 shows the functional display of scFv on phage surface using vector pMAG9. The data showed a dose-dependent VEGF binding activity of anti-VEGF scFv antibody displayed on phage surface.
Figures 9A, B and C show the fluorescence microscopic images of COS 6 cells displaying anti-VEGF scFv on surface. COS 6 cells transfected with pMAG9 and pMAG2 vectors were grown on chamber slides. Transfected cells were stained with anti-HA antibody-Alexa Fluor-488 (green) and DAPI (blue) nuclear staining. Figure 9A is the merged staining patterns. Figure 9B provides the Alex Fluor-488 staining, and Figure 9C provides DAPI nuclear staining. The data demonstrated the mammalian display function of pMAG9 vector.
Figures 10A and 10B provide schematic representations of phage and yeast cross-species or multi-species expression vectors pMAT2 and pMAT 5. Figure 10 illustrates the components of vector pMAT2. Figure 10B illustrates the components of vector pMAT5. In pMAT2 and pMAT5 vectors, the expression of adapter1 fusion protein is under control of a yeast promoter and a bacterial promoter.
Figure 11A, B and C provide schematic representations of yeast helper display vectors pMAT3, pMAT7, and pMAT8. In these vectors, adapter2 is fused to different yeast cell outer wall proteins as follows: C-terminal domain of Flo 1 for pMAT3; Aga2 for pMAT7; and Cwp2 for pMAT8.
Figure 12 shows the results of a functional expression assay for of soluble scFv in E. *coli cells* using expression vector pMAT5. The ELISA data showed a dose-dependent VEGF binding activity of anti-VEGF scFv antibody in TG1 culture supernatants.
Figure 13 shows the functional display of scFv on phage surface using cross-species display vector pMAT5.. The data showed a dose-dependent VEGF binding activity of anti-VEGF scFv antibody displayed on pMAT5 phage surface.
Figures 14 A and B provide schematic representations of yeast and mammalian cross-species or multi-species expression vectors pMAT4 (14A) and pMAT6 (14B). In pMAT4 and pMAT6 vectors, the expression of adapter1 fusion protein is under control of three promoters: mammalian, yeast and bacterial promoter. The signal peptide is functional in yeast and mammalian cells.
Figures 15A and B provide schematic representations of the bacterial helper vector pMAL1 (15A) and pMAL2 (15 B). The vectors contain a chloramphenicol-resistance gene for antibiotic selection (Cam), and an expression cassette for adapter fusion to bacterial out membrane domain of Lpp-OmpA.

### DETAILED DESCRIPTION OF THE INVENTION

As used in this specification and claims, the singular form "a," "an," and "the" include plural references unless the context clearly dictates otherwise. As used herein the term "species" refers to a group of organisms which are very similar in morphology, anatomy, physiology and genetics due to having relatively recent common ancestors. Different species usually demonstrate common features in performing common function of life regardless their other differences. For example, human and mouse cells share certain molecular landmarks, and are considered to be members of the same species (i.e., mammalian cells) while human cells and yeast cells are different species of eukaryotic host cells.

As used herein the term "genetic packages" refers to viruses or cells, in which polynucleotide sequences encoding proteins of interest are packaged for expression and/or surface display.

As used herein the term "multispecies display" refers to a display strategy which allows an investigator to express a diverse repertoire of polynucleotide sequences encoding a library of polypeptide sequences on the surface of different types of prokaryotic genetic packages (i.e, phages or bacteria) or different types of eukaryotic host cells (i.e. yeast or mammalian cells). For example a multispecies display strategy enables the display of an antibody on the surface of yeast and mammalian cells.

As used herein the term "cross-species display" refers to a display strategy which allows an investigator to display a diverse repertoire of polynucleotide sequences encoding a library of polypeptide sequences sequentially on the surface of prokaryotic genetic packages and eukaryotic host cells. For example, a cross-species display strategy enables the display an antibody library on phage and subsequently on yeast.

The terms "prokaryotic system" and "prokaryotic genetic packages" are used interchangeably herein to refer to prokaryotic cells such as bacterial cells or prokaryotic viruses such as phages or bacterial spores.

The term "eukaryotic system" and "eukaryotic host cells" are used interchangeably herein to refer to eukaryotic cells including cells of animal, plants, fungi and protists, and eukaryotic viruses such as retrovirus, adenovirus, beculovirus.

As used herein the term "gene," is used to refer to a DNA sequence which codes for a protein. The term does not include untranslated flanking regions such as RNA transcription initiation signals, polyadenylation addition sites, promoters or enhancers.

The term "expression cassette" is used here to refer to a functional unit that is built in a vector for the purpose of expressing recombinant proteins/peptides. It usually consists of a promoter or promoters, a ribosome binding site or ribosome binding sites, and the cDNA of the expression target. Other accessory components can be added to construct an expression cassette.

As used herein the term "vector" refers to a nucleic acid molecule, preferably self-replicating, which transfers an inserted nucleic acid molecule into and/or between host cells. Typically vectors are circular DNA comprising a replication origin, a selection marker, and or viral package signal, and other regulatory elements. Vector, vector DNA, plasmid DNA are interchangeable terms in description of this invention. The term includes vectors that function primarily for insertion of DNA or RNA into a cell, replication of vectors that function primarily for the replication of DNA or RNA, and expression vectors that function for transcription and/or translation of the DNA or RNA. Also included are vectors that provide more than one of the above functions.

As used herein the term "expression vector" is a polynucleotide which, when introduced into an appropriate host cell, can be transcribed and translated into a polypeptide(s).

The terms "expression vector," multi-species expression vector" and "cross species expression vector" refer to vectors that direct the soluble expression of proteins of interest fused in frame with an adapter sequence which is characterized by an ability to associate in a pairwise fashion with an adapter sequence produced by a helper vector of the invention.

The term "helper vector" refers to a genetic package, or host cell-specific vector designed to produce fusion proteins comprising an anchor protein fused in frame with an adapter sequence which is characterized by an ability to associate in a pairwise fashion with an adapter sequence produced by an expression vector of the invention. Helper vectors can be introduced into recipient host cells, in combination with an expression vector, transiently by co-transformation, or permanently by integration into host genome.

As used herein the term "multi-species display vector set" refers to particular combinations of expression vectors and helper vectors which are designed to comprise complementary adapter sequences which function to display polypeptides on the surface of particular species of genetic packages or host cells. For example, a set of vectors pMAG9 (Fig 2) and pMAG2 ( Fig 5) for mammalian display, a set of vectors pMAT5 (Fig 10) and pMAT3 (Fig 11) for yeast display.

As used herein the term "cross-species display vector set" refers to particular combinations of expression vectors and helper vectors which are designed to comprise complementary adapter sequences which function to allow the sequential display of polypeptides in both a prokaryotic eukaryotic systems. For example, the vector set pMAG9 (Fig 2) and GMCT (Fig 4) facilitates phage display, while the use of pMAG9 in combination with and pMAG2 (Fig 5) directs cell surface display in mammalian cells.

As used herein the term "expression system" usually connotes a suitable host cell comprised of an expression vector that can function to yield a desired expression product.

As used herein, the term "surface antigen" refers to the plasma membrane components of a cell. It encompasses integral and peripheral membrane proteins, glycoproteins, polysaccharides and lipids that constitute the plasma membrane. An "integral membrane protein" is a transmembrane protein that extends across the lipid bilayer of the plasma membrane of a cell. A typical integral membrane protein consists of at least one "membrane spanning segment" that generally comprises hydrophobic amino acid residues. Peripheral membrane proteins do not extend into the hydrophobic interior of the lipid bilayer and they are bound to the membrane surface by noncovalent interaction with other membrane proteins.

The term "outer surface anchor" as used herein is to refer a polypeptide, or protein, or protein domain, which will be integrated into or attached on the out surface of a genetic package. It may be from the nature, or be artificially created by any means. The term as used interchangeably with the terms "surface anchor sequence" or "signal coat protein", "outer surface sequences", "outer membrane protein", "membrane anchor protein", "anchor protein", "cell wall protein", "GPI anchor signal," "GPI attachment signal," and signal anchor sequence.

The term "Signal sequence" and "leader sequence" are used interchangeably herein to refer a DNA sequence encoding a secretory peptide that is a component of a larger peptide on DNA level. It may also refer the amino acide sequence of a secretory peptide. The function of secretory peptide is to direct the larger polypeptide through a secretory pathway of a cell.

As used herein the terms "polynucleotides", "nucleic acids", "nucleotides" and "oligonucleotides" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non nucleotide components. A polynucleotide may be further modified after polymerization, such as by conjugation with a labeling component.

As used herein the term "amino acid" refers to either natural and/or unnatural or synthetic amino acids, including glycine and both the D or L optical isomers, and amino acid analogs and peptidomimetics.

As used herein the terms "polypeptide", "peptide, "protein," and "protein of interest" are used interchangeably herein to refer to polymers of amino acids of any length. The polymer may be linear, cyclic, or branched, it may comprise modified amino acids, and it may be interrupted by non amino acids. The terms also encompass amino acid polymers that have been modified, for example, via sulfation, glycosylation, lipidation, acetylation, phosphorylation, iodination, methylation, oxidation, proteolytic processing, phosphorylation, prenylation, racemization, selenoylation, transfer-RNA mediated addition of amino acids to proteins such as arginylation, ubiquitination, or any other manipulation, such as conjugation with a labeling component.

As used herein the term "antibody" refers to immunoglobulin molecules and immunologically active portions of immunoglobulin molecules, i.e., molecules that contain an antigen-binding site which specifically binds ("immunoreacts with") an antigen. Structurally, the simplest naturally occurring antibody (e.g., IgG) comprises four polypeptide chains, two heavy (H) chains and two light (L) chains inter-connected by disulfide bonds. The immunoglobulins represent a large family of molecules that include several types of molecules, such as IgD, IgG, IgA, IgM and IgE. The term "immunoglobulin molecule" includes, for example, hybrid antibodies, chimeric antibodies, humanized antibodies and fragments thereof. Non-limiting examples of antibody fragments include a Fab fragment consisting of the VL, VH, CL and CH1 domains; (4) an Fd fragment consisting of the VH and CH1 domains; (5) an Fv fragment consisting of the VL and VH domains of a single arm of an antibody; (6) an F(ab')2 fragment, a bivalent fragment comprising two Fab fragments linked by a disulfide bridge at the hinge region; (7) a diabody consisting of two identical single chain Fv with shorter linker; (8) a ccFv antibody consisting of Fv stabilized by a pair of coiled-coil domains interaction..

As used herein the term "pairwise interaction" means that the two adapters can interact with and bind to each other to form a stable complex. The stable complex must be sufficiently long-lasting to permit packaging the polypeptide onto the outer surface of a genetic package. The complex or dimer must be able to withstand whatever conditions exist or are introduced between the moment of formation and the moment of detecting the displayed polypeptide, these conditions being a function of the assay or reaction which is being performed.

As used herein the term "host cell" includes an individual cell or cell culture which can be or has been a recipient for the subject vectors. Host cells include progeny of a single host cell. The progeny may not necessarily be completely identical (in morphology or in genomic of total DNA complement) to the original parent cell due to natural, accidental, or deliberate mutation. A host cell includes cells transfected in vivo with a vector of this invention.

As used herein the term "repertoire" refers to the total collection of variant members of a functional or physical origin. A library is the total collection of homologous variant members. In general, a repertoire depicts much wider and larger functional and physical landscape, therefore, it can include libraries that are functionally defined. For example, the entire genetic capacity of immunoglobulin in a species is its immunoglobulin repertoire; for the purpose of protein engineering, a library usually refers to a collection of variant molecules that derived from one or defined number of ancestors. A repertoire of certain practical purpose, such as generation of a therapeutic antibody, includes all libraries generated for such a purpose.

As used herein the term "adapters" refer to complementary elements or components that are capable of a pair-wise interaction with each other to form a physical unity based on the physical and/or functional match between the two different interacting adapter. Adapters can be proteins, protein domains, peptides, compounds of non-polypeptide, etc. derived from natural or artificial origins. Typical examples for adapters include two interacting polypeptides that form a coiled-coil heterodimer such as GR1 and GR2 (depicted in SEQ #.19 and 20); c-fos and c-jun; natural and artificial leucine zippers, and etc; specific protein-protein interaction derived from specific binding between ligand and its cognate receptor; heterodimeric complex of two different proteins to form a functional unit, and etc; Specific binding from two different non-polypeptide components such as biotin and strepavidin, etc. Adapters used in surface display described in this invention can be endogenous and/or exogenous to the host species, and/or artificially derived.

As used herein, a linear sequence of peptide is "essentially identical" to another linear sequence, if both sequences exhibit substantial amino acid or nucleotide sequence homology. Generally, essentially identical sequences are at least about 60% identical with each other, after alignment of the homologous regions. Preferably, the sequences are at least about 70% identical; more preferably, they are at least about 80% identical; more preferably, they are at least about 90% identical; more preferably, the sequences are at least about 95% identical; still more preferably, the sequences are 100% identical.

### Prior Art Prokaryotic Display Systems

### Phage display

The display of polypeptides on the surface of genetic packages represents a powerful methodology for screening libraries of polypeptide sequences. The ability to construct libraries of enormous molecular diversity and to select for molecules with desired properties has made this technology broadly applicable to numerous applications, including screening/discovery protocols as well as molecular evolution protocols. The origins of phage display date to the mid-1980s when George Smith first expressed an exogenous segment of a protein on the surface of bacteriophage M13 virus particles by fusing the exogenous sequence to a phage coat protein (Science (1985) 228: 1315 1317). Since then, a range of display systems have been developed based on George Smith's findings. These systems can be broadly classified into two categories (U.S. Pat. Nos. 5,969,108 and 5,837,500). The first generation system is a one-vector system. The vector in this system contains the entire phage genome, insert therein an exogenous sequence in-frame with a coat protein gene. Because the resulting phage particles carry the entire phage genomes, they are relatively unstable and less infectious. The second generation system, commonly referred to as the phagemid system, has two components: (1) a phagemid vector carrying the exogenous sequence fused to phage coat protein, and a phage-derived origin of replication to allow packaging of the phagemid into a phage particle; and (2) a helper phage carrying all other sequences required for phage packaging.

The helper phage is typically replication-defective such as M13KO7 helper phage manufactured by Amersham Pharmacia Biotech and its derivative VCSM13 that is produced by Stratagen. Upon superinfection of a bacterial cell with the helper phages, newly packaged phages carrying the phagemid vector and displaying the exogenous sequence are produced. As such, the prior phagemid system requires fusion of the exogenous sequence to at least part of a phage outer-surface sequence (i.e. the coat sequence). The fusion or display sites most commonly used are within genes III and VIII of M13 bacteriophage, although genes VI, VII and IX fusions have been reported.

Alternative to the coat protein fusion system, various modifications to the fusion phagemid system have been described. Crameri et al. devised a system to display cDNA products, in which Fos oncogene was inserted adjacent to the exogenous sequence to be displayed on a phagemid vector, and Jun oncogene was inserted adjacent to gene III on the same vector (see Crameri et al. (1993) Gene 137:69 75). The Crameri approach exploits the preferential interaction between fos and jun proteins: as the Fos-exogenous polypeptide is expressed and secreted into the periplasmic space, it forms a complex with pIII-Jun which is then packaged into the phage particles upon super infection with M13KO7 helper phage.

Another variant similar to the Crameri system is the "cysteine-coupled" display system described in WO 01/05950, US patent 6753136. The attachment and display of the exogenous polypeptide are mediated by the formation of disulfide bond between two cysteine residues in the bacterial periplamic space, one of which is contained in the exogenous sequence, and the other is inserted in the outer-surface sequence. Although those two systems avoid the expression of a fusion comprising the exogenous protein linked to an outer-surface protein, the systems fails to minimize the toxicity of coat proteins to the host cells because of the constitutive expression of the coat protein pIII in display vectors. In addition, the formation of disulfide bond between two cysteine residues require high level expression of both of exogenous sequence and coat protein pIII. Therefore, any lower expression member will lose the chance to display.

Recently, Wang et al described an alternative phage display system based on an adapter-directed display system (US Patent 7,175,983); which comprise: (a) an expression vector comprising a coding sequence that encodes the exogenous polypeptide fused in-frame to a first adapter sequence; (b) a helper vector comprising outer-surface sequences encoding outer-surface proteins necessary for packaging the phage particle, and one of the outer-surface proteins is fused in-frame to a second adapter. Therefore, displays of the exogenous polypeptides are achieved by pairwise interaction between the first and second adapters.

### E. coli Display

Display of polypeptides on the surface of E. coli was developed as an alternative to phage display technology. Similar to phage display, bacterial display is an attractive method due to the availability of various genetic tools and mutant strains, and its high transformation efficiency that makes it ideal for large size library construction and screening. In gram-negative bacteria, surface display systems based on fusion of protein to be display to various anchoring proteins have been reported, in which outer membrane proteins (Chang and Lo 2000, J Biotechnol 78:115―122; Lee et al. 2004, Appl Environ Microbiol 70:5074―5080), pili and flagella (Westerlund-Wikström et al. 1997, Protein Eng 10:1319-1326), modified lipoproteins (Georgiou et al. 1996, Protein Eng 9: 239―247), ice nucleation proteins (Jung et al. 1998, Nat Biotechnol 16:576-580), and autotransporters (Veiga et al. 2003, J Bacteriol 185:5585―5590) were used as the anchors for display.

### Prior Art Eukaryotic Display SystemsYeast display

The display of heterologous protein on the cell wall of the eukaryotic host cell *Saccharomyces cerevisiae* was first described in 1993 by fusion of alpha-galactosidase to C-terminal half of cell well protein alpha-agglutinin AGA1 (Schreuder MP et al, Yeast 9:399-409). Since then, various yeast display systems base on fusion of the protein of interest to various cell well proteins were reported (Kondo M et al, review). Almost all of the cell-surface display systems developed for yeast are glycosyl phosphatidylinositol (GPI) anchor-dependent. More than a dozen of yeast cell well proteins with a putative GPI attachment signal at the C-termini have been proven capable of displaying peptides and proteins, which includes a-agglutinin (Agal and Aga2), Cwp1, Cwp2, Gas1p, Yap3p, Flolp, Crh2p, Pir1, Pir2, Pir4, and Icwp in S.cerevisiae; HpSED1, HpGAS1, HpT1P1, HPWP1 in Hansenula polymorpha, and Hwp1p, Als3p, Rbt5p in Candida albicans. To date, over twenty heterologous proteins have been successfully displayed on yeast cell surface.

Among all of the cell-surface display systems described above, the system created by Dane Wittrup base on a-agglutinin receptor has been widely used for display various peptides and proteins such as scFv antibody and antibody libraries (US patent 6300065, 6423538, 6696251, and 6699658). In *S. cerevisiae,* the a-agglutinin receptor acts as an adhesion molecule to stabilize cell-cell interactions and facilitate fusion between mating "a" and α haploid yeast cells. The receptor consists of a core subunit Aga1 and small subunit Aga2. Aga1 is secreted from the cell and becomes covalently attached to □-linked glucans in the extra cellular matrix of the yeast cell wall though its GPI anchor-attachment signal. Aga2 binds to Aga1 through two disulfide bonds, presumably in the golgi, and after secretion remains attached to the cell via Agal. This yeast display system takes advantage of the association of Agal and Aga2 proteins to display a recombinant protein on the yeast cell surface through fusion of protein with the Aga2 subunit.

The Wittrup system has been adapted for multi-chain polypeptides such as immunoglobulin Fab fragments (Hufton et al, US patent application 20030186374 A1). Hufton *et al* mention the possible use of the Fos/Jun interaction as the basis of a display system suitable for use in eukaryotic cells. However, Hufton et al did not provide an enabling description of how the Fos/Jun interaction can be utilized to direct protein display in eukaryotic host cells. However the reference only teaches how to use Ag2 fusion developed by Dane Wittrup (US patent 6300065,6423538,6696251, and 6699658) for yeast display of Fab antibody, and how to transfer gene from phage display vector to yeast vector by molecular cloning.

### Mammalian Display

A number of approaches have been used to achieve display of proteins on the surface of mammalian cells by fusion to various membrane anchor proteins, which includes membrane domains of cell surface receptors (Chesnut et al, 1996, J Immunological Methods; Ho et al, 2006, PNAS, 103:9637-9642), GPI anchor sequences (US patent 6838446), non-cleavable type II signal anchor sequences (US patent 7125973). A typical example is the pDisplay vector, which is a commercially available vector to display protein on mammalian cell surface provided by Invitrogen Corp. In this vector, the protein of interest will be fused with a membrane domain of cell surface receptor PDGFR. An alternative approach was also reported in US patent 6919183. In this system, a cell surface capture molecule such as protein G, protein A was used to capture the antibody molecules on mammalian cell surface.

### Multi-species and Cross-species Display Systems of the Invention

Phage display is the most commonly used systems for a variety of applications such as receptor ligand selections, antibody engineering, and protein epitop identification. Due to the convenience and efficiency of phage genetic manipulation, a large diverse library of size 10⁹ can be achieved in a single phage library. However, phage system can only be used in situations in which post-translational modifications and other intracellular processing are not necessary or desired. For most eukaryotic proteins such as antibody fragments, their biological functions are associated with the intracellular processing such as glycosylations. Moreover, some eukaryotic proteins can not be functionally folded in prokaryotic cells.

The limitations of prokaryotic system can be overcome by display of foreign proteins on the surface of eukaryotic cells. It allows the folding and glycosylation of expressed eukaryotic proteins, allows display of relatively large proteins. However the poor efficiency of eukaryotic cells transformation has limited the practice to a very small size of library, which make it impossible to match the scale that phage display can provide.

Thus development of a display system that combines the power of both prokaryotic and eukaryotic display technology for various applications is the ultimate challenge. When the protein of interest is fused with a specific out surface sequence, this protein can be only displayed on surface of the corresponding species. To transfer from phage display to yeast display, Hufton et al ( US patent application 20030186374 A1) described a method to transfer of genes of interest from prokaryotic display vectors (phage pIII coat protein fusion vector) to eukaryotic display vectors (yast Aga2 fusion vector) through digestion and ligation.

This invention provides a new display system that is capable or multi-species display. More specifically, using the same display vector, without any molecular manipulations such as DNA digestions and cloning, a protein of interest can be displayed on the surface of multi-species such as phage, and bacterial cells, or /and yeast cells, or/and mammalian cells. Moreover this display system provides a function of cross-species display methods. For example, using the disclosed vector sets a protein of interest can be displayed in prokaryotic system, such as phage or bacterial cell, and subsequently the protein, or proteins of interest in the case of a recombinant library of proteins, can also be displayed on the surface of eukaryotic cells such as yeast or mammalian cells without any molecular manipulations of the vector(s). Each of the display systems of the invention utilizes a particular set of display vectors. The different vector sets of the invention comprise a multi-species expression vector, encoding a library of polypeptide sequences fused to a first adapter (i.e. adapter 1), in combination with a helper vectors that is specific for particular genetic packages or host cell. Each of the helper vectors comprise a cell surface anchor protein fused to a second adapter (i.e., adapter 2). As shown herein, the coexpression of a multi-species display vector in combination with a helper vector which comprises a corresponding adapter produces a collection of genetic packages (or host cells) which has a repertoire of polypeptide sequences displayed on its surface via the pairwise interaction of the adapters (i.e. adapter 1 and adapter 2).

### Components of the Vectors

### Adapters

Adapter sequences applicable for constructing the display and helper vectors of the subject display system can be derived from a variety of sources. Generally, any protein sequences involved in the formation of stable multimers are candidate adapter sequences. As such, these sequences may be derived from any homomultimeric or heteromultimeric protein complexes. Representative homomultimeric proteins are homodimeric receptors (e.g. platelet-derived growth factor homodimer BB (PDGF), homodimeric transcription factors (e.g. Max homodimer, NF-kappaB p65 (Re1A) homodimer), and growth factors (e.g. neurotrophin homodimers). Non-limiting examples of heteromultimeric proteins are complexes of protein kinases and SH2-domain-containing proteins (Cantley et al. (1993) Cell 72: 767 778; Cantley et al. (1995) J. Biol. Chem. 270(44): 26029 26032), heterodimeric transcription factors, and heterodimeric receptors.

A vast number of heterodimeric receptors are known, including but not limited to receptors that bind to growth factors (e.g. heregulin), neurotransmitters (e.g..gamma.-Aminobutyric acid), and other organic or inorganic small molecules (e.g. mineralocorticoid, glucocorticoid). Preferred heterodimeric receptors are nuclear hormone receptors (Belshaw et al (1996) Proc. Natl. Acad. Sci. U.S.A 93(10):4604 4607), erbB3 and erbB2 receptor complex, and G-protein-coupled receptors including but not limited to opioid (Gomes et al. (2000) J. Neuroscience 20(22): RC110); Jordan et al. (1999) Nature 399:697 700), muscarinic, dopamine, serotonin, adenosine/dopamine, and GABA.sub.B families of receptors. For majority of the known heterodimeric receptors, their C-terminal sequences are found to mediate heterodimer formation.

GABA_{B}-R1/GABA-R2 receptors exhibit the above-mentioned physical properties. These two receptors are essentially incapable of forming homodimers under physiological conditions (e.g. in vivo) and at physiological body temperatures. Research by Kuner et al. and White et al. (Science (1999) 283: 74 77); Nature (1998) 396: 679 682)) has demonstrated the heterodimerization specificity of GABA_{B} R1 and GABA_{B} R2 C in vivo. In fact, White et al. were able to clone GABA.sub.B-R2 from yeast cells based on the exclusive specificity of this heterodimeric receptor pair. In vitro studies by Kammerer et al. (Biochemistry, 1999, 38: 13263-13269) has shown that neither GABA_{B} -R1 nor GABA_{B}-R2 C-terminal sequence is capable of forming homodimers in physiological buffer conditions when assayed at physiological body temperatures. Specifically, Kammerer et al. have demonstrated by sedimentation experiments that the heterodimerization sequences of GABA_{B} receptor 1 and 2, when tested alone, sediment at the molecular mass of the monomer under physiological conditions and at physiological body temperatures. When mixed in equimolar amounts, GABA_{B} receptor 1 and 2 heterodimerization sequences sediment at the molecular mass corresponding to the heterodimer of the two sequences (see Table 1 of Kammerer et al.). However, when the GABA_{B} R1 and GABA_{B} R2 C-terminal sequences are linked to a cysteine residue, homodimers may occur via formation of disulfide bond.

A diverse variety of coiled coils involved in multimer formation can be employed as the adapters in the subject display system. Preferred coiled coils are derived from. heterodimeric receptors. Accordingly, the present invention encompasses coiled-coil adapters derived from GABA_{B} receptors 1 and 2. In one aspect, the subject coiled coils adapters comprises a C-terminal sequences of GABA_{B} receptor 1, referred to herein as GR1 (SEQ ID NO: 19) EEKSRLLEKENRELEKIIAEKEERVSELRHQLQSVGGC and a sequence of GABA_{B} receptor 2, referred to herein as GR2 (SEQ ID NO:20) TSRLEGLQSENHRLRMKITELDKDLEEVTMQLQDVGGC.

It is to be understood that although the examples describe the use of vector sets which comprise the same pair of adapter sequences (referred to as adapter 1 in the context of expression vectors and adapter 2 in the context of helper display vectors), the vectors described herein can be prepared, and the methods of the invention can be practiced, using alternative adapters. For example, based on the disclosure provided herein suitable adapter sequence can be derived from any of a number of coiled coil domains including for example Winzip-A2B1(Katja M Arndt et al, Structure, 2002, 10:1235-1248); Winzip-A1B1(Katja M Arndt et al, JMB, 2000, 295:627-639); FNfn10(Sanjib Dutta et al, Protein Science, 2005, 14:2838-2848), IAAL-E3/K3(Jennifer R. Litowski and Robert S Hodges, JBC, 2002, 277(40):37272-37279), PcrV/PcrG (Max Nanao et al, BMC Microbiology, 2003:1-9),bZip and derivatives (Jumi A. Shin, Pure Appl. Chem., 2004, 76(7-8):1579-1590),ESCRT-I/II (David J. Gill et al, The EMBO Journal, 2007, 26:600-612), EE1234/RR1234 and derivatives (Johnthan R. Moll et al, Protein Science, 2001, 10:649-655), Laminin a, b, g,.(Atsushi Utani et al, JBC 1995, 270(7):3292-3298), Peptides A/B and derivatives(Ilian Jelesarov and Hans Rudolf Bosshard, JMB, 1996, 263:344-358), artificially designed peptides (Derek N. Woolfson and Tom Alber, Protein Science, 1995, 4:1596-1607), DcoH-HNF-p1(Robert. B Rose et al, Nat. Struct. Biol., 2000; 7(9):744-748), and APC peptides (Catherine L. Day and Tom Alber, JMB, 2000, 301:147-156).

Depending upon the affinity of the adapter subunit interaction associated with a particular pair of adapter subunits it may be possible to eliminate the need for using a disulfide bond to stabilize the resulting coiled coil interaction. For example, the affinities reported in the literature for some of the coiled coil domains listed above range from .00001 nM to 70 nM (4,5 nM for Winzip-A2B1, 24 nM for Winzip-A1B1, 3 nM for FNfn10, 70 nM for IAAL-E3/K3, 15, 6 nM for PcrV/PcrG and 0.0001 nM for EE1234/RR1234 and derivatives).

Alternative heterodimeric transcription factors that are suitable for use as adapters include alpha-Pal/Max complexes and Hox/Pbx complexes Hox represents a large family of transcription factors involved in patterning the anterior-posterior axis during embryogenesis. Hox proteins bind DNA with a conserved three alpha helix homeodomain. In order to bind to specific DNA sequences, Hox proteins require the presence of hetero-partners such as the Pbx homeodomain. Wolberger et al. solved the 2.35 ANG. crystal structure of a HoxB1-Pbx1-DNA ternary complex in order to understand how Hox-Pbx complex formation occurs and how this complex binds to DNA. The structure shows that the homeodomain of each protein binds to adjacent recognition sequences on opposite sides of the DNA. Heterodimerization occurs through contacts formed between a six amino acid hexapeptide N-terminal to the homeodomain of HoxB1 and a pocket in Pbx1 formed between helix 3 and helices 1 and 2. A C-terminal extension of the Pbx1 homeodomain forms an alpha helix that packs against helix 1 to form a larger four helix homeodomain (Wolberger et al. (1999) Cell 96: 587 597; Wolberger et al. J Mol Biol. 291: 521 530).

For example, sequences from novel hetermultimeric proteins can be employed as adapters. In such situation, the identification of candidate sequences involved in formation of heteromultimers can be determined by any genetic or biochemical assays without undue experimentation. Additionally, computer modeling and searching technologies further facilitates detection of heteromultimeric sequences based on sequence homologies of common domains appeared in related and unrelated genes. Non-limiting examples of programs that allow homology searches are Blast (http://www.ncbi.nlm.nih.gov/BLAST/), Fasta (Genetics Computing Group package, Madison, Wis.), DNA Star, Clustlaw, TOFFEE, COBLATH, Genthreader, and MegAlign. Any sequence databases that contains DNA sequences corresponding to a target receptor or a segment thereof can be used for sequence analysis. Commonly employed databases include but are not limited to GenBank, EMBL, DDBJ, PDB, SWISS-PROT, EST, STS, GSS, and HTGS.

Suitable adapters that are derived from heterodimerization sequences can be further characterized based on their physical properties. Preferred heterodimerization sequences exhibit pairwise affinity resulting in predominant formation of heterodimers to a substantial exclusion of homodimers. Preferably, the predominant formation yields a heteromultimeric pool that contains at least 60% heterodimers, more preferably at least 80% heterodimers, more preferably between 85 90% heterodimers, and more preferably between 90 95% heterodimers, and even more preferably between 96-99% heterodimers that are allowed to form under physiological buffer conditions and/or physiological body temperatures. In certain embodiments of the present invention, at least one of the heterodimerization sequences of the adapter pair is essentially incapable of forming a homodimer in a physiological buffer and/or at physiological body temperature. By "essentially incapable" is meant that the selected heterodimerization sequences when tested alone do not yield detectable amounts of homodimers in an in vitro sedimentation experiment as detailed in Kammerer et al. (1999) Biochemistry 38: 13263 13269), or in the in vivo two-hybrid yeast analysis (see e.g. White et al. Nature (1998) 396: 679 682). In addition, individual heterodimerization sequences can be expressed in a host cell and the absence of homodimers in the host cell can be demonstrated by a variety of protein analyses including but not limited to SDS-PAGE, Western blot, and immunoprecipitation. The in vitro assays must be conducted under a physiological buffer conditions, and/or preferably at physiological body temperatures. Generally, a physiological buffer contains a physiological concentration of salt and at adjusted to a neutral pH ranging from about 6.5 to about 7.8, and preferably from about 7.0 to about 7.5. A variety of physiological buffers is listed in Sambrook et al. (1989) supra and hence is not detailed herein. Preferred physiological conditions are described in Kammerer et al., (Biochemistry, 1999, 38: 13263-13269)

Adapters can be further characterized based on their secondary structures. Preferred adapters consist of amphiphilic peptides that adopt a coiled-coil helical structure. The helical coiled coil is one of the principal subunit oligomerization sequences in proteins. Primary sequence analysis reveals that approximately 2 3% of all protein residues form coiled coils (Wolf et al. (1997) Protein Sci. 6:1179 1189). Well-characterized coiled-coil-containing proteins include members of the cytoskeletal family (e.g..alpha.-keratin, vimentin), cytoskeletal motor family (e.g. myosine, kinesins, and dyneins), viral membrane proteins (e.g. membrane proteins of Ebola or HIV), DNA binding proteins, and cell surface receptors (e.g. GABA_{B} receptors 1 and 2).

Coiled-coil adapters of the present invention can be broadly classified into two groups, namely the left-handed and right-handed coiled coils. The left-handed coiled coils are characterized by a heptad repeat denoted "abcdefg" with the occurrence of apolar residues preferentially located at the first (a) and fourth (d) position. The residues at these two positions typically constitute a zig-zag pattern of "knobs and holes" that interlock with those of the other stand to form a tight-fitting hydrophobic core. In contrast, the second (b), third (c) and sixth (f) positions that cover the periphery of the coiled coil are preferably charged residues. Examples of charged amino acids include basic residues such as lysine, arginine, histidine, and acidic residues such as aspartate, glutamate, asparagine, and glutamine. Uncharged or apolar amino acids suitable for designing a heterodimeric coiled coil include but are not limited to glycine, alanine, valine, leucine, isoleucine, serine and threonine. While the uncharged residues typically form the hydrophobic core, inter-helical and intra-helical salt-bridge including charged residues even at core positions may be employed to stabilize the overall helical coiled-coiled structure (Burkhard et al (2000) J. Biol. Chem. 275:11672 11677). Whereas varying lengths of coiled coil may be employed, the subject coiled coil adapters preferably contain two to ten heptad repeats. More preferably, the adapters contain three to eight heptad repeats, even more preferably contain four to five heptad repeats.

In designing optimal coiled-coil adapters, a variety of existing computer software programs that predict the secondary structure of a peptide can be used. An illustrative computer analysis uses the COILS algorithm which compares an amino acid sequence with sequences in the database of known two-stranded coiled coils, and predicts the high probability coiled-coil stretches (Kammerer et al. (1999) Biochemistry 38:13263 13269). Base on design and selection, a variety of engineered coiled coil sequences were reported, with affinity of nanomole to fentomole region (Structure. 2002, 10(9):1235-48; J Mol Biol. 2000, 21;295(3):627-39; Protein Sci. 2005,14(11):2838-48; J Biol Chem. 2002, 277(40):37272-9; BMC Microbiol. 2003, 18;3:21; Protein Science, 2001, 10:649-655). For Example, re-design heterodimeric coiled coil sequences derived from human B-ZIP give a fentomole dissociate constant, which is similar to that for Biotin/steptavidin interaction.

Another class of preferred coiled coil adapters are leucine zippers. The leucine zipper have been defined in the art as a stretch of about 35 amino acids containing 4 5 leucine residues separated from each other by six amino acids (Maniatis and Abel, (1989) Nature 341:24). The leucine zipper has been found to occur in a variety of eukaryotic DNA-binding proteins, such as GCN4, C/EBP, c-fos gene product (Fos), c-jun gene product (Jun), and c-Myc gene product. In these proteins, the leucine zipper creates a dimerization interface wherein proteins containing leucine zippers may form stable homodimers and/or heterodimers. Molecular analysis of the protein products encoded by two proto-oncogenes, c-fos and c-jun, has revealed such a case of preferential heterodimer formation (Gentz et al., (1989) Science 243:1695; Nakabeppu et al., (1988) Cell 55:907; Cohen et al., (1989) Genes Dev. 3:173). Synthetic peptides comprising the leucine zipper regions of Fos and Jun have also been shown to mediate heterodimer formation, and, where the amino-termini of the synthetic peptides each include a cysteine residue to permit intermolecular disulfide bonding, heterodimer formation occurs to the substantial exclusion of homodimerization.

The leucine-zipper adapters of the present invention have the general structural formula known as the heptad repeat (Leucine-X.sub.1-X.sub.2-X.sub.3-X.sub.4-X.sub.5-X.sub.6).sub.n, where X may be any of the conventional 20 amino acids, but are most likely to be amino acids with alpha-helix forming potential, for example, alanine, valine, aspartic acid, glutamic acid, and lysine, and n may be 2 or greater, although typically n is 3 to 10, preferably 4 to 8, more preferably 4 to 5. Preferred sequences are the Fos or Jun leucine zippers.

Sequence of antibody chains that are involved in dimerizing the L and H chains can also be used as adapters for constructing the subject display systems. These sequences include but are not limited to constant region sequences of an L or H chain. Additionally, adapter sequences can be derived from antigen-binding site sequences and its binding antigen. In such case, one adapter of the pair contains antigen-binding site amino acid residues that is recognized (i.e. being able to stably associate with) by the other adapter containing the corresponding antigen residues.

The pairwise interaction between the first and second adapters may be covalent or non-covalent interactions. Non-covalent interactions encompass every exiting stable linkage that do not result in the formation of a covalent bond. Non-limiting examples of noncovalent interactions include electrostatic bonds, hydrogen bonding, Van der Waal's forces, steric interdigitation of amphiphilic peptides. By contrast, covalent interactions result in the formation of covalent bonds, including but not limited to disulfide bond between two cysteine residues, CC bond between two carbon-containing molecules, C--O or C--H between a carbon and oxygen- or hydrogen-containing molecules respectively, and 0--P bond between an oxygen- and phosphate-containing molecule.

Based on the wealth of genetic and biochemical data on vast families of genes, one of ordinary skill will be able to select and obtain suitable adapter sequences for constructing the subject display system without undue experimentation

### Outer Surface Anchor Protein

Suitable anchor proteins for phage display include any of the coat proteins, such as pIII, pVI, pVII, pVIII, and pIX , or the domain of such coat proteins, or any artificial sequences that be assembled into or attach on the our surface of phage particles. As shown herein. Example 7, a scFv antibody was displayed on phage by using helper vector YGMCT, in which the out surface anchor protein is c-terminal domain of pIII illustrated in Fig. 4.

Suitable anchor proteins for bacterial display include a bacterial outer membrane protein, such as pili and flagella, lipoproteins, ice nucleation proteins, and autotransporters. Alternatively, the anchor protein can be an artificial sequence that is assembled into, or attaches to the outer surface of E *coli.* As shown herein, Examples 29-32 show scFv antibody display by using helper vector pMAL1 and pMAL2 (Fig 15), in which bacterial out surface domain Lpp-OmpA is the outer surface anchor protein.

For yeast display, suitable outer surface anchor proteins can be any of the outer wall proteins, with or without, GPI signal, which includes a-agglutinin (Agal and Aga2; Note: Agal with GPI, and Aga2 without), Cwp1, Cwp2, Gas1p, Yap3p, Flo1p, Crh2p, Pir1, Pir2, Pir4, and Icwp in S.cerevisiae; HpSED1, HpGAS1, HpTIP1, HPWP1 in Hansenula polymorpha, and Hwp1p, Als3p, Rbt5p in Candida albicans. Alternatively, the methods of the invention can be practiced in the context of yeast using a cell surface anchor which is an artificial sequence that can be assembled into, or attached to the outer wall of yeast. As shown herein, Example 18 shows yeast display of scFv antibody by using helper vector pMAT3, or pMAT7, or pMAT8, in which the yeast outer surface anchor protein is from C-terminal domain of Flo1, or Cwp2, or Aga2 depicted in Fig. 11.

Mammalian cell surface display can be practiced using a transmembrane domain of any known cell membrane proteins, or a polypeptides with GPI anchor sequences, or a non-cleavable type II signal anchor sequences as a surface anchor. Alternatively, the methods of the invention can be practiced in the context of mammalian cells using a cell surface anchor which is an artificial sequence that can be assembled into, or attached to the cell membrane of mammalian cells. As shown herein, Example 8 shows the display of scFv protein on the mammalian cells by using helper vector pMAG2 (Fig. 5), in which the transmembrane domain of human EGF receptor fused to adapter2 is used as surface anchor for display.

### Signal Sequences

Signal sequences from both prokaryotes and eukaryotes are built along the same general lines. They are about 15-30 amino acids in length and consist of three regions: a positively charged N-terminal region, a central hydrophobic region, and a more polar C-terminal region. There is a large amount of functional and structural homology between the signal peptides of prokaryotic and eukaryotic systems. Therefore, it is expected that some native signal peptides will function in both prokaryotes and eukaryotes.

Consistent with this expectation, some eukaryotic signal peptides have been reported to be functional in prokaryotic cells. For example, the signal peptide from human growth hormone (hGH) and rat proinsulin protein function in E. *coli* (Gene, 1985, 39:247-254); yeast signal peptide of Endo-beta-1,3-glucanase are also functional in *E coli* (Protein Exp. Puri, 2000, 20:252-264). In addition, the prokaryotic signal peptides of *Staphlococcal* protein A, bacterial b-lactamase protein, and bacterial OmpA are functional in mammalian cells (Humphreys et al, Protein Exp. Purif. 2000, 20:252-264). As shown herein, Example 6 and 7 showed the signal peptide from human growth hormone 1 functioned in E. coli cells for scFv secretion and phage display. Similarly, yeast signal peptide of Endo-beta-1,3-glucanase was functional in *E coli* for scFv secretion and phage display demonstrated in Example 16 and 17 in the invention.

Examples of signal peptides that work cross between yeast and mammalian cells are the signal peptides for human pancreatic lipase protein 1 (HPLRP1), human interferon, Human bile salt-stimulated lipase, and yeast Saccharomyces cerevisiae invertase (SUC2) (Tohoku J Exp Med, 1996, 180: 297-308; Protein Exp. Puri, 2006, 47:415-421; Protein Exp. Purif, 1998, 14:425-433). As shown herein, Example 22 shows the use of signal peptide of human pancreatic lipase protein 1 for yeast/mammalian cross-species display.

Any of the native signal peptides identified above for their ability to function in multiple species may be used as signal peptides for the multispecies expression vector of this invention. In addition, an artificial signal peptide sequence characterized by the ability to function across different species of host cells may also be used to practice the methods disclosed herein. The artificial signal peptides may be isolated from the design signal peptide libraries by the method described in Example 28 of this disclosure or any other methods.

The vectors of the present invention generally comprise transcriptional or translational control sequences required for expressing the exogenous polypeptide. Suitable transcription or translational control sequences include but are not limited to replication origin, promoter, enhancer, repressor binding regions, transcription initiation sites, ribosome binding sites, translation initiation sites, and termination sites for transcription and translation.

The origin of replication (generally referred to as an ori sequence) permits replication of the vector in a suitable host cell. The choice of ori will depend on the type of host cells and/or genetic packages that are employed. Where the host cells are prokaryotes and the genetic packages are phage particles, the expression vector typically comprises two ori sequences, one directing autonomous replication of the vector within the prokaryotic cells, and the other ori supports packaging of the phage particles. Preferred prokaryotic ori is capable of directing vector replication in bacterial cells. Non-limiting examples of this class of ori include pMB1, pUC, as well as other E. Coli origins. Preferred ori supporting packaging of the phage particles includes but is not limited to f1 ori, Pf3 phage replication ori. For example, the pUC ori and f1 ori are built in the cross-species expression vector in this invention for phage display.

In the eukaryotic system, higher eukaryotes contain multiple origins of DNA replication (estimated 104-106 ori/mammalian genome), but the ori sequences are not so clearly defined. The suitable origins for mammalian vectors are normally from eukaryotic viruses. Preferred eukaryotic ori includes but is not limited to SV40 ori, EBV ori, HSV oris. The suitable ori for yeast cells includes but is not limited to 2u ori CEN6/ARS4 ori.

As used herein, a "promoter" is a DNA region capable under certain conditions of binding RNA polymerase and initiating transcription of a coding region located downstream (in the 3' direction) from the promoter. It can be constitutive or inducible. In general, the promoter sequence is bounded at its 3' terminus by the transcription initiation site and extends upstream (5' direction) to include the minimum number of bases or elements necessary to initiate transcription at levels detectable above background. Within the promoter sequence is a transcription initiation site, as well as protein binding domains responsible for the binding of RNA polymerase. Eukaryotic promoters will often, but not always, contain "TATA" boxes and "CAT" boxes.

The choice of promoters will largely depend on the host cells in which the vector is introduced. For prokaryotic cells, a variety of robust promoters are known in the art. Preferred promoters are lac promoter, Trc promoter, T7 promoter and pBAD promoter. Normally, to obtain expression of exogenous sequence in multiple species, the prokaryotic promoter can be placed after eukryotic promoter immediately, or inside the intron sequence after eukaryotic promoter.

Suitable promoter sequences for other eukaryotic cells include the promoters for 3-phosphoglycerate kinase, or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Preferred promoters for mammalian cells are SV40 promoter, CMV promoter, b-actin promoter and their hybrids. Preferred promoter for yeast cell includes but is not limited to GAL10, GAL1, TEF1 in S. cerevisia, and GAP, AOX1 in *P. pastoris.*

In constructing the subject vectors, the termination sequences associated with the exogenous sequence are also inserted into the 3' end of the sequence desired to be transcribed to provide polyadenylation of the mRNA and/or transcriptional termination signal. The terminator sequence preferably contains one or more transcriptional termination sequences (such as polyadenylation sequences) and may also be lengthened by the inclusion of additional DNA sequence so as to further disrupt transcriptional read-through. Preferred terminator sequences (or termination sites) of the present invention have a gene that is followed by a transcription termination sequence, either its own termination sequence or a heterologous termination sequence. Examples of such termination sequences include stop codons coupled to various yeast transcriptional termination sequences or mammalian polyadenylation sequences that are known in the art, widely available, and exemplified below. Where the terminator comprises a gene, it can be advantageous to use a gene which encodes a detectable or selectable marker; thereby providing a means by which the presence and/or absence of the terminator sequence (and therefore the corresponding inactivation and/or activation of the transcription unit) can be detected and/or selected.

In addition to the above-described elements, the vectors may contain a selectable marker (for example, a gene encoding a protein necessary for the survival or growth of a host cell transformed with the vector), although such a marker gene can be carried on another polynucleotide sequence co-introduced into the host cell. Only those host cells into which a selectable gene has been introduced will survive and/or grow under selective conditions. Typical selection genes encode protein(s) that (a) confer resistance to antibiotics or other toxins, e.g., ampicillin, kanamycin, neomycin, G418, methotrexate, etc.; (b) complement auxotrophic deficiencies; or (c) supply critical nutrients not available from complex media. The choice of the proper marker gene will depend on the host cell, and appropriate genes for different hosts are known in the art.

In one embodiment of the invention, the expression vector is a shuttle vector, capable of replicating in at least two unrelated expression systems. In order to facilitate such replication, the vector generally contains at least two origins of replication, one effective in each expression system. Typically, shuttle vectors are capable of replicating in a eukaryotic expression system and a prokaryotic expression system. This enables detection of protein expression in the eukaryotic host (the expression cell type) and amplification of the vector in the prokaryotic host (the amplification cell type). Preferably, one origin of replication is derived from SV40 and one is derived from pUC although any suitable origin known in the art may be used provided it directs replication of the vector. Where the vector is a shuttle vector, the vector preferably contains at least two selectable markers, one for the expression cell type and one for the amplification cell type. Any selectable marker known in the art or those described herein may be used provided it functions in the expression system being utilized

The vectors encompassed by the invention can be obtained using recombinant cloning methods and/or by chemical synthesis. A vast number of recombinant cloning techniques such as PCR, restriction endonuclease digestion and ligation are well known in the art, and need not be described in detail herein. One of skill in the art can also use the sequence data provided herein or that in the public or proprietary databases to obtain a desired vector by any synthetic means available in the art. Additionally, using well-known restriction and ligation techniques, appropriate sequences can be excised from various DNA sources and integrated in operative relationship with the exogenous sequences to be expressed in accordance with the present invention.

The examples and figures provided with this disclosure illustrate practice of the present invention in multi-species and cross-species display of protein of interest on the prokaryotic and eukaryotic systems.. The following examples are meant to be illustrative of an embodiment of the present invention and should not limit the scope of the invention in any way. A number of modifications and variations will be apparent to the skilled artisan from reading this disclosure. Such modifications and variations constitute part of the invention.

The practice of the present invention employs, unless otherwise indicated, conventional techniques of cell biology, molecular biology, cell culture and the like which are in the skill of one in the art. All publications and patent applications cited in the specification are indicative of the level of skill of those skilled in the art to which this invention pertains and are hereby incorporated by reference in their entirety.

Although the various compositions and methods of the invention (multispecies and cross-species display strategies) of the invention are exemplified herein using a coding sequence for an anti-VEGF antibody, a skilled artisan will readily appreciate that libraries of expression cassettes encoding diverse libraries of antibody sequences can be used in the expression and display vector sets of the invention to accomplish antibody discovery and engineering protocols.

The practice of the present invention will employ, unless otherwise indicated, conventional techniques of immunology, biochemistry, chemistry, molecular biology, microbiology, cell biology, genomics and recombinant DNA, which are within the skill of the art. See, e.g., PHAGE DISPLAY OF PEPTIDES AND PROTEINS (B.K. Kay et al., 1996); PHAGE DISPLAY, A LABORATORY MANUAL (C.F. Barbas III et al., 2001) Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

### PHAGE AND MAMMALIAN CELL CROSS-SPECIES DISPLAY SYSTEMS

### Example 1: Display Vector pMAG1

The vector pMAG1 exemplifies an expression vector suitable for use in a cross-species display-and production protocols in which a polypeptide library is displayed and/or produced in a prokaryotic display system (phage and bacterial cells) and subsequently displayed and/or produced in a eukaryotic display system (mammalian cells).

pMAG1 vector, which is depicted in Fig. 2A, is built on the backbone of commercial vector pUC19 by insertion at EcoRI and PciI sites with a fully synthetic DNA fragment of 3799 bp. This fully synthetic DNA comprises the following elements: (1) f1 ori for phage package; (2) a cross-species expression cassette, in which the expression of the adapter GR1 fusion is driven by two promoters: a CMV enhancer/Chicken β-actin promoter for mammalian cells and pLac promoter for bacterial cells. The sequence of multiple cloning sites (MCS) for gene of interest cloning is built in the downstream of a signal sequence from human growth hormone 1 with function in E. *coli* and mammalian cells. The HA-His6 tag (DH-tag) sequences are upstream of a coding sequence for GR1 sequence (adapter 1) (SEQ ID NO: 19) for protein detection and Ni-NTA purification. (3) an expression cassette for mammalian selection marker neomycin.

Briefly, the gene synthesis involved dividing the synthetic DNA into 3 pieces of segments of 1379, 1227, and 1287bp for gene synthesis by using Codon Devices BioFab platform technology (Boston). The errors generated from oligo synthesis were corrected by oligo selection with sequences complementary to the synthetic genes, and affinity purification of Mut-S protein column. These synthetic DNA segments with tag sequences containing type II restriction sites were digested and ligated to a full DNA fragment, which was then cloned into pUC19 vector. The resulting vector sequence (SEQ ID NO:1) was confirmed by standard DNA sequencing method.

### Example 2: Display Vector pMAG9

The vector pMAG9 (SEQ.ID. NO: 2) was derived from MAGI vector by inserting a gene encoding a anti-VEGF antibody scFv downstream of signal sequence by SacI and NotI sites. A skilled artisan will readily appreciate that pMAG9 can be used to display a library of coding sequences encoding antibodies of various formats. The scFv gene was amplified by PCR. Briefly, the pABMX268 (US patent application 20040133357A1) DNA was used as template, the PCR primers were listed below: AM-90: 5'-AGTCAGGTAAGCGCTCGCGCTCCGAGGTGCAGCTGGTGCAGAGCG-3' (SEQ ID NO: 3) and AM-91: 5'-ATGACCTCCTGCGTAGTCTGGTACGTC-3 (SEQ ID NO: 4). The PCR reactions were prepared by mixing template/primers solution with pfuUtra Hotstart PCR Master mix according to the instruction manual from Stratagene. Thermal cycle conditions were set as following: 3 minutes denaturation at 94 °C; 30 cycles of 45 second denaturation at 94 °C, 45 second annealing at 55 °C, and 1 minute 30 second extension at 72 °C; and following 10 minutes polishing step at 72°C. The PCR product was digested and purified from 1% agarose gel, and cloned into pMAG1 vector at SaI and NotI sites. Sequence of the scFv was confirmed by standard DNA sequencing method. The resulting pMAG9 vector is showed in Fig.2B.

Having a pUC ori for DNA replication, the β-lactamase gene for ampicillin selection, and a f1 ori for phagemid DNA package, this vector functions in *E coli* cells. In addition, the transcription and expression of the fusion ofscFv-adapter GR1 is driven by the pLac promoter in E. *coli* cells, and by the chicken b-actin promoter in mammalian cells. The signal peptide of human growth hormone 1, with function in both mammalian and bacterial cells (Gene, 1985, 39:247-254), directs the adapter fusion protein through the secretory pathway in both mammalian and bacterial cells.

### Example 3: Phage Helper Vector GMCT

Vector GMCT was constructed from a well-characterized vector, namely M13KO7 (from Amersham Pharmacia) in two steps according to the procedure detailed below. In the first step, the KpnI site was introduced into the gene III signal sequence of KO7 helper phage vector by PCR-based site-directed mutagenesis. This silent mutation did not change the coding sequence of the pIII signal peptide. The K07 genome was amplified by PCR using the following primers which contain KpnI site: p3KN1: 5'-TTTAGTGGTA CCTTTCTATTCTCACTCCGCTG-3' (SEQ ID NO: 5) and p3KN2: 5'-TAGAAAGGTACCACTAAAGGAATTGCGAATAA-3' (SEQ ID NO: 6). These primers share partial sequence homology to the gene III signal sequence. PCR was performed in a 100 ul reaction mixture containing 100 ng KO7 vector DNA, 20 pmol each of primers, 250 uM dNTP, and 1X pfu buffer and pfu DNA polymerase (Stratagene). The reaction mixture was initially incubated at about 96 °C, and then subjected to 15 cycles of PCR in a thermocycler as follows: denaturation at 96 °C for 30 seconds, annealing at 55 °C for 30 seconds, extension at 72 °C for 10 minutes. After amplification, the products were gel purified, cut with KpnI and ligated, then transformed into TG1 bacterial cells by electroporation.

The bacterial cells were selected for kanamycin resistance. Specifically, the kanamycin-resistant colonies were grown in 96-well microtiter plates in 2YT medium with 70 ug/ml Kanamycin, and culture supernatants were used for phage screening by phage ELISA assay to eliminate the loss-of-function mutants caused by PCR errors. Briefly, the phage ELISA was conducted as follows: 100 ul of the supernatants containing phage particles were employed to coat wells of the ELISA plates at 4 °C overnight. After blocking with 5% milk in PBS buffer for 30 minutes at room temperature, the phage particles bound on ELISA plates were further incubated with 100 ul of HRP-conjugated anti-M13 antibody (Amersham Pharmcia) for 1 hour at room temperature.

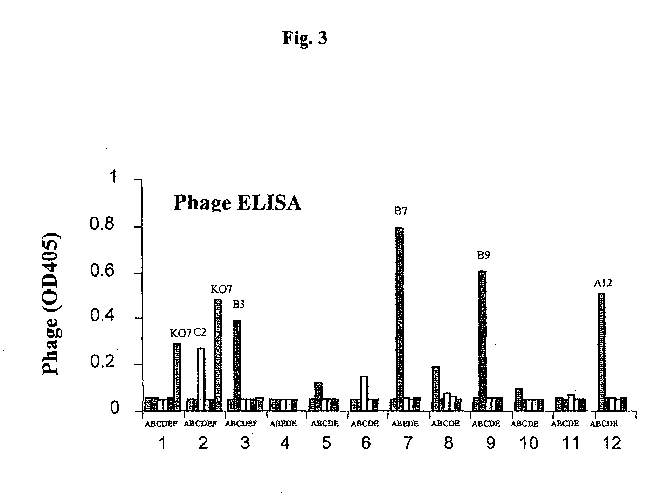

The free anti-M13 antibody was washed away by PBS containing 0.05% Tween 20. The substrate ABTS [2,2'Azino-bis(3-ethylbenzthiazoline-6-sulfonic acid)] was then added. The HRP activity was determined by the absorbance at 405 nm. Total 48 clones were screened for phage generation. The clones C2, B3, B7, B9, and A12 were phage positive as showed in Fig. 3. The DNAs extracted from clones B7, B9 and A12 were prepared from TG1 cultures. Double digestion of vector DNA with Acc65I (isoschizomer of Kpnl) and BamHI showed a 600 bp DNA fragment, which confirms the presence of KpnI site in all of the three K07kpn vector clones. The resulting vector is identical to KO7 except that a unique KpnI restriction site has been introduced silently into the gene III signal sequence (Engineered gene III signal, SEQ ID NO: 7) without disrupting the gene III coding region.

The GMCT phage helper vector was constructed by replacing the KpnI/BamHI fragment of gene III in the KO7kpn B7 helper vector with the synthetic DNA fragment (SEQ ID NO: 8). The resulting GMCT phage vector (Fig. 4) encodes an additional copy of engineered pIII capsid, which comprises a GR2 domain (SEQ ID NO: 20) (adapter 2), a myc-tag sequence for detection of engineered pIII protein and C-terminal domain of pIII (CT domain). Downstream of this engineered gene III, a ribosome binding sequence (S/D) and a signal sequence from the bacterial protein OmpA were fused to gene III sequence. Those two copies of gene III-containing sequences are placed under the control of original gene III promoter.

The ligated vector DNA was transformed into TG1 cells. The kanamycin-resistant colonies were grown in 96-well microtiter plates in 2YT medium with 70 ug/ml Kanamycin, and supernatants were used for phage screening by phage ELISA assay to select phage-positive clones as described above. Out off 10 clones were found to generate phage particles. Clone # 3 was used for large-scale phage preparation.

### Example 4: Generation of GMCT Helper Phage

The supernatant containing GMCT helper phages produced from clone #3 was streaked on a 2YT agar plate. 4 ml of soft agar mixed with 0.5 ml of TG1 culture (OD₆₀₀=0.5) was poured on the plate. Phage plaques were formed after incubation at 37 °C overnight. A single phage plaque was picked and used to inoculate 10 ml 2YT culture with 70 ug/ml kanamycin. After incubating at 37 °C for 2 hours with constant shaking at 250 rpm, the culture was transferred to a 2 liter flask containing 500 ml 2YT with 70 ug/ml kanamycin. The culture was incubated overnight with constant shaking at 37 °C. The phages in the supernatants were then precipitated using polyethylene glycol (PEG)/NaCl, and re-suspended in phosphate-buffed saline (PBS). The phage concentration was determined by OD268 measurement. Generally, a reading of 1 unit at OD268 indicates that 1 ml of supernatant contains approximately 5 X10¹² viral particles. The phage yield for GMCT helper phage was approximately 8 X 10¹¹/ml culture, which was similar with that of M13KO7 helper phages, indicating that the adapter GR2-pIII fusion didn't affect phage particle assembly.

The GR2-Myc assembly on the helper phage particle was confirmed by anti-Myc tag phage ELISA. Briefly, the anti-Myc tag antibody 9E10 (form BD Pharmingen) was coated on ELISA plate, and the phage bound to 9E10 antibody was detected by HRP-conjugated anti-M13 antibody (Amersham Pharmcia), with substrate ABTS [2,2'Azino-bis(3-ethylbenzthiazoline-6-sulfonic acid)].

### Example 5: Mammalian Helper Vector pMAG2

The mammalian helper vector pMAG2 (Fig. 5) was created on the backbone of commercial vector pUC19 by insertion at EcoRI and PciI sites with a fully synthetic DNA fragment of 3134 bp. This fully synthetic DNA comprises sequences for 2 components: (1) Zeocin expression cassette, with SV40 ori/promoter and SV40 polyA; (2) adapter 2 (GR2, SEQ ID: 20) fusion with transmembrane domain of human epidermal growth factor receptor (hEGFR), driven by a CMV promoter and terminated by BGH polyA. The secretory signal sequence for adapter GR2 fusion is from hEGFR.

Briefly, the vector was synthesized by dividing the synthetic DNA into 4 pieces of segments of 808, 790, 829, and 817 bp for synthesis using BioFab platform technology (Codon Devices). The errors generated from oligo synthesis were corrected by oligo selection with sequences complementary to the synthetic genes, and affinity purification of Mut-S protein column. These DNA segments with tag sequences containing type II restriction sites were digested and ligated into full DNA fragment, which was then cloned into pUC19 vector. The resulting vector sequence (SEQ ID NO: 9) was confirmed by standard DNA sequencing method.

### Example 6: Expression of Soluble Fv Protein (scFv) in E. coli cells using pMAG9 Vector

Use of the pMAG9 expression vector in the absence of a helper vector results in expression and secretion of the protein (or proteins) of interest as a fusion protein comprising the product of the coding sequence in combination with adapter 1 (GR1).

In order to display the expressed fusion proteins on the surface the surface of a genetic package, coexpression in the presence of a helper vector which provides a second fusion protein comprising an anchor protein fused in frame with a second adapter (for example GR2) that is capable of associating in a pairwise manner with adapter 1 (GR1) is required. The pairwise interaction between the two adapters facilitates the display of protein of interest on surface of the genetic package or host cell.

In order to demonstrate that in the absence of a helper vector, pMAG9 functions as an expression vector, pMAG9 was directly transformed into TG1 cells for soluble protein expression. Cells transformed with pMAG9 vector were grown at 37 °C shaker in 2YT medium with 100 ug/ml of ampilicin to OD₆₀₀ 0.9. IPTG was then added to final concentration of 0.5 mM for overnight induction at 30 °C shaker.

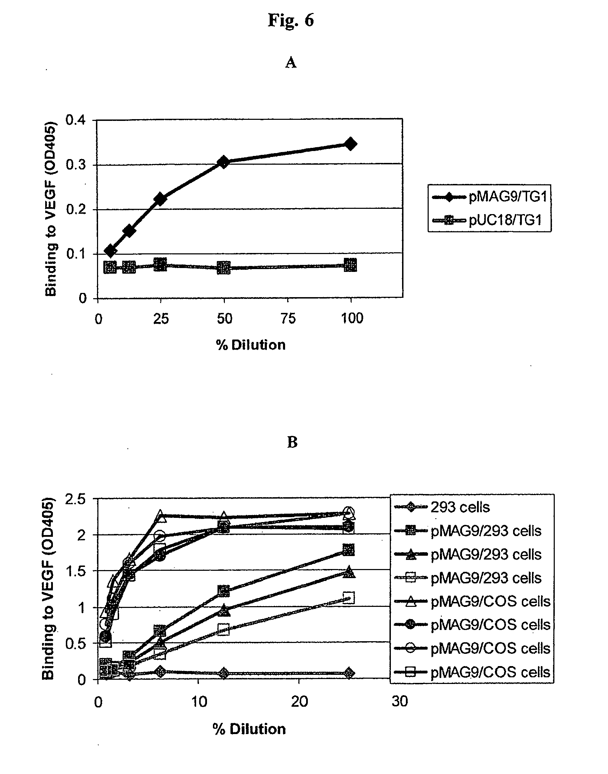

The soluble scFv protein in the supernatant was detected by ELISA assay. Briefly, 100 ul supernatants were added to the ELISA plate coated with of VEGF, the scFv antibody bound to antigen coated on plate was probed with HRP-conjugated anti-HA tag antibody (12CA5). The substrate ABTS [2,2'Azino-bis(3-ethylbenzthiazoline-6-sulfonic acid)] was then added to determine HRP activity. The ELISA in Figure 6A showed that the soluble scFv antibody in pMAG9 culture supernatant bound the VEGF antigen in a dose-dependent manner. In contrast, supernatants generated from negative control cells with pUC18 vector had no binding activity to VEGF. These results provided in Figure 6A demonstrate that pMAG9 functions as an expression vector which is capable of directing soluble protein expression in E. *coli* cells, without having to practice the step of transferring gene of interest from display vector to expression vector.

Furthermore, the soluble protein can be purified from both the supernatants and cell periplamic extracts. Briefly, cell pellet is resuspended into prechilled PPB buffer (200 mg/ml sucrose, 1 mM EDTA, 30 mM Tris-HCl, PH 8.0) at 1/40 volume of growth medium, and incubated on ice for 30 min. Then repeat above process with prechilled 5 mM MgSO4. The periplasmic extracts from cell pellet and culture supernatant are combined, and loaded to a Ni-NTA column. The bound His-tag protein is eluted with 500 mM Imidazole in PBS. The fractions are collected, and analyzed by SDS-PAGE and western blot with anti-HA antibody.

### Example 7: Display of scFv protein on Phage using pMAG9

Use of the pMAG9 vector in combination with a helper vector enables an investigator to display a protein of interest, or a library of proteins on the surface of genetic package or host cells of interest. For example, in order to display scFv on the surface of phage, pMAG9 is used in combination with the GMCT helper phage described above in Example 4.

In order to demonstrate that pMAG9 functions as a display vector capable of directing phage display when it is coexpressed in combination with a helper phage which expresses a fusion protein comprising a viral coat protein fused in frame with a second adapter (adapter 2) that is capable of pairwise combination with the adapter (GR1) present in the scFv-adapter/GR1 fusion protein produced by pMAG9, TG1 cells bearing the pMAG9 vector were super infected with GMCT helper phage at the multiplicity of infection (MOl) of 10. The infected TG1 cells were grown overnight in 2YT/Amp/Kan medium in a 30 °C shaker. The phagemid viral particles in the culture supernatant were precipitated twice by PEG/NaCl, and resuspended in PBS.

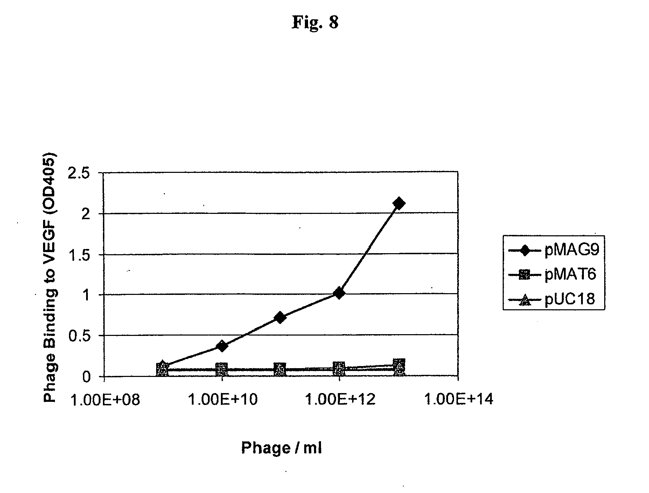

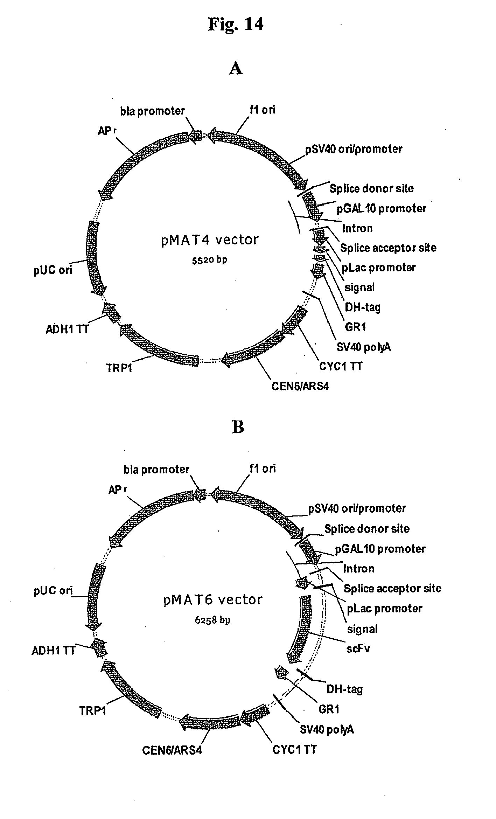

The single chain antibody displayed on the phage surface was detected by phage ELISA using plates coated with antigen VEGF. Briefly, the 96-well ELISA plate, coated with 100ul of 2ug/ml VEGF overnight at 4°C, was blocked with 5% milk in PBS buffer for one hour at room temperature. Thus 100 ul of diluted phages in 5% milk -PBS was employed to coat wells of the ELISA plates for 1 hour at room temperature. The phage particles bound on ELISA plates were further incubated with 100 ul of HRP-conjugated anti-M13 antibody (Amersham Pharmcia) for 1 hour at room temperature. The free anti-M13 antibodies were washed away by PBS containing 0.05% Tween 20. The substrate ABTS [2,2'Azino-bis(3-ethylbenzthiazoline-6-sulfonic acid)] was then added. The HRP activity was determined by the absorbance at 405 nm. The ELISA results illustrated in Figure 8 establish that phage generated from the superinfected cells bearing pMAG9 vector bound to VEGF in a dose-dependent manner. As the negative control, the phage generated from cells with pUC 18 vector, and from cells with pMAT6 vector (a yeast/mammalian cross display vector shown in Fig. 14B) did not show any binding activity to VEGF. These results demonstrate use of the PMAG9/GMCT vector set for the purpose of phage display.

### Example 8: Expression of soluble Fv (scFv) in Mammalian Cells using pMAG9

The experiment performed in Example 6 establishes that pMAG9 functions as an expression vector in bacterial cells (i.e., *E*. *coli*)*.* pMAG9 was also designed to function as a cross-species expression and display vector. In order to demonstrate the cross-species functionality of the vector, pMAG9 was used to direct anti-VEGF scFV expression and production in mammalian cells.

Briefly, soluble anti-VEGF scFv was expressed in mammalian cells using Freestyle 293F cells (Human embryonic kidney cell line, Invitrogen) and COS 6 (transformed monkey kidney cells). FuGENE 6 transfection reagent (Roche Applied Science) was used to perform transfection according to the manufacturer's operation manual. Briefly, 1 or 2 ug of pMAG9 vector DNA was added to diluted FuGENE 6 reagent at ratio of 6:1, or 3:1, or 3:2 in serum-free medium. After 15 minutes of incubation, the FuGENE 6 reagent DNA complex was transferred to cells (1.0 x 10⁶ cells in 2 ml Expression Medium (Invitrogen)) in a 6-well plate. The cells were incubated for 72 h in 95% CO2 atmosphere at 37°C. Supernatants were harvested for evaluation in ananti-HA western blot analysis of scFv-HA-GR1 protein, and an anti-VEGF ELISA.

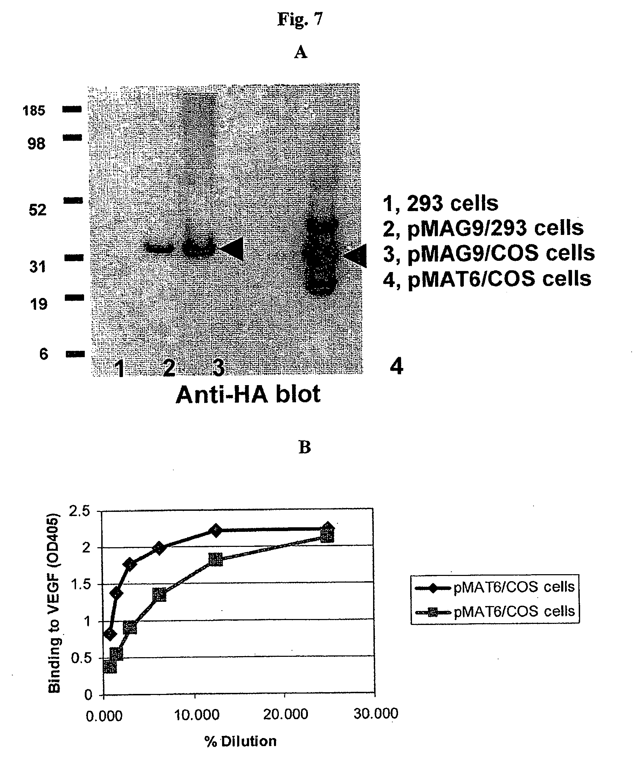

Anti-VEGF scFv protein was purified from 800 ul of culture supernatants with anti-HA antibody beads according to the instruction of ProFound HA Tag IP/Co-IP kit (Pierce). Purified scFv protein was loaded into a 4-12% SDS-PAGE gel (Invitrogen) for protein separation, and transferred to a 0.45 nitrocellose membrane for western blotting analysis by following the instruction of ECL pus western blotting detection system (Amersham). Briefly, the membrane was blocked with 5% non-fat milk in TBST, and incubated with mouse antibody HA-probe F7 (Santa Cruz Biotech) for 1 hour at room temperature. After three times wash with TBST, the membrane was probed with peroxidase labeled anti-mouse antibody for 1 hour incubation. The detection solutions were added on to the washed membrane for 5 min incubation. The chemiluminescent signals were then detected by an x-ray film. The anti-HA blotting results are presented in Figure 7A. The results of the western blot demonstrate the presence of a scFv protein (∼35 KD) in both 293 and COS6 culture supernatants, indicating the soluble protein expression by cross-species display vector pMAG9 in mammalian cells.

The function of scFv protein obtained from mammalian cell culture supernatants was further analyzed by an anti-VEGF ELISA. Briefly, MaxiSorp plate was coated overnight with 100 ul of diluted rh VEGF (R&D System) in bicarbonate buffer pH 9.6 in the concentration 2 ug/ml. After washing twice with PBS, plate was blocked with 5% milk PBS at room temperature for 1 hour. After additional washing, 50 ul of solutions with purified scFv proteins at different dilution were added to the wells and incubated for 1 hr at room temperature. After wash, 50 ul of mouse antibody HA-probe F7 (Santa Cruz Biotech) was added for 1 hour incubation at room temperature, following 1 hour incubation with anti-mouse antibody-HRP conjugate (Santa Cruz Biotech) at dilution of 1:2,000 in 5% milk PBS. Finally, ABTS substrate (Pierce) was then added for color development. Plate was read at OD405 by 96 well plate-reader (Molecular Devices).

The ELISA results presented in Figure 6B show a dose-dependent VEGF binding activity in pMAG9 culture supernatants from 3 individual 293 cell transfections and 4 individual COS 6 cell transfections. Taken together with data in Figure 6A and Figure 7A, the ELISA results establish the pMAG9 functions as a cross-species expression vector in both *E. coli* and mammalian cells.

### Example 9: Display of scFv protein in Mammalian Cells using pMAG9 vector

After establishing that pMAG9 in combination with helper vector GMCT directs the display of scFv on phage (see Example 7 supra) and that pMAG9 expressed in mammalian cells in the absence of a helper vectors functions to direct the expression of soluble scFv in mammalian cells (see Example 8), the following experiment was performed to establish the cross-species display function of this vector.

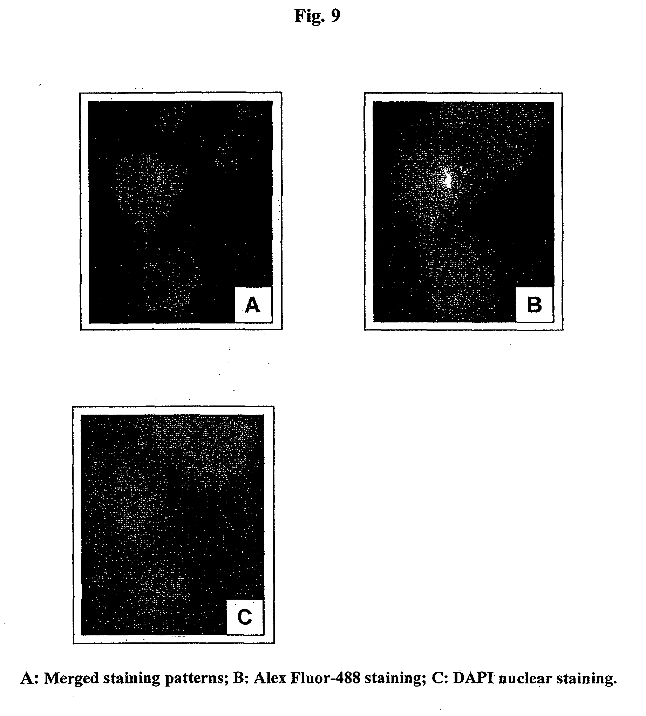

COS 6 cells were grown on coverslip in 6-well plates with Dulbecco's modified Eagle's medium supplemented with 10% fetal bovine serum, 100 units/ml penicillin G, 100 µg/ml streptomycin. pAMG9 expression vector and helper vector pMAG2 were co-transfected into COS 6 cells using FuGene 6 transfection reagent (Roche Applied Science) according to the manufacturer's instructions. Briefly, 0800 ng of plamid DNA (400 ng of pMAG9 + 400 ng of pMAG2) was added to diluted FuGENE 6 reagent at 3:2 ratio of FuGene 6 reagent (ul):DNA complex (ug) in serum-free medium. The FuGENE reagent DNA complex was incubated for 15 min at room temperature and then added to the cells. After 48 hr, HA tagged scFv -GR1 fusion protein (from pMAG9 vector) displayed on the cell surface was detected with Alexa 488 conjugated anti-HA antibody. Briefly, COS 6 cells were fixed with 4 % formaldehyde for 20 min, blocked with 5% BSA in PBS for 30 min at 25 °C and then incubated with Alexa 488 conjugated anti-HA antibody (1:100 dilution, Invitrogen) in PBS (1:500) for 60 min. Cells were also stained with DAPI (Invitrogen) to visualize the nucleus. Cells were observed under a Zeiss Axiovert 135 microscope with Plan-Neofluar 100/1.30 oil objective lens. The results depicted in Figure 9 establish clear surface localization of HA-tagged scFv-GR1, demonstrating the cell surface display of scFv using pMAG9 vector.

Alternatively, flow cytometric analysis can be performed 48 hours after transfection to detect the presence of surface displayed anti-VEGF scFv. Briefly, biotinylated rhVEGF or VEGF-Fc is added to 25 ul of the cell suspension (4 x 10⁶ cells/ml) for 60 min incubation at 4°C. As a negative staining control, an identical sample of cells is stained with biotinylated soybean trypsin inhibitor. Cells are then incubated with avidin-FITC or anti-Fc antibody-FITC for a further 30 min at 4 °C in the dark. Cells are washed twice with RDF1 buffer and resuspend the cells in 0.2 mL RDF1 buffer for flow cytometry analysis in an FACSvantage flow cytometer (BD Biosciences) or Agilent 2100 bioanalyzer (Agilent technology). (Note: This is a prophetic FACS experiment)

### Example 10: Selection and Cross-species Display of desired Polypeptides on Phage and Mammalian cells (Note: Prophetic experiment)

A library of DNA sequences encoding a diverse repertoire of polypeptides can be cloned into the pMAG1 or pMAG9 vector for expression of soluble polypeptides, or display, in *E. coli* and/or mammalian cells.

Expression libraries can be displayed on the surface of prokaryotic genetic packages or eukaryotic host cells, by the coexpression of a helper vector, such as GMCT helper vector for phage display or pMAG2 for mammalian cell display. Polypeptides characterized by a desired specificity (binding activity) can be enriched by sequential rounds of panning. Briefly, a phage panning process would proceed as follows: 96-well plates are coated with specific antigens at a concentration of 1-10 ug/ml for overnight at 4 °C. After washing with PBS and blocking with 5% milk/PBS, 10 ¹¹⁻¹² phages in 5% milk/PBS are added and incubated for 2 hours at room temperature. After several washing with PBST and PBS, the bound phages are eluted with 10 ug/ml trypsin for 30 min incubation, since the subject helper phage have a cleavable sites at Myc-tag fused to pIII protein. Trypsin elution is more efficient than 100 mM triethylamine (usually used in conventional phage panning) in our experiments. Upon repeating the process for 2-3 times, a pool of phages displaying the desired polypeptides can be enriched. The enriched phages can infect E. *coli* cells, which can be used for preparation of vectors with genes encoding desired polypeptides.

This small pool of vector DNAs selected from phage library can thus be directly transfected into mammalian cells for cell surface display by co-transfection with mammalian helper vector pMAG2. The leads with desired properties can be isolated by performing a couple of rounds of FACS sorting. Briefly, 10-20 ug DNA is mix with 3x10⁶ of HEK 293 cells or COS cells washed with ice-cold PBS in a cuvette. After 10 min incubation on ice, the cell/DNA mixture is electroporated with GenePulser Xcell (Bio-Rad) set at 250 uF, 660 V. After 10 min incubation at room temperature, cells are transferred to 20 ml medium in 75 cm culture flask, and incubated at 37°C, 5% CO₂ for 2-3 days. Alternatively, FuGene 6 transfection reagent can be used for transfections following the protocol described by Roche Applied Science.

A suitable selection process for identifying antibodies with a binding specificity for a target antigen comprises incubating approximately 10⁷ transfected cells with 0.2 nM biotinylated target antigen and 20 ug/ml of anti-HA tag mAb 12CA5 (Santa Cluze Biotech) in PBS buffer at 25°C for 1 h. The cells should then be rinsed with ice-cold PBS buffer, and labeled with FITC-labeled goat anti-mouse FITC (AMI0408; BioSource International, Camarillo, CA for monitoring antibody expression) and streptavidin-R-PE conjugate (S866; Molecular Probes, for labeling antigen binding cell) by 1 hour incubation at 4C. The cells are thus sorted on a FACSVantageSE (BD Biosciences) with a proper sort window. The top 0.1% PE-positive cells are collected. The DNAs of both display and helper vectors recovered from selected cells are transformed into E. *coli* cells. Only display vector with f1 ori can be packaged by regular helper phage such as KO7, therefore be separated from mammalian helper vector. The recovery display vector DNAs can then be used for another round FACS sorting or for DNA sequencing.

### PHAGE AND YEAST CROSS-SPECIES DISPLAY SYSTEM

### Example 11: Display Vector pMAT2

pMAT2 provides an expression vectors that is suitable for expression and cross-species display between prokaryotic system (phage and bacterial cell) and eukaryotic system (yeast cells). As shown in Figure 10A, pMAT2 is created on the backbone of commercial vector pUC19 by insertion at AatII and PciI sites with a fully synthetic DNA fragment of 3184 bp. This fully synthetic DNA comprises (1) f1 ori for phage package; (2) a cross-species expression cassette for the adapter GR1 (SEQ ID: 19) fusion, which is driven by two promoters: a yeast pGAL1 promoter for expression in yeast cells, and plac promoter for bacterial cells. The sequence of multiple cloning sites (MCS) for gene of interest cloning is built in the downstream of a dual functional signal sequence (yeast endo-ß-1,3-glucanase protein Bg12p). The HA-His6 tag (DH-tag) sequences are upstream of GR1 sequence for protein detection and Ni-NTA purification. (3) yeast CEN/ARS ori for replication; and (4) a expression cassette for yeast TRP1 auxotrophic marker.

Briefly, the vector was synthesized by dividing the DNA into 3 segments of 1196, 804, and 1294 bp for gene synthesis by using BioFab platform technology (Codon Devices). The errors generated from oligo synthesis were corrected by oligo selection with sequences complementary to the synthetic genes, and affinity purification of Mut-S protein column. These synthetic DNA segments with tag sequences containing type II restriction sites were digested and ligated into a full DNA fragment, which was then cloned into pUC19 vector. The resulting vector sequence (SEQ ID NO:10) was confirmed using standard DNA sequencing methods. (Note: this is phage/yeast expression vector without scFv gene).

### Example 12: Cross-display vector pMAT5

The vector pMAT5 (SEQ ID NO: 11) was derived from pMAT2 vector by insertion of a scFv gene (expression cassette) encoding an anti-VEGF antibody downstream of signal sequence by SacI and NotI sites. A skilled artisan will readily appreciate that pMAT2 can be used to display a library of coding sequences encoding antibodies of diverse formats. The scFv gene was amplified by PCR. Briefly, the pABMX268 DNA was used as template, the primers for PCR were listed below:AM-90: 5'-AGTCAGGTAAGCGCT CGCGCTCCGAGGTGCAGCTGGTGCAGAGCG-3' (SEQ. ID. NO 3) and AM-91: 5'-ATGACCTCCTGCGTAGTCTGGTACGTC-3 (SEQ. ID. NO 4).

The PCR reaction was prepared by mixing template/primer solutions with pfuUtra Hotstart PCR Master mix as described in instruction manual from Stratagene. Thermal cycle conditions were as follows: 3 minutes denaturation at 94°C; 30 cycles of 45 second denaturation at 94°C; 45 second annealing at 55°C, and 1 minute 30 second extension at 72 °C; followed by a 10 minute polishing step at 72°C. The PCR product was digested and purified from 1% agarose gel, thus cloned into pMAT2 vector at SacI and NotI sites.

The sequence of scFv was confirmed by DNA sequencing. Components of the pMAT5 vector are depicted in Figure10B. In pMAT5, the transcription and expression of the fusion of the polypeptide of interest/adapter GR1-protein driven by the pLac promoter in E. *coli* cells, and by the yeast GAL1 promoter in yeast cells. The signal peptide of yeast endo-β-1,3-glucanase protein Bgl2p, functions in both yeast and bacterial cells (Protein Exp. Puri, 2000, 20:252-264), and directs the adapter fusion protein through the secretory pathway of both yeast and bacterial cells.

### Example 13: Yeast helper vector pMAT3

pMAT3, which is graphically depicted in Figure 11A is a yeast display helper vector which expresses a fusion protein comprising the C-terminal yeast out surface GPI anchor protein Flo 1 in frame with adapter 2. This vector was constructed on the backbone of commercial vector pUC19 by insertion at AatII and PciI sites with a fully synthetic DNA fragment of 7030 bp. This fully synthetic DNA comprises sequences for three expression cassettes: (1) yeast URA3 selection marker; (2) Zeocin marker for yeast selection; (3) adapter 2 (GR2) (SEQ ID NO: 20) fusion with yeast out well protein Flo1 C-terminal 1100 amino acids, under control of yeast pGAL1 promoter and the secretory signal sequence of Flo1. Briefly, the synthetic DNA was divided into 8 pieces of segments of 1112, 740, 748, 1042, 897, 1152, 802, and 821 bp (#1 to #8) for gene synthesis by using BioFab platform technology of Codon Devices. The errors generated from oligo synthesis were corrected by oligo selection with sequences complementary to the synthetic genes, and affinity purification of Mut-S protein column.

The synthetic DNA segments #1 to 4 were digested by the type II restriction enzymes and ligated into one DNA fragment, which was then cloned into pUC19 vector. The synthetic DNA segments #5 to 8 were digested by the type II restriction enzymes and ligated into another one DNA fragment, which was then cloned into the pUC19 vector with the first fragment. The resulting vector sequence (SEQ ID NO: 12) was confirmed by standard DNA sequencing methods.

### Example 14: Yeast helper vector pMAT7 vector

pMAT7, which is graphically depicted in Figure 11B, is another yeast display helper vector which expresses a fusion protein comprising the yeast outer surface protein Aga2 in frame with adapter 2 (GR2) (SEQ ID NO: 20). This vector was created from the yeast helper vector pMAT3 by replacing BamHI-HindIII fragment with a synthetic DNA fragment of 208 bp. This synthetic DNA comprises the sequence encoding yeast out well protein Aga2. Using BioFab platform technology of Codon Devices, the errors generated from oligo synthesis were corrected by oligo selection with sequences complementary to the synthetic genes, and affinity purification of Mut-S protein column. The final vector sequence (SEQ ID NO:13) was confirmed by standard DNA sequencing.

### Example 15: Yeast helper vector pMAT8

pMAT8, which is graphically depicted in Figure 11C, embodies an alternative yeast display helper vector, which expresses a fusion protein comprising C-terminal yeast out surface GPI anchor protein Cwp2 in frame with adapter 2 (GR2) (SEQ ID NO: 20). This vector was created from the yeast helper vector pMAT3 by replacing BamHI-HindIII fragment with a synthetic DNA fragment of 1252 bp. This synthetic DNA comprises the sequence encoding yeast out well protein Cwp2 fused with S/T rich region of Flo1. Using BioFab platform technology of Codon Devices at Boston, the errors in synthetic DNAs were corrected by oligo selection with sequences complementary to the synthetic genes, and affinity purification of Mut-S protein column. The final vector sequence (SEQ ID NO: 14) was confirmed by standard DNA sequencing.

### Example 16: Expression of soluble scFv protein in E. coli cells using pMAT5

Phage/yeast expression vector pMAT5 was directly transformed into E. *coli* TG1 cells for soluble protein expression. Transformed cells from 3 individual clones were grown at 37°C shaker in 2YT medium with 100 ug/ml of ampilicin to OD₆₀₀ 0.9. IPTG was added to final concentration of 0.5 mM for overnight induction at 30 °C shaker. The soluble scFv protein in the supernatant was detected by ELISA assay. Briefly, 100 ul supernatants were added to the ELISA plate coated with of VEGF, the scFv antibody bound to antigen on plate was probed with HRP-conjugated anti-HA tag antibody (12CA5). The substrate ABTS [2,2'Azino-bis(3-ethylbenzthiazoline-6-sulfonic acid)] was then added to determine HRP activity.

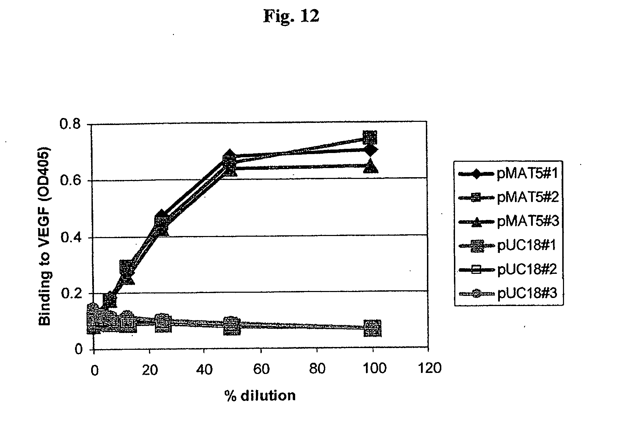

The data presented in Figure 12 establishes a dose-dependent biding activity to VEGF in the pMAT5 supernatant from three individual clones, and demonstrated the function of pMAT5 vector for expression of soluble protein in *E. coli* cells.

### Example 17: Display of scFv protein on phage surface using pMAT5

The data presented in Example 16 established that pMAT5 functions as an expression vector for soluble scFv expression in *E. coli* cells. In order to demonstrate that coexpression of pMAT5 in combination with the GMCT helper phage functions to display scFv (or a library of scFvs) on the surface of phage, the pMAT5 vector was transformed into TG1 cells, and the transformed *E. coli* cells were super infected with GMCT helper phage at a multiplicity of infection (MOI) of 10.

The infected TG1 cells were grown in 2YT/Amp/Kan at 30°C overnight. The phagemid particles in the culture supernatant were precipitated twice by PEG/NaCl, and resuspended in PBS. The single chain antibody displayed on the phage surface were detected by phage ELISA using plates coated with VEGF. Briefly, the 96-well ELISA plate, coated with 100ul of 2ug/ml VEGF overnight at 4°C, was blocked with 5% milk in PBS buffer for one hour at room temperature. Thus 100 ul of diluted phages in PBS-milk were employed to coat wells of the ELISA plates for 1 hour at room temperature. The phage particles bound on ELISA plates were further incubated with 100 ul of HRP-conjugated anti-M13 antibody (Amersham Pharmcia) for 1 hour at room temperature. The free anti-M13 antibodies were washed away by PBS containing 0.05% Tween 20. The substrate ABTS [2,2'Azino-bis(3-ethylbenzthiazoline-6-sulfonic acid)] was then added. The HRP activity was determined by the absorbance at 405 nm.

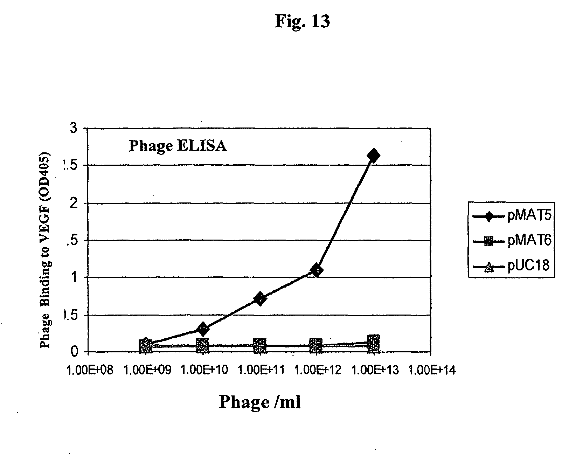

The ELISA results presented in Figure 13 establish that phage generated from the cells bearing pMAT5 vector bound to VEGF in a dose-dependent manner. As the negative control, the phage generated from cells with pUC18 vector, and from cells with pMAT6 vector (a yeast/mammalian cross display vector shown in Fig. 14B) did not show any binding activity to VEGF. These results demonstrated the vector set pMAT5 and helper vector GMCT function to direct the display of polypeptide sequences on the surface of phage.

### Example 18: Expression of Soluble scFv protein in Yeast using pMAT5

pMAT5 is a cross-species expression vector. The data presented in Example 16 establishes that pMAT5 functions to direct the expression of soluble fusion proteins comprising scFv polypeptides fused in frame with adapter 1 (GR1 protein) in bacterial cells. In order to establish that pMAT5 can direct expression in yeast cells, the vector could be transformed into yeast YHP499 cells using Frozen-EZ Yeast Transformation II Kit according to Zymo Research's instruction.

Yeast cells from a single colony can be grown in 50 ml of SD-CAA/Trp⁻ selective medium at 3°C until saturated (OD600=2 ∼3), and resuspended in 50 ml SG/ R-CAA/Trip medium with 100 ug/ml ampcillin, 0.1% dex. After 2-3 days induction, the supernatant should be harvested, and dialyzed against PBS buffer.

The soluble scFv protein in the supernatant can be measured by ELISA Briefly, 100 ul supernatant or anti-HA beads purified scFv from the supernatant are added to the ELISA plate coated with of VEGF, the scFv antibody bound to the plated antigen can be detected with HRP-conjugated anti-HA tag antibody (12CA5). The substrate ABTS [2,2'Azino-bis(3-ethylbenzthiazoline-6-sulfonic acid)] should then added to determine HRP activity. The ELISA results may be used to demonstrate the function of pMAT5 vector for expression of soluble protein in yeast cells. Alternatively, culture supernatants can be loaded to a Ni-NTA column for purification. The bound His-tag protein can be eluted with 500 mM Imidazole in PBS. The fractions may be collected, for analysis by SDS-PAGE and western blot with an anti-HA antibody.

### Example 19: Display of scFv protein on yeast cell surface using pMAT5

The pMAT5 expression vector can also be used to direct the display of scFv antibody the surface of yeast host cells. Briefly, pMAT5 can be cotransformed into yeast cells in the presence of a yeast display helper vector (i.e., pMAT3, or pMAT7, or pMAT8) using a suitable protocol, such as Frozen-EZ Yeast Transformation II Kit. Alternatively, yeast cells, bearing a copy of yeast helper vector pMAT3 or pMAT7 or pMAT8 in its genome, can be transformed with expression vector pMAT5.

Yeast cells comprising both an expression vector and a helper display vector (i.e, a yeast display vector set) can be selected on SD-CAA/Trp-/Ura- agar plates or SD-CAA/Trp⁻/Zeocine, and grown in the same selective medium until saturated. The cells should then be transferred to induction medium containing 100 ug/ml ampicillin, 0.1% dex, and incubated at 20° or 25 °C with shaking overnight. After washing with ice-cold PBS buffer, yeast cells are incubated with 100 nM biotinylated VEGF, and 5 mg/ml anti-HA (12CA5) antibody for 30 min at room temperature in FACS buffer (PBS/0.5% BSA/2 mM EDTA), and washed three times in ice cold wash buffer. The cells may be probed by incubation with 5 mg/ml streptavidin-R-PE conjugate (SA-PE) and Goat anti-mouse-488 for 30 min on ice in the dark, washed three times, and resuspended in 1 ml FACS buffer. The fluorescences associated with scFv displayed on the yeast cells can be measured by flow cytometry using a FACS Cailibur flow cytometry or Agilent 2100 bioanalyzer.

### Example 20: Selection of desired Polypeptides displayed on Phage and Yeast cells

A diverse collection of DNA sequences (i.e., a repertoire) can be cloned into pMAT2 vector for expression of soluble polypeptides sequentially in prokaryotic (i.e, E. *coli*) and prokaryotic (i.e. yeast cells). The resulting expression library can be displayed on the surface of phage or yeast cells, using different helper display vectors of the invention. More specifically, GMCT may be used as a phage display helper vector, and the yeast display helper vectors pMAT3 or pMAT7, or pMAT8 may be used for display on yeast cells.

The specific proteins or peptides displayed on phage can be initially enriched by several rounds . Briefly, a suitable panning process would involve coating a 96-well plate with a target antigens(s) at a concentration of 1 -10 ug/ml for overnight at 4°C. After washing with PBS and blocking with 5% milk/PBS. 10 11-12 phages are added and incubated for 2 hours at room temperature. After several washing with PBST and PBS, the bound phages may be eluted with 10 ug/ml trypsin for 30 min incubation, since the subject helper phage have a cleavable Myc-tag fused to pIII protein. TG1 cells are infected with eluted phages, and are ready for next round phage preparation and panning. Upon repeating the process for 2-3 times, a pool of phages displaying the desired polypeptides can be enriched. The enriched phages can infect E. *coli* cells, which can be used to prepare vector DNAs with genes encoding desired polypeptides.

This small pool of vector DNAs selected from phage library can subsequently be directly transformed into recipient yeast host cells comprising a copy of yeast helper vector pMAT3 or pMAT7 or pMAT8. Transformed yeast cells displaying leads with a desired property can be isolated by few round flow cytometry sorting. Briefly, yeast cells may be grown in SD-CAA selective medium for 18-22 h at 30oC, and then transferred to SG/ R-CAA expression medium for 16-18 h at 20 or 25oC. Yeast cells are incubated with biotinylated antigen and 5 mg/ml anti-HA (12CA5) antibody for 30 min at room temperature in FACS wash buffer (PBS/0.5% BSA/2 mM EDTA), and washed with ice cold wash buffer. Thus cells can be probed by incubation with 5 mg/ml streptavidin-R-PE conjugate and Goat anti-mouse -488 for 30 min on ice in the dark, and resuspended in FACS wash buffer for sorting by flow cytometry. Selections can be performed using a BD Bioscience FACS Vantage DiVa set for purifying selection, and sort gates will be determined to select the desired double positive cells. Collected cells will be plated on SD-CAA plates with Penn/Strep and grown at 30 °C for 2 days. Cells are then resuspended and amplified for the next round. The individual colonies can be picked after the selections. The vector DNA from the yeast cells can be recovered for sequencing by using the Zymoprep yeast plasmid miniprep kit (Zymo Research) according to the instruction.

### MULTI-SPECIES DISPLAY BETWEEN YEAST AND MAMMALIAN HOST CELLS

### Example 21: Display vector pMAT4

pMAT4, which is depicted in Figure 14A, is a multi- or cross-species expression vector for the sequential expression, or display, of polypeptide libraries in yeast and mammalian cells. It was created from vector pMAT2 by replacing the PacI-SacII fragment with a fully synthetic DNA fragment of 1420 bp. This fully synthetic DNA was generated by using BioFab platform technology (Codon Devices), and comprises a yeast pGAL10promoter inside an intron sequence. Therefore, the expression of library proteins fused in frame to adapter 1 (GR1) (SEQ ID NO: 19) is driven by three promoters: SV40 promoter for mammalian cells; pGAL10 for yeast cells; and pLac promoter for E. *coli.* The signal peptide of human pancreatic lipase protein 1 (HPLRP1) is functional in both yeast and mammalian cells (Protein Exp. Puri, 2006, 47:415-421). The errors generated from oligo synthesis were corrected by oligo selection with sequences complementary to the synthetic genes, and affinity purification of Mut-S protein column. The finpolynucleotide sequence of pMAT4 (SEQ ID No:15) was confirmed by standard DNA sequencing.

### Example 22: Display vector pMAT6

pMAT6 was derived from pMAT4 vector by insertion of a scFv gene of an anti-VEGF antibody downstream of signal sequence by XhoI and NotI sites. The scFv gene was amplified by PCR from pABMX268DNA. The PCR primers were listed as follows:AM-90: 5'-AGTCAGGTAAGCGCTCGCGCTCCGAGGTGCAG CTGGTGCAGAGCG-3' (SEQ. ID. NO 3) and AM-91: 5'-ATGACCTCCTGCGTAGTC TGGTACGTC-3 (SEQ. ID. NO 4). The PCR reactions were prepared by mixing templet /primers solution with pfuUtra Hotstart PCR Master mix as described in instruction manual from Stratagene. Thermal cycle conditions were as follows: 3 minutes denaturation at 94°C; 30 cycles of 45 second denaturation at 94°C; 45 second annealing at 55°C, and 1 minute 30 second extension at 7 °C; followed by a 10 minute polishing step at 72°C. The PCR product was digested and purified from 1% agarose gel, thus cloned into pMAT4 vector at XhoI and NotI sites. The sequence of scFv was confirmed by DNA sequencing.

pMAT6 (SEQ ID NO: 16) is depicted graphically in Figure14B. The human pancreatic lipase protein 1 (HPLRP1) signal peptide is functional in both yeast and mammalian cells (Protein Exp. Puri, 2006, 47:415-421), and will direct the expressed fusion protein (scFv polypeptide in frame with adapter 1) through the secretory pathway of eukaryotic (yeast and mammalian) host cells.

### Example 23: Expression of soluble scFv in Yeast cell using pMAT6

The expression vector pMAT6 can be transformed into yeast cells YHP499 using Frozen-EZ Yeast Transformation II K according to Zymo Research's instruction. Cells from single colony are grown in 50 ml of SD-CAA/Trp- selective medium at 30°C until saturated, and resuspended in 50 ml TEP G/R medium with 100 ug/ml ampcillin, 0.1% dex. After 2-3 days induction, the supernatants can be harvested, and dialyzed against PBS buffer.

The soluble scFv protein in the supernatant can be measured in an ELISA assay. Briefly, 100 ul supernatant is added to the ELISA plate coated with of VEGF, the scFv antibody bound to antigen on plate is probed with HRP-conjugated anti-HA tag antibody (12CA5). The substrate ABTS [2,2`Azino-bis(3-ethylbenzthiazoline-6-sulfonic acid)] is then added to determine HRP activity. Moreover, the supernatant can be loaded to a Ni-NTA column for purification. The bound His-tag protein is eluted with 500 mM Imidazole in PBS. The fractions are collect, and analyzed by SDS-PAGE and western blot with anti-HA antibody.

### Example 24: Display of scFv on Yeast using pMAT6

The expression vector pMAT6 vector and a yeast helper display vector, such as pMAT3, or pMAT7, or pMAT8 can be co-transformed into yeast cells as described above. Alternatively, yeast cells baring a copy of yeast helper vector pMAT3 or pMAT7 or pMAT8 can be used for transformation of cross display vector only.

Recipient host cells having both display vector and helper vector can be selected on SD-CAA/Trp-/Ura- or SD-CAA/Trp-/Zeocine agar plate, and grown in the same selective medium until saturated. The cells can then transferred to induction medium with 100 ug/ml ampcillin, 0.1 % dex, and incubated at 20C with shaking for overnight. After wash with ice-cold PBS buffer, yeast cells are incubated with 100 nM biotinylated VEGF, and 5 mg/ml anti-HA (12CA5) antibody for 30 min at room temperature in FACS buffer (PBS/0.5% BSA/2 mM EDTA), then washed three times in ice cold wash buffer. The cells may be probed by incubation with 5 mg/ml each SA-PE and GaM-488 for 30 min on ice in the dark, then washed three times again and resuspended in 1 ml FACS buffer. The fluorescences associated with scFv displayed on cells are measured in FACs Cailibur flow cytometry or Agilent 2100 bioanalyzer (Agilent Techologies).

### Example 25: Expression of Soluble scFv in Mammalian Cells using pMAT6

The ability of pMAT6 to direct expression of soluble anti-VEGF scFv fusion protein in mammalian cells was evaluated using COS cells according the description in Example 8. Briefly, 1 or 2 ug of pMAT6 vector DNA was used to diluted FuGENE 6 reagent at ratio of 3:1, or 3:2 in serum-free medium. After 15 minutes of incubation, the FuGENE 6 reagent DNA complex is transferred to cells (1.0 x 10⁶ cells in 2 ml Expression Medium (Invitrogen)) in a6-well plate. The cells should be incubated for 72 h in 95% CO2 atmosphere at 37°C. Supernatants can be harvested for anti-HA western blot analysis of scFv-HA-GR1 protein, and anti-VEGF ELISA assay to measure functional scFv protein.

First, the scFv protein was purified from 800 ul of culture supernatants with anti-HA antibody beads according to the instruction of ProFound HA Tag IP/Co-IP kit (Pierce). Thus, the purified scFv protein was loaded into a 4-12% SDS-PAGE gel (Invitrogen) for protein separation, and transferred to a 0.45 nitrocellose membrane for western blotting analysis by following the instruction of ECL pus western blotting detection system (Amersham). Briefly, the membrane was blocked with 5% non-fat milk in TBST, and incubated with mouse antibody HA-probe F7 (Santa Cruz Biotech) for 1 hour at room temperature. After three times wash with TBST, the membrane was probed with peroxidase labeled anti-mouse antibody for 1 hour incubation. The detection solutions were added on to the washed membrane for 5 min incubation. The chemiluminescent signals were then detected using x-ray film. The anti-HA blotting results provided in Figure 7A demonstrate that pMAT6 functions to direct the expression ofscFv protein (∼35 KD) into COS6 cell culture supernatants.

The function of scFv protein from COS 6 culture supernatants was further analyzed by an anti-VEGF ELISA assay. Briefly, MaxiSorp plate was coated overnight with 100 ul of diluted rh VEGF (R&D System) in bicarbonate buffer pH 9.6 in the concentration 2 ug/ml. After washing twice with PBS, plate was blocked with 5% milk PBS at room temperature for 1 hour. After additional washing, 50 ul of solutions with purified scFv proteins at different dilution were added to the wells and incubated for 1 hr at room temperature. After wash, 50 ul of mouse antibody HA-probe F7 (Santa Cruz Biotech) was added for 1 hour incubation at room temperature, following 1 hour incubation with anti-mouse antibody-HRP conjugate (Santa Cruz Biotech) at dilution of 1:2,000 in 5% milk PBS. Finally, ABTS substrate (Pierce) was then added for color development. Plate was read at OD405 by 96 well plate-reader (Molecular Devices). The ELISA results presented in Figure 7B showed a dose-dependent VEGF binding activity in pMAT6 culture supernatants from 2 individual COS 6 cell transfections, indicating the functional expression of scFv from yeast/mammalian pMAT6 vector in mammalian cells.

### Example 26: Display scFv on Mammalian cells using pMAT6

The cross-species display function of pMAT6 in mammalian cells can be determined by the following protocol. COS 6 cells are grown on coverslip in 6-well plates with Dulbecco's modified Eagle's medium supplemented with 10% fetal bovine serum, 100 units/ml penicillin G, 100 µg/ml streptomycin. pAMT6 cross display vector and mammalian helper vector pMAG2 are co-transfected into COS 6 cells using FuGene 6 transfection reagent (Roche Applied Science) according to the manufacturer's instructions. Briefly, 0800 ng of plamid DNA (400 ng of pMAT6 + 400 ng of pMAG2) is added to diluted FuGENE 6 reagent at 3:2 ratio of FuGene 6 reagent (ul):DNA complex (ug) in serum-free medium. The FuGENE reagent DNA complex is incubated for 15 min at room temperature and then added to the cells.

After 48 hr, HA tagged scFv/GR1 fusion protein (from pMAT6 vector) displayed on the cell surface can be detected with Alexa 488 conjugated anti-HA antibody. Briefly, COS 6 cells are fixed with 4 % formaldehyde for 20 min, blocked with 5% BSA in PBS for 30 min at 25 °C and then incubated with Alexa 488 conjugated anti-HA antibody (1:100 dilution, Invitrogen) in PBS (1:500) for 60 min. Cells are also stained with DAPI (Invitrogen) to visualize the nucleus. Cells are observed under a Zeiss Axiovert 135 microscope with Plan-Neofluar 100/1.30 oil objective lens. HA-tagged scFv-GR1 on the cell surface will be observed in green fluorescence.

Alternatively a FACS assay can be performed to identify host cells that are expressing a polypeptide of interest. 48 hours after transfection, a final concentration of 4 x 10⁶ cells/ml is used for staining. Biotinylated rhVEGF or VEGF-Fc is added to 25 ul of the cell suspension for 60 min incubation at 4 °C. As a negative staining control, an identical sample of cells is stained with biotinylated soybean trypsin inhibitor. Cells are then incubated with avidin-FITC or anti-Fc antibody-FITC for a further 30 min at 4 oC in the dark. Cells are washed twice with RDF1 buffer and resuspend the cells in 0.2 mL RDF1 buffer for flow cytometry analysis in an FACSvantage flow cytometer (BD Biosciences) or Agilent 2100 bioanalyzer (Agilent Technologies).

### Example 27: Selection of desired Polypeptides displayed on yeast and mammalian cells

A library of DNAs can be inserted into pMAT4 vector, and directly transformed into yeast cells comprising a copy of yeast helper vector pMAT3 or pMAT7 or pMAT8 integrated into its genome.

Transformed yeast cells displaying leads with desired properties can be isolated by performing rounds of flow cytometric sorting. Briefly, the yeast cells can be grown in SD-CAA selective medium for 18-22 h at 30°C, and then transferred to SG/ R-CAA expression medium for 16-18 h at 20C. Yeast cells are incubated with biotinylated antigen and 5 mg/ml anti-HA (12CA5) antibody for 30 min at room temperature in FACS wash buffer (PBS/0.5% BSA/2 mM EDTA), and washed with ice cold wash buffer. Cell samples are probed by incubation with 5 mg/ml SA-PE and GaM-488 for 30 min on ice in the dark, and resuspended in FACS wash buffer for sorting by flow cytometry.

Selections can be performed using a BD Bioscience FACS Vantage DiVa set for purifying selection, and sort gates will be determined to select the desired double positive cells. Collected cells will be plated on SD-CAA plates with Penn/Strep and grown at 30°C for 2 days. Cells are then resuspended and amplified for the next round. After 2-3 round selection, a pool of leads of vector DNAs from the yeast cells can be recovered by using the Zymoprep yeast plasmid miniprep kit (Zymo Research).

This resulting pool of vector DNAs isolated from the yeast display library can then be directly transfected into mammalian cells for cell surface display by co-transfection with mammalian helper vector pMAG2. The leads with desired properties can be identified and collected a by few round flow cytometry sorting. Briefly, 10-20 ug DNA is mix with 3X106 of HEK 293 or COS 6 cells washed with ice-cold PBS in a cuvette. After 10 min incubation on ice, the cell/DNA mixture is electroporated with GenePulser Xcell (Bio-Rad) set at 250 uF, 660 V. After 10 min incubation at room temperature, cells are transferred to 20 ml medium in 75 cm culture flask, and incubated at 3 °C, 5% CO2 for 2-3 days. Alternatively, FuGene 6 transfection reagent can be used for transfections.

A suitable selection process described below. Approximately transfected 107 cells will be incubated with 0.2 nM biotinylated antigen protein and 20 ug/ml of anti-HA mAb 12CA5 in PBS buffer at 25°C for1 h. Finally, the cells were rinsed with ice-cold PBS buffer, and labeled with FITC-labeled goat anti-mouse FITC (AMI0408; BioSource International, Camarillo, CA) and streptavidin-R-PE conjugate (S866; Molecular Probes) by 1 hour incubation at 4C. The cells are thus sorted on a FACSVantageSE (BD Biosciences) with a proper sort window. The top 0.1 % PE-positive cells are collected. The DNAs of both display and helper vectors recovered from selected cells are transformed into E. *coli* cells. Only display vectors with an f1 ori can be packaged by regular helper phage such as KO7, which will have the effect of separating the vectors from from mammalian helper vector. The recovered display vector DNAs can then be used for another round FACS sorting or for DNA sequencing.

### Example 28: Selection of Signal Peptides with Function in E. coli, Yeast and Mammalian Cells

In pMAT6 vector, expression of the adapter GR1/polypeptide fusion protein is driven by three promoters: SV40 promoter for mammalian cells; pGAL10 promoter for yeast cells; and pLac promoter for bacterial cells. Therefore, if the vector includes a signal peptide which functions (i.e., directs the fusion protein to the secretory pathway) in all *E. coli,* yeast and mammalian cells this vector could potentially be used to direct the cross-species display of polypeptides on phage, yeast and mammalian cells.

In order to isolate a signal peptide with secretory functional in E. *coli,* yeast and mammalian cells; a library of signal sequences can be cloned into pMAT6 vector which includes an expression cassette for a test protein, such as anti-VEGF cFv. The resulting library can be displayed on phage and selected using a panning protocol using the VEGF antigen. The isolated phages which will have been identified base on their VEGF binding activity will be characterized by the presence of a signal peptide which functions in E. *coli* cells. A suitable panning process includes coating a 96-well plate with VEGF at a concentration of 5 ug/ml for overnight at 4°C, and washing the plate PBS and blocking with 5% milk/PBS. 100 ul 10 ¹¹⁻¹²phages are added and incubated for 2 hours at room temperature. After several washES with PBST and PBS, the bound phages are eluted with 10 ug/ml trypsin for 30 min. TG1 cells are infected with eluted phages, and used for next round phage preparation and panning. After repeating the process for 2-3 rounds, a pool of phages displaying can be enriched. The enriched phages are used to infect *E. coli* cells, which are used for the preparation of vector DNAs for next yeast display.

This small pool of vector DNAs selected from phage library can subsequently be directly transformed into yeast cells bearing a copy of yeast helper vector pMAT3 or pMAT7 or pMAT8. In order to display functional scFv on yeast cell surface, signal peptides that can direct the secretion in yeast cells are needed. The yeast cells with functional signal peptides can be isolated by performing a few rounds of flow cytometric sorting. A suitable sorting protocol could involve growing yeast cells in SD-CAA selective medium for 18-22 h at 30°C, then transferring to SG/ R-CAA expression medium for 16-18 h at 20°C. The cells can be incubated with biotinylated VEGF and 5 mg/ml anti-HA (12CA5) antibody for 30 min at room temperature in FACS wash buffer (PBS/0.5% BSA/2 mM EDTA), and washed with ice cold wash buffer. The cells can subsequently be probed by incubation with 5 mg/ml SA-PE and goat anti-mouse Ab-488 for 30 min on ice in the dark, and resuspended in FACS wash buffer for sorting by flow cytometry. Selections can be performed using a BD Bioscience FACS Vantage DiVa set for purifying selection, and sort gates will be determined to select the desired double positive cells. Collected cells are plated on SD-CAA plates with Penn/Strep and grown at 30 °C for 2 days. Cells are then resuspended and amplified for the next round. After 2-3 round selections, the vector DNA from the yeast cells are recovered by using the Zymoprep yeast plasmid miniprep kit (Zymo Research) according to the instruction.

After the above-described phage and yeast selection protocols have been used to identify a signal peptide that functions in E. *coli* and yeast, a signal peptide characterized by the additional feature of being able to function in mammalian cells can be identified by performing a third selection by introducing the library into a mammalian display system. The lead DNAs selected from phage and yeast display can be directly transfected into mammalian cells for surface display. Briefly, 20 ug of lead DNA are transfected into HEK 293 cells or COS 6 cells described above. After two days of culture, transfected cells will be incubated with 0.2 nM biotinylated antigen VEGF and 20 ug/ml of anti-HA mAb 12CA5 in PBS buffer at 25°C for1 h. Finally, the cells were rinsed with ice-cold PBS buffer, and labeled with FITC-labeled goat anti-mouse FITC (AMI0408; BioSource International, Camarillo, CA) and streptavidin-R-PE conjugate (S866; Molecular Probes) by 1 hour incubation at 4°C. The cells are thus sorted on a FACSVantageSE (BD Biosciences) with a proper sort window. The top 0.1% PE-positive cells are collected. The DNAs of both display and helper vectors recovered from selected cells are transformed into E. coli cells. The display vector DNA with f1 ori can be isolated by phage package. After those selections, the signal peptides isolated though phage, yeast, and mammalian display are functional in E. *coli*, yeast, and mammalian cells. To confirm it, the DNA from individual clone is directly used to transform into E. *coli,* yeast, and mammalian cells respectively. The soluble scFv secreted from those cells can be detected by anti-VEGF ELISA as described above.

### CROSS-SPECIES DISPLAY ON PROKARYOTIC AND EUKARYOTIC HOST CELLS

### Example 29: Bacterial helper vector pMAL1

pMAL1, which is depicted in Figure 13A was created from a fully synthetic DNA fragment of 3822 bp, by self ligation with PciI site. This fully synthetic DNA comprises (1) sequences for bacterial regulatory elements including pUC ori for replication, f1 ori for phage package, and expression cassette of chloramphenical selection marker; (2) an expression cassette of adapter 2 (GR2) (SEQ ID: 20) fusion with bacterial out surface domain (PelB signal-Lpp-OmpA). Briefly, the synthetic DNA was divided into 2 pieces of segments of 1851 and 2019 bp for gene synthesis by using Codon Devices' BioFab platform technology. The errors generated from oligo synthesis were corrected by oligo selection with sequences complementary to the synthetic genes, and affinity purification of Mut-S protein column. These synthetic DNA segment with PciI restriction site at two ends was digested and ligated. The resulting vector (SEQ ID NO: 17) was confirmed by standard DNA sequencing.

### Example 30: Bacterial Helper Vector pMAL2

pMAL2, which is depicted graphically in Figure13B, was derived from the pMAL1 vector by replacing PciI-BssHII fragment with a fully synthetic DNA fragment of 1006 bp, which comprises an expression cassette for the bacterial outer surface domain of Lpp-OmpA fused to adapter 2 (GR2) (SEQ ID NO: 20). Expression of the fusion protein is directed by phage p8 signal peptide.

Briefly, the synthetic DNA was generated by using Codon Devices' BioFab platform technology. The errors generated from oligo synthesis were corrected by oligo selection with sequences complementary to the synthetic genes, and affinity purification of Mut-S protein column. The resulting vector (SEQ ID NO: 18) was confirmed by standard DNA sequencing.

### Example 31: Cross-species Display of scFv on E.coli and Mammalian Host Cells

As shown above vector pMAG9 was used for the expression of polypetides in multiple species of host cell, and when used in combination with helper display vectors of the invention, to direct the surface display of protein libriares on phage and mammalian cells described in example 2, 6 to 9. Similarly, this vector can also be used for cross-display on the bacterial and mammalian cells. The bacterial cell display can be done as follows.

Co-transformation of the expression vector pMAG9 vector in combination with bacterial helper display vector pMAL1 or pAML2, will direct E. *coli* to express two fusion proteins: soluble scFv-GR1 (protein/adapter 1) and Lpp-OmpA-GR2 (surface protein/adapter 2). The GR1 and GR2 fusion proteins secrete into periplamic space, and will assemble to form a protein complex by GR1 and GR2 interaction. The resulting protein complex is displayed on the cell surface by virtue of the Lpp-OmpA outer membrane anchor domain.

Briefly, cells can be transformed with the pMAG9 expression vector with ampicillin selection marker, are then infected with phagemid packaging helper vector pMAL1 or pMAL2 with chloramphenical selection marker. The cells with both display and helper vectors will grown in 2YT medium containing 1% glucose and antibiotics of 100 ug/ml Ampicillin and 30 ug/ml chloramphenical until OD₆₀₀ =0.5, then transferred to growth medium without glucose for expressions of adapter fusion proteins. After 4-6 hours incubation, cells are incubated with biotinylated antigen VEGF and 5 mg/ml anti-HA (12CA5) antibody for 30 min at room temperature in FACS wash buffer (PBS/0.5% BSA/2 mM EDTA), and washed with ice cold wash buffer. Thus cells are probed by incubation with 5 mg/ml SA-PE and GaM-488 for 30 min on ice in the dark, and resuspended in FACS wash buffer for FACS analysis. When a library of anit-VEGF scFv is displayed with this method, cells displaying scFv proteins may be probed as described above, and sorting by flow cytometry. The leads of display vectors can be separated from helper vector through growth in 2YT/glucose medium with antibiotics ampicillin only.

### Example 32: Cross-species Display of scFv between E.coli and Yeast

As shown herein pMAT5 can be used for cross display between phage and yeast cells (described in example 12 to 19), and for cell surface display between bacterial and yeast cells. Recipient host cells that are co-transformed with pMAT6 vector and bacterial helper vector pMAL1 or pAML2, cells then express two fusion proteins: soluble scFv-GR1 and Lpp-OmpA-GR2 fusion. GR1 and GR2 fusion proteins secrete into periplamic space, and form a protein complex by GR1 and GR2 interaction. The scFv-GR1 protein will be displayed on the cell surface through Lpp-OmpA out membrane domain.

Briefly, cells can be initially transformed with a display vector comprising an ampicillin selection marker, and subsequently infected with phagemid packaging helper vector pMAL1 or pMAL2 with chloramphenical selection marker. The cells with both display and helper vectors will grown in 2YT medium containing 1% glucose and antibiotics of 100 ug/ml Ampicillin and 30 ug/ml chloramphenical until OD₆₀₀ =0.5, then transferred to growth medium without glucose for expressions of adapter fusion proteins. After 4-6 hours incubation, cells are incubated with biotinylated antigen VEGF and 5 mg/ml anti-HA (12CA5) antibody for 30 min at room temperature in FACS wash buffer (PBS/0.5% BSA/2 mM EDTA), and washed with ice cold wash buffer. Thus cells are probed by incubation with 5 mg/ml SA-PE and GaM-488 for 30 min on ice in the dark, and resuspended in FACS wash buffer for FACS analysis. When a library of anit-VEGF scFv is displayed with this method, cells display scfv proteins can be probed as described above, and sorting by flow cytometry. The leads of display vectors can be separated from helper vector through growth in 2YT/glucose medium with antibiotics ampicillin only. pMAT5 display vector for yeast cell display is described in example 19.

## Claims

1. A cross-species display system for the sequential display of a repertoire of polypeptide sequences of interest in a prokaryotic host and a eukaryotic host cell without having to manipulate the expression vectors encoding the polypeptide sequences of interest comprising a cross-species display vector set consisting of 1) a cross-species expression vector comprising one or more promoters and a cross-species expression cassettes encoding proteins of interest fused in frame to a first adapter sequence, 2) a first helper vector encoding a fusion protein consisting of a second adapter sequence which interacts in a pair-wise manner with the first adapter sequence of the display vector fused to an outer surface protein expressed by the prokaryotic host, and 3) a second helper vector encoding a fusion protein consisting of the second adapter sequence fused to an outer surface protein expressed by the eukaryotic host cell.

2. The cross-species display system of claim 1 wherein the repertoire of polypeptide sequences of interest is an antibody library.

3. The cross-species display system of claim 2 wherein the prokaryotic host is phage and the eukaryotic host is yeast.

4. The display system of claim 3 wherein the cross-species expression vector is selected from pMAG1, pMAG9, pMAT2, pMAT4, pMAT6, or pMAT5 and the first and second helper vectors are selected from GMCT, pMAG2, pMAL1, pMAL2, pMAT3, pMAT7 and pMAT8.

5. The cross-species display system of claim 4 wherein the cross-species expression set comprises
a) the display vector pMAT5, b) helper vector GMCT and c) a second yeast helper vector selected from pMAT3, pMAT7, or pMAT8.

6. The display system of claim 2 wherein the first and second adapter sequences are derived from coiled coil domains.

7. The display system of claim 6 wherein the first adapter sequence consists of SEQ ID NO:19 and the second adapter sequence consists of SEQ ID NO: 20.

8. The display system of claim 2 wherein the prokaryotic host is phage and the eukaryotic host is mammalian and further wherein the cross-species vector set comprises the display vector pAMG9 and helper vectors GMCT and pMAG2.

9. A yeast host cell transformed with a helper vector selected from the group pMAT3, pMAT7, or pMAT8.

10. A kit comprising the cross-species display vector set according to claim 1 in suitable packaging.

11. A multi-species display system comprising 1) a multi-species expression vector comprising one or more promoters, expression cassettes encoding proteins of interest fused in frame to a first adapter sequence 2) a helper vector encoding a fusion protein consisting of a second adapter sequence which interacts in a pair-wise manner with the first adapter sequence fused to an outer surface protein expressed by a species of host cells selected for display, and 3) at least two different species of host cells.

12. The display system of claim 11 wherein the proteins of interest are an antibody library.

13. The display system of claim 11 wherein the first and second adapter sequences are derived from coiled coil domains.

14. The display system of claim 13 wherein the first adapter sequence consists of SEQ ID NO:19 and the second adapter sequence consists of SEQ ID NO: 20.

15. The multi-species display system of claim 12 wherein the two different species of host cells are prokaryotic.

16. The display system of claim 15 wherein the display vector is selected from pMAG1 or pMAG9.

17. The display system of claim 3 wherein the cross-species expression vector is selected from pMAG1, pMAG9, pMAT2, pMAT4, pMAT6, or pMAT5 and the first and second helper vectors are selected from GMCT, pMAG2, pMAL1, pMAL2, pMAT3, pMAT7 and pMAT8.

18. The display system of claim 11 wherein the two different species of host cells are eukaryotic.

19. The display system of claim 18 wherein the display vector is selected from pMAG1 (SEQ ID NO: 1) or pMAG9 (SEQ. ID. NO: 2).

20. The display system of claim 18 wherein the display vector is pMAG9, the helper vector is pMAG2 and the host cells are yeast and mammalian.


SEQUENCE LISTING

<110> Abmaxis, Inc.

<120> MULTI SPECIES DISPLAY SYSTEMS

<130> AX0006Y PCT

<150> 60/746,489
<151> 2006-05-04

<160> 20

<170> FastSEQ for Windows Version 4.0

<210> 1
<211> 6081
<212> DNA
<213> Artificial Sequence

<220>
<223> Vector pMAG1

<400> 1
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cgcgttaaat ttttgttaaa 420
tcagctcatt ttttaaccaa taggccgaaa tccccaaaat cccttataaa tcaaaagaat 480
agaccgagat agggttgagt gttgttccag tttggaacaa gagtccacta ttaaagaacg 540
tggactccaa cgtcaaaggg cgaaaaaccg tctatcaggg cgatggccca ctacgtgaac 600
catcacccta atcaagtttt ttggggtcga ggtgccgtaa agcactaaat cggaacccta 660
aagggatgcc ccgatttaga gcttgacggg gaaagccggc gaacgtggcg agaaaggaag 720
ggaagaaagc gaaaggagcg ggcgctaggg cgctggcaag tgtagcggtc acgctgcgcg 780
taaccaccac acccgccgcg cttaatgcgc cgctacaggg cgcgtttaat taactctagt 840
tattaatagt aatcaattac ggggtcatta gttcatagcc catatatgga gttccgcgtt 900
acataactta cggtaaatgg cccgcctggc tgaccgccca acgacccccg cccattgacg 960
tcaataatga cgtatgttcc catagtaacg ccaataggga ctttccattg acgtcaatgg 1020
gtggagtatt tacggtaaac tgcccacttg gcagtacatc aagtgtatca tatgccaagt 1080
acgcccccta ttgacgtcaa tgacggtaaa tggcccgcct ggcattatgc ccagtacatg 1140
accttatggg actttcctac ttggcagtac atctacgtat tagtcatcgc tattaccatg 1200
catggtcgag gtgagcccca cgttctgctt cactctcccc atctcccccc cctccccacc 1260
cccaattttg tatttattta ttttttaatt attttgtgca gcgatggggg cggggggggg 1320
gggggggcgc gcgccaggcg gggcggggcg gggcgagggg cggggcgggg cgaggcggag 1380
aggtgcggcg gcagccaatc agagcggcgc gctccgaaag tttcctttta tggcgaggcg 1440
gcggcggcgg cggccctata aaaagcgaag cgcgcggcgg gcgggagtcg tgcgcgctgc 1500
cttcgccccg tgccccgctc cgccgccgcc tcgcgccgcc cgccccggct ctgactgacc 1560
gcgttactcc cacaggtgag cgggcgggac ggcccttctc cttcgggctg taattagcgc 1620
ttggtttaat gacggcttgt ttcttttctg tggctgcgtg aaagccttga ggggctccgg 1680
gagggccctt tgtgcggggg gagcggctcg gggctgtccg cggggggacg gctgccttcg 1740
ggggggacgg ggcagggcgg ggttcggctt ctggcgtgtg accggcggct ctagagcctc 1800
tgctaaccat gttcatgcct tcttcttttt cctacagctc ctgggcaacg tgctggttat 1860
tgtgctgtct catcattttg gcaaagaatt ggatcggacc gaagcttgcg caacgcaatt 1920
aatgtgagtt agctcactca ttaggcaccc caggctttac actttatgct tccggctcgt 1980
atgttgtgtg gaattgtgag cggataacaa tttcacacta aggaggttta aaccatggct 2040
acaggctccc ggacgagtct gctcctggct tttggcctgc tctgcctgcc ctggcttcaa 2100
gagggatccg cgagctccgg tacctcgagc gcggccgctt atccatacga cgtaccagac 2160
tacgcaggag gtcatcacca tcatcaccat gtcgacggat ctggaggagg tgaggagaag 2220
tcccggctgt tggagaagga gaaccgtgaa ctggaaaaga tcattgctga gaaagaggag 2280
cgtgtctctg aactgcgcca tcaactccag tctgtaggag gttgttaata agtcgactaa 2340
tgaagatcta ttaacctcag gtgcaggctg cctatcagaa ggtggtggct ggtgtggcca 2400
atgccctggc tcacaaatac cactgagatc gatctttttc cctctgccaa aaattatggg 2460
gacatcatga agccccttga gcatctgact tctggctaat aaaggaaatt tattttcatt 2520

gcaatagtgt gttggaattt tttgtgtctc tcactcggaa ggacatatgg gagggcaaat 2580
catttaaaac atcagaatga gtttttggtt tagagtttgg caacatatgc ccatatgtaa 2640
ctagcataac cccttggggc ctctaaacgg gtcttgaggg gttttttgat atccagacat 2700
gataagatac attgatgagt ttggacaaac cacaactaga atgcagtgaa aaaaatgctt 2760
tatttgtgaa atttgtgatg ctattgcttt atttgtaacc attataagct gcaataaaca 2820
agttggggtg ggcgaagaac tccagcatga gatccccgcg ctggaggatc atccagccgg 2880
cgtcccggaa aacgattccg aagcccaacc tttcatagaa ggcggcggtg gaatcgaaat 2940
ctcgtgatgg caggttgggc gtcgcttggt cggtcatttc gcgaacccca gagtcccgct 3000
cagaagaact cgtcaagaag gcgatagaag gcgatgcgct gcgaatcggg agcggcgata 3060
ccgtaaagca cgaggaagcg gtcagcccat tcgccgccaa gctcttcagc aatatcacgg 3120
gtagccaacg ctatgtcctg atagcggtcc gccacaccca gccggccaca gtcgatgaat 3180
ccagaaaagc ggccattttc caccatgata ttcggcaagc aggcatcgcc atgggtcacg 3240
acgagatcct cgccgtcggg catgcgcgcc ttgagcctgg cgaacagttc ggctggcgcg 3300
agcccctgat gctcttcgtc cagatcatcc tgatcgacaa gaccggcttc catccgagta 3360
cgtgctcgct cgatgcgatg tttcgcttgg tggtcgaatg ggcaggtagc cggatcaagc 3420
gtatgcagcc gccgcattgc atcagccatg atggatactt tctcggcagg agcaaggtga 3480
gatgacagga gatcctgccc cggcacttcg cccaatagca gccagtccct tcccgcttca 3540
gtgacaacgt cgagcacagc tgcgcaagga acgcccgtcg tggccagcca cgatagccgc 3600
gctgcctcgt cctgcagttc attcagggca ccggacaggt cggtcttgac aaaaagaacc 3660
gggcgcccct gcgctgacag ccggaacacg gcggcatcag agcagccgat tgtctgttgt 3720
gcccagtcat agccgaatag cctctccacc caagcggccg gagaacctgc gtgcaatcca 3780
tcttgttcaa tcatgcgaaa cgatcctcat cctgtctctt gatcagatcc gaaaatggat 3840
atacaagctc ccgggagctt tttgcaaaag cctaggcctc caaaaaagcc tcctcactac 3900
ttctggaata gctcagaggc agaggcggcc tcggcctctg cataaataaa aaaaattagt 3960
cagccatggg gcggagaatg ggcggaactg ggcggagtta ggggcgggat gggcggagtt 4020
aggggcggga ctatggttgc tgactaattg agatgcatgc tttgcatact tctgcctgct 4080
ggggagcctg gggactttcc acacctggtt gctgactaat tgagatgcat gctttgcata 4140
cttctgcctg cctggggagc ctggggactt tccacaccct aactgacaca cattccacag 4200
acatgtgagc aaaaggccag caaaaggcca ggaaccgtaa aaaggccgcg ttgctggcgt 4260
ttttccatag gctccgcccc cctgacgagc atcacaaaaa tcgacgctca agtcagaggt 4320
ggcgaaaccc gacaggacta taaagatacc aggcgtttcc ccctggaagc tccctcgtgc 4380
gctctcctgt tccgaccctg ccgcttaccg gatacctgtc cgcctttctc ccttcgggaa 4440
gcgtggcgct ttctcatagc tcacgctgta ggtatctcag ttcggtgtag gtcgttcgct 4500
ccaagctggg ctgtgtgcac gaaccccccg ttcagcccga ccgctgcgcc ttatccggta 4560
actatcgtct tgagtccaac ccggtaagac acgacttatc gccactggca gcagccactg 4620
gtaacaggat tagcagagcg aggtatgtag gcggtgctac agagttcttg aagtggtggc 4680
ctaactacgg ctacactaga aggacagtat ttggtatctg cgctctgctg aagccagtta 4740
ccttcggaaa aagagttggt agctcttgat ccggcaaaca aaccaccgct ggtagcggtg 4800
gtttttttgt ttgcaagcag cagattacgc gcagaaaaaa aggatctcaa gaagatcctt 4860
tgatcttttc tacggggtct gacgctcagt ggaacgaaaa ctcacgttaa gggattttgg 4920
tcatgagatt atcaaaaagg atcttcacct agatcctttt aaattaaaaa tgaagtttta 4980
aatcaatcta aagtatatat gagtaaactt ggtctgacag ttaccaatgc ttaatcagtg 5040
aggcacctat ctcagcgatc tgtctatttc gttcatccat agttgcctga ctccccgtcg 5100
tgtagataac tacgatacgg gagggcttac catctggccc cagtgctgca atgataccgc 5160
gagacccacg ctcaccggct ccagatttat cagcaataaa ccagccagcc ggaagggccg 5220
agcgcagaag tggtcctgca actttatccg cctccatcca gtctattaat tgttgccggg 5280
aagctagagt aagtagttcg ccagttaata gtttgcgcaa cgttgttgcc attgctacag 5340
gcatcgtggt gtcacgctcg tcgtttggta tggcttcatt cagctccggt tcccaacgat 5400
caaggcgagt tacatgatcc cccatgttgt gcaaaaaagc ggttagctcc ttcggtcctc 5460
cgatcgttgt cagaagtaag ttggccgcag tgttatcact catggttatg gcagcactgc 5520
ataattctct tactgtcatg ccatccgtaa gatgcttttc tgtgactggt gagtactcaa 5580
ccaagtcatt ctgagaatag tgtatgcggc gaccgagttg ctcttgcccg gcgtcaatac 5640
gggataatac cgcgccacat agcagaactt taaaagtgct catcattgga aaacgttctt 5700
cggggcgaaa actctcaagg atcttaccgc tgttgagatc cagttcgatg taacccactc 5760
gtgcacccaa ctgatcttca gcatctttta ctttcaccag cgtttctggg tgagcaaaaa 5820
caggaaggca aaatgccgca aaaaagggaa taagggcgac acggaaatgt tgaatactca 5880
tactcttcct ttttcaatat tattgaagca tttatcaggg ttattgtctc atgagcggat 5940
acatatttga atgtatttag aaaaataaac aaataggggt tccgcgcaca tttccccgaa 6000
aagtgccacc tgacgtctaa gaaaccatta ttatcatgac attaacctat aaaaataggc 6060
gtatcacgag gccctttcgt c 6081

<210> 2
<211> 6804
<212> DNA
<213> Artificial Sequence

<220>
<223> Vector pMAG9

<400> 2
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cgcgttaaat ttttgttaaa 420
tcagctcatt ttttaaccaa taggccgaaa tcggcaaaat cccttataaa tcaaaagaat 480
agaccgagat agggttgagt gttgttccag tttggaacaa gagtccacta ttaaagaacg 540
tggactccaa cgtcaaaggg cgaaaaaccg tctatcaggg cgatggccca ctacgtgaac 600
catcacccta atcaagtttt ttggggtcga ggtgccgtaa agcactaaat cggaacccta 660
aagggatgcc ccgatttaga gcttgacggg gaaagccggc gaacgtggcg agaaaggaag 720
ggaagaaagc gaaaggagcg ggcgctaggg cgctggcaag tgtagcggtc acgctgcgcg 780
taaccaccac acccgccgcg cttaatgcgc cgctacaggg cgcgtttaat taactctagt 840
tattaatagt aatcaattac ggggtcatta gttcatagcc catatatgga gttccgcgtt 900
acataactta cggtaaatgg cccgcctggc tgaccgccca acgacccccg cccattgacg 960
tcaataatga cgtatgttcc catagtaacg ccaataggga ctttccattg acgtcaatgg 1020
gtggagtatt tacggtaaac tgcccacttg gcagtacatc aagtgtatca tatgccaagt 1080
acgcccccta ttgacgtcaa tgacggtaaa tggcccgcct ggcattatgc ccagtacatg 1140
accttatggg actttcctac ttggcagtac atctacgtat tagtcatcgc tattaccatg 1200
catggtcgag gtgagcccca cgttctgctt cactctcccc atctcccccc cctccccacc 1260
cccaattttg tatttattta ttttttaatt attttgtgca gcgatggggg cggggggggg 1320
gggggggcgc gcgccaggcg gggcggggcg gggcgagggg cggggcgggg cgaggcggag 1380
aggtgcggcg gcagccaatc agagcggcgc gctccgaaag tttcctttta tggcgaggcg 1440
gcggcggcgg cggccctata aaaagcgaag cgcgcggcgg gcgggagtcg tgcgcgctgc 1500
cttcgccccg tgccccgctc cgccgccgcc tcgcgccgcc cgccccggct ctgactgacc 1560
gcgttactcc cacaggtgag cgggcgggac ggcccttctc cttcgggctg taattagcgc 1620
ttggtttaat gacggcttgt ttcttttctg tggctgcgtg aaagccttga ggggctccgg 1680
gagggccctt tgtgcggggg gagcggctcg gggctgtccg cggggggacg gctgccttcg 1740
ggggggacgg ggcagggcgg ggttcggctt ctggcgtgtg accggcggct ctagagcctc 1800
tgctaaccat gttcatgcct tcttcttttt cctacagctc ctgggcaacg tgctggttat 1860
tgtgctgtct catcattttg gcaaagaatt ggatcggacc gaagcttgcg caacgcaatt 1920
aatgtgagtt agctcactca ttaggcaccc caggctttac actttatgct tccggctcgt 1980
atgttgtgtg gaattgtgag cggataacaa tttcacacta aggaggttta aaccatggct 2040
acaggctccc ggacgagtct gctcctggct tttggcctgc tctgcctgcc ctggcttcaa 2100
gagggatccg cgagctccga ggtgcagctg gtgcagagcg gcggcggcgt ggtgcagccg 2160
ggcggcagcc tgcgtctgag ctgcgccgcg agcggctaca ccttcaccaa ctacggcatg 2220
aactggattc gtcaggcccc cgggaagggc ctggagtggg tgggctggat caacacctac 2280
accggcgagc cgacctacgc agctgacttc aagcgtcgtg tcaccttcag cctcgacacc 2340
agcaagagca cggcgtacct gcaactgaac agcctgaggg ccgaggacac tgcagtttac 2400
tactgcgcga aatacccgta ctactacggt cgtagccact ggtacttcga cgtctggggc 2460
caagggaccc ttgtcaccgt ctcgagcggc ggtggcggtt ctggtggtgg tggctctggt 2520
ggcggcggat ccgatatcgt gatgacccag agcccgagca ccctgagcgc gagtccgggt 2580
gagcgcgcga ccatcacctg cagtgcgagc cagagcatca gcacctacct ggcgtggtat 2640
cagcagaaac caggtcaagc gccgcaagtg ctgatctacg ctgcgagcaa cctggcgtcc 2700
ggagtgccga accgtttcag cggtagccgt agcgggaccg atttcaccct gaccatcagc 2760
agcttgcagc cggaagactt cgcggtgtac tactgccagc agtactacag caccccgtgg 2820
accttcggtg gtggtaccaa agtggaaatc aaagcggccg cttatccata cgacgtacca 2880
gactacgcag gaggtcatca ccatcatcac catgtcgacg gatctggagg aggtgaggag 2940
aagtcccggc tgttggagaa ggagaaccgt gaactggaaa agatcattgc tgagaaagag 3000
gagcgtgtct ctgaactgcg ccatcaactc cagtctgtag gaggttgtta ataagtcgac 3060
taatgaagat ctattaacct caggtgcagg ctgcctatca gaaggtggtg gctggtgtgg 3120
ccaatgccct ggctcacaaa taccactgag atcgatcttt ttccctctgc caaaaattat 3180
ggggacatca tgaagcccct tgagcatctg acttctggct aataaaggaa atttattttc 3240
attgcaatag tgtgttggaa ttttttgtgt ctctcactcg gaaggacata tgggagggca 3300
aatcatttaa aacatcagaa tgagtttttg gtttagagtt tggcaacata tgcccatatg 3360
taactagcat aaccccttgg ggcctctaaa cgggtcttga ggggtttttt gatatccaga 3420
catgataaga tacattgatg agtttggaca aaccacaact agaatgcagt gaaaaaaatg 3480
ctttatttgt gaaatttgtg atgctattgc tttatttgta accattataa gctgcaataa 3540
acaagttggg gtgggcgaag aactccagca tgagatcccc gcgctggagg atcatccagc 3600
cggcgtcccg gaaaacgatt ccgaagccca acctttcata gaaggcggcg gtggaatcga 3660
aatctcgtga tggcaggttg ggcgtcgctt ggtcggtcat ttcgcgaacc ccagagtccc 3720
gctcagaaga actcgtcaag aaggcgatag aaggcgatgc gctgcgaatc gggagcggcg 3780
ataccgtaaa gcacgaggaa gcggtcagcc cattcgccgc caagctcttc agcaatatca 3840

cgggtagcca acgctatgtc ctgatagcgg tccgccacac ccagccggcc acagtcgatg 3900
aatccagaaa agcggccatt ttccaccatg atattcggca agcaggcatc gccatgggtc 3960
acgacgagat cctcgccgtc gggcatgcgc gccttgagcc tggcgaacag ttcggctggc 4020
gcgagcccct gatgctcttc gtccagatca tcctgatcga caagaccggc ttccatccga 4080
gtacgtgctc gctcgatgcg atgtttcgct tggtggtcga atgggcaggt agccggatca 4140
agcgtatgca gccgccgcat tgcatcagcc atgatggata ctttctcggc aggagcaagg 4200
tgagatgaca ggagatcctg ccccggcact tcgcccaata gcagccagtc ccttcccgct 4260
tcagtgacaa cgtcgagcac agctgcgcaa ggaacgcccg tcgtggccag ccacgatagc 4320
cgcgctgcct cgtcctgcag ttcattcagg gcaccggaca ggtcggtctt gacaaaaaga 4380
accgggcgcc cctgcgctga cagccggaac acggcggcat cagagcagcc gattgtctgt 4440
tgtgcccagt catagccgaa tagcctctcc acccaagcgg ccggagaacc tgcgtgcaat 4500
ccatcttgtt caatcatgcg aaacgatcct catcctgtct cttgatcaga tccgaaaatg 4560
gatatacaag ctcccgggag ctttttgcaa aagcctaggc ctccaaaaaa gcctcctcac 4620
tacttctgga atagctcaga ggcagaggcg gcctcggcct ctgcataaat aaaaaaaatt 4680
agtcagccat ggggcggaga atgggcggaa ctgggcggag ttaggggcgg gatgggcgga 4740
gttaggggcg ggactatggt tgctgactaa ttgagatgca tgctttgcat acttctgcct 4800
gctggggagc ctggggactt tccacacctg gttgctgact aattgagatg catgctttgc 4860
atacttctgc ctgcctgggg agcctgggga ctttccacac cctaactgac acacattcca 4920
cagacatgtg agcaaaaggc cagcaaaagg ccaggaaccg taaaaaggcc gcgttgctgg 4980
cgtttttcca taggctccgc ccccctgacg agcatcacaa aaatcgacgc tcaagtcaga 5040
ggtggcgaaa cccgacagga ctataaagat accaggcgtt tccccctgga agctccctcg 5100
tgcgctctcc tgttccgacc ctgccgctta ccggatacct gtccgccttt ctcccttcgg 5160
gaagcgtggc gctttctcat agctcacgct gtaggtatct cagttcggtg taggtcgttc 5220
gctccaagct gggctgtgtg cacgaacccc ccgttcagcc cgaccgctgc gccttatccg 5280
gtaactatcg tcttgagtcc aacccggtaa gacacgactt atcgccactg gcagcagcca 5340
ctggtaacag gattagcaga gcgaggtatg taggcggtgc tacagagttc ttgaagtggt 5400
ggcctaacta cggctacact agaaggacag tatttggtat ctgcgctctg ctgaagccag 5460
ttaccttcgg aaaaagagtt ggtagctctt gatccggcaa acaaaccacc gctggtagcg 5520
gtggtttttt tgtttgcaag cagcagatta cgcgcagaaa aaaaggatct caagaagatc 5580
ctttgatctt ttctacgggg tctgacgctc agtggaacga aaactcacgt taagggattt 5640
tggtcatgag attatcaaaa aggatcttca cctagatcct tttaaattaa aaatgaagtt 5700
ttaaatcaat ctaaagtata tatgagtaaa cttggtctga cagttaccaa tgcttaatca 5760
gtgaggcacc tatctcagcg atctgtctat ttcgttcatc catagttgcc tgactccccg 5820
tcgtgtagat aactacgata cgggagggct taccatctgg ccccagtgct gcaatgatac 5880
cgcgagaccc acgctcaccg gctccagatt tatcagcaat aaaccagcca gccggaaggg 5940
ccgagcgcag aagtggtcct gcaactttat ccgcctccat ccagtctatt aattgttgcc 6000
gggaagctag agtaagtagt tcgccagtta atagtttgcg caacgttgtt gccattgcta 6060
caggcatcgt ggtgtcacgc tcgtcgtttg gtatggcttc attcagctcc ggttcccaac 6120
gatcaaggcg agttacatga tcccccatgt tgtgcaaaaa agcggttagc tccttcggtc 6180
ctccgatcgt tgtcagaagt aagttggccg cagtgttatc actcatggtt atggcagcac 6240
tgcataattc tcttactgtc atgccatccg taagatgctt ttctgtgact ggtgagtact 6300
caaccaagtc attctgagaa tagtgtatgc ggcgaccgag ttgctcttgc ccggcgtcaa 6360
tacgggataa taccgcgcca catagcagaa ctttaaaagt gctcatcatt ggaaaacgtt 6420
cttcggggcg aaaactctca aggatcttac cgctgttgag atccagttcg atgtaaccca 6480
ctcgtgcacc caactgatct tcagcatctt ttactttcac cagcgtttct gggtgagcaa 6540
aaacaggaag gcaaaatgcc gcaaaaaagg gaataagggc gacacggaaa tgttgaatac 6600
tcatactctt cctttttcaa tattattgaa gcatttatca gggttattgt ctcatgagcg 6660
gatacatatt tgaatgtatt tagaaaaata aacaaatagg ggttccgcgc acatttcccc 6720
gaaaagtgcc acctgacgtc taagaaacca ttattatcat gacattaacc tataaaaata 6780
ggcgtatcac gaggcccttt cgtc 6804

<210> 3
<211> 45
<212> DNA
<213> Artificial Sequence

<220>
<223> PCR Primer

<400> 3
agtcaggtaa gcgctcgcgc tccgaggtgc agctggtgca gagcg 45

<210> 4
<211> 27
<212> DNA
<213> Artificial Sequence

<220>
<223> PCR Primer

<400> 4
atgacctcct gcgtagtctg gtacgtc                                          27

<210> 5
<211> 32
<212> DNA
<213> Artificial Sequence

<220>
<223> PCR Primer

<400> 5
tttagtggta cctttctatt ctcactccgc tg                                    32

<210> 6
<211> 32
<212> DNA
<213> Artificial Sequence

<220>
<223> PCR Primer

<400> 6
tagaaaggta ccactaaagg aattgcgaat aa                                    32

<210> 7
<211> 57
<212> DNA
<213> Artificial Sequence

<220>
<223> KO7kpn Helper Vector

<400> 7
gtgaaaaaat tattattcgc aattccttta gtggtacctt tctattctca ctccgct    57

<210> 8
<211> 1467
<212> DNA
<213> Artificial Sequence

<220>
<223> GMCT Phage Vector

<400> 8
ggtaccggta cctttctatt ctcactccgc tacatcccgc ctggagggcc tacagtcaga 60
aaaccatcgc ctgcgaatga agatcacaga gctggataaa gacttggaag aggtcaccat 120
gcagctgcag gacgtcggag gttgcgcggc cgcagaacaa aaactgatct cagaagagga 180
tctgacgcgt gctggcggcg gctctggtgg tggttctggt ggcggctctg agggtggcgg 240
ctctgagggt ggcggttctg agggtggcgg ctctgagggt ggcggttccg gtggcggctc 300
cggttccggt gattttgatt atgaaaaaat ggcaaacgct aataaggggg ctatgaccga 360
aaatgccgat gaaaacgcgc tacagtctga cgctaaaggc aaacttgatt ctgtcgctac 420
tgattacggt gctgctatcg atggtttcat tggtgacgtt tccggccttg ctaatggtaa 480
tggtgctact ggtgattttg ctggctctaa ttcccaaatg gctcaagtcg gtgacggtga 540
taattcacct ttaatgaata atttccgtca atatttacct tccctccctc aatcggttga 600
atgtcgccct tttgtctttg gcgctggtaa accatatgaa ttttctattg attgtgacaa 660
aataaactta ttccgtggtg tctttgcgtt tcttttatat gttgccacct ttatgtatgt 720
attttctacg tttgctaaca tactgcgtaa taaggagtct taataaggcg cgccacaatt 780
tcacagtaag gaggtttaat aaatgaaaaa gacagctatt gcgattgcag tggcactggc 840
tggtttcgct accgtagcgc aggctagatc tggaggcggt actgttgaaa gttgtttagc 900
aaaacctcat acagaaaatt catttactaa cgtctggaaa gacgacaaaa ctttagatcg 960
ttacgctaac tatgagggct gtctgtggaa tgctacaggc gttgtggttt gtactggtga 1020
cgaaactcag tgttacggta catgggttcc tattgggctt gctatccctg aaaatgaggg 1080
tggtggctct gagggtggcg gttctgaggg tggcggttct gagggtggcg gtactaaacc 1140

tcctgagtac ggtgatacac ctattccggg ctatacttat atcaaccctc tcgacggcac 1200
ttatccgcct ggtactgagc aaaaccccgc taatcctaat ccttctcttg aggagtctca 1260
gcctcttaat actttcatgt ttcagaataa taggttccga aataggcagg gtgcattaac 1320
tgtttatacg ggcactgtta ctcaaggcac tgaccccgtt aaaacttatt accagtacac 1380
tcctgtatca tcaaaagcca tgtatgacgc ttactggaac ggtaaattca gagactgcgc 1440
tttccattct ggctttaatg aggatcc 1467

<210> 9
<211> 5416
<212> DNA
<213> Artificial Sequence

<220>
<223> Helper Vector pMAG2

<400> 9
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cggatcggga gatctcccga 420
tcccctatgg tcgactctca gtacaatctg ctctgatgcc gcatagttaa gccagtatct 480
gctccctgct tgtgtgttgg aggtcgctga gtagtgcgcg agcaaaattt aagctacaac 540
aaggcaaggc ttgaccgaca attgcatgaa gaatctgctt agggttaggc gttttgcgct 600
gcttcgcgat gtacgggcca gatatacgcg ttgacattga ttattgacta gttattaata 660
gtaatcaatt acggggtcat tagttcatag cccatatatg gagttccgcg ttacataact 720
tacggtaaat ggcccgcctg gctgaccgcc caacgacccc cgcccattga cgtcaataat 780
gacgtatgtt cccatagtaa cgccaatagg gactttccat tgacgtcaat gggtggacta 840
tttacggtaa actgcccact tggcagtaca tcaagtgtat catatgccaa gtacgccccc 900
tattgacgtc aatgacggta aatggcccgc ctggcattat gcccagtaca tgaccttatg 960
ggactttcct acttggcagt acatctacgt attagtcatc gctattacca tggtgatgcg 1020
gttttggcag tacatcaatg ggcgtggata gcggtttgac tcacggggat ttccaagtct 1080
ccaccccatt gacgtcaatg ggagtttgtt ttggcaccaa aatcaacggg actttccaaa 1140
atgtcgtaac aactccgccc cattgacgca aatgggcggt aggcgtgtac ggtgggaggt 1200
ctatataagc agagctctct ggctaactag agaacccact gcttactggc ttatcgaaat 1260
taatacgact cactataggg agacccaagc tggctagcac catgcgaccc tccgggacgg 1320
ccggggcagc gctcctggcg ctgctggctg cgctctgccc ggcgtctaga gctaccagcc 1380
gcctggaggg cctgcagagc gagaaccacc gcctgcgcat gaagatcacc gagctggaca 1440
aggacctgga ggaggtgacc atgcagctgc aggacgtggg cggctgcgcg gccgccgagc 1500
agaagctgat cagcgaggag gacctgaccg gtggaggctc cggaggaggt agcggatccg 1560
gtacgaatgg gcctaagatc ccgtccatcg ccactgggat ggtgggggcc ctcctcttgc 1620
tgctggtggt ggccctgggg atcggcctct tcatgcgaag gcgccacatc gttcggaagc 1680
gcacgctgcg gaggctgctg caggagaggg agcttgtgga gcctcttaca cccagttgat 1740
aagcttgttt aaacccgctg atcagcctcg actgtgcctt ctagttgcca gccatctgtt 1800
gtttgcccct cccccgtgcc ttccttgacc ctggaaggtg ccactcccac tgtcctttcc 1860
taataaaatg aggaaattgc atcgcattgt ctgagtaggt gtcattctat tctggggggt 1920
ggggtggggc aggacagcaa gggggaggat tgggaagaca atagcaggca tgctggggat 1980
gcggtgggct ctatggcttc tgaggcggaa agaaccagct ggggctctag ggggtatccc 2040
cggcgcgcca atttaacgcg aattaattct gtggaatgtg tgtcagttag ggtgtggaaa 2100
gtccccaggc tccccagcag gcagaagtat gcaaagcatg catctcaatt agtcagcaac 2160
caggtgtgga aagtccccag gctccccagc aggcagaagt atgcaaagca tgcatctcaa 2220
ttagtcagca accatagtcc cgcccctaac tccgcccatc ccgcccctaa ctccgcccag 2280
ttccgcccat tctccgcccc atggctgact aatttttttt atttatgcag aggccgaggc 2340
cgcctctgcc tctgagctat tccagaagta gtgaggaggc ttttttggag gcctaggctt 2400
ttgcaaaaag ctcccgggag cttgtatatc cattttcgga tctgatcagc acgtgttgac 2460
aattaatcat cggcatagta tatcggcata gtataatacg acaaggtgag gaactaaacc 2520
atggccaagt tgaccagtgc cgttccggtg ctcaccgcgc gcgacgtcgc cggagcggtc 2580
gagttctgga ccgaccggct cgggttctcc cgggacttcg tggaggacga cttcgccggt 2640
gtggtccggg acgacgtgac cctgttcatc agcgcggtcc aggaccaggt ggtgccggac 2700
aacaccctgg cctgggtgtg ggtgcgcggc ctggacgagc tgtacgccga gtggtcggag 2760
gtcgtgtcca cgaacttccg ggacgcctcc gggccggcca tgaccgagat cggcgagcag 2820
ccgtgggggc gggagttcgc cctgcgcgac ccggccggca actgcgtgca cttcgtggcc 2880
gaggagcagg actgacacgt gctacgagat ttcgattcca ccgccgcctt ctatgaaagg 2940
ttgggcttcg gaatcgtttt ccgggacgcc ggctggatga tcctccagcg cggggatctc 3000
atgctggagt tcttcgccca ccccaacttg tttattgcag cttataatgg ttacaaataa 3060
agcaatagca tcacaaattt cacaaataaa gcattttttt cactgcattc tagttgtggt 3120

ttgtccaaac tcatcaatgt atcttatcat gtctgtatac cgtcgacctc tagctagagc 3180
ttggcgtaat catggtcata gctgtttcct gtgtgaaatt gttatccgct cacaattcca 3240
cacaacatac gagccggaag cataaagtgt aaagcctggg gtgcctaatg agtgagctaa 3300
ctcacattaa ttgcgttgcg ctcactgccc gctttccagt cgggaaacct gtcgtgccag 3360
ctgcattaat gaatcggcca acgcgcgggg agaggcggtt tgcgtattgg gcgctcttcc 3420
gcttcctcgc tcactgactc gctgcgctcg gtcgttcggc tgcggcgagc ggtatcagct 3480
cactcaaagg cggtaatacg gttatccaca gaatcagggg ataacgcagg aaagaacatg 3540
tgagcaaaag gccagcaaaa ggccaggaac cgtaaaaagg ccgcgttgct ggcgtttttc 3600
cataggctcc gcccccctga cgagcatcac aaaaatcgac gctcaagtca gaggtggcga 3660
aacccgacag gactataaag ataccaggcg tttccccctg gaagctccct cgtgcgctct 3720
cctgttccga ccctgccgct taccggatac ctgtccgcct ttctcccttc gggaagcgtg 3780
gcgctttctc atagctcacg ctgtaggtat ctcagttcgg tgtaggtcgt tcgctccaag 3840
ctgggctgtg tgcacgaacc ccccgttcag cccgaccgct gcgccttatc cggtaactat 3900
cgtcttgagt ccaacccggt aagacacgac ttatcgccac tggcagcagc cactggtaac 3960
aggattagca gagcgaggta tgtaggcggt gctacagagt tcttgaagtg gtggcctaac 4020
tacggctaca ctagaaggac agtatttggt atctgcgctc tgctgaagcc agttaccttc 4080
ggaaaaagag ttggtagctc ttgatccggc aaacaaacca ccgctggtag cggtggtttt 4140
tttgtttgca agcagcagat tacgcgcaga aaaaaaggat ctcaagaaga tcctttgatc 4200
ttttctacgg ggtctgacgc tcagtggaac gaaaactcac gttaagggat tttggtcatg 4260
agattatcaa aaaggatctt cacctagatc cttttaaatt aaaaatgaag ttttaaatca 4320
atctaaagta tatatgagta aacttggtct gacagttacc aatgcttaat cagtgaggca 4380
cctatctcag cgatctgtct atttcgttca tccatagttg cctgactccc cgtcgtgtag 4440
ataactacga tacgggaggg cttaccatct ggccccagtg ctgcaatgat accgcgagac 4500
ccacgctcac cggctccaga tttatcagca ataaaccagc cagccggaag ggccgagcgc 4560
agaagtggtc ctgcaacttt atccgcctcc atccagtcta ttaattgttg ccgggaagct 4620
agagtaagta gttcgccagt taatagtttg cgcaacgttg ttgccattgc tacaggcatc 4680
gtggtgtcac gctcgtcgtt tggtatggct tcattcagct ccggttccca acgatcaagg 4740
cgagttacat gatcccccat gttgtgcaaa aaagcggtta gctccttcgg tcctccgatc 4800
gttgtcagaa gtaagttggc cgcagtgtta tcactcatgg ttatggcagc actgcataat 4860
tctcttactg tcatgccatc cgtaagatgc ttttctgtga ctggtgagta ctcaaccaag 4920
tcattctgag aatagtgtat gcggcgaccg agttgctctt gcccggcgtc aatacgggat 4980
aataccgcgc cacatagcag aactttaaaa gtgctcatca ttggaaaacg ttcttcgggg 5040
cgaaaactct caaggatctt accgctgttg agatccagtt cgatgtaacc cactcgtgca 5100
cccaactgat cttcagcatc ttttactttc accagcgttt ctgggtgagc aaaaacagga 5160
aggcaaaatg ccgcaaaaaa gggaataagg gcgacacgga aatgttgaat actcatactc 5220
ttcctttttc aatattattg aagcatttat cagggttatt gtctcatgag cggatacata 5280
tttgaatgta tttagaaaaa taaacaaata ggggttccgc gcacatttcc ccgaaaagtg 5340
ccacctgacg tctaagaaac cattattatc atgacattaa cctataaaaa taggcgtatc 5400
acgaggccct ttcgtc 5416

<210> 10
<211> 5001
<212> DNA
<213> Artificial Sequence

<220>
<223> Vector pMAT2

<400> 10
gacgtcaatt cgcgttaaat ttttgttaaa tcagctcatt ttttaaccaa taggccgaaa 60
tccccaaaat cccttataaa tcaaaagaat agaccgagat agggttgagt gttgttccag 120
tttggaacaa gagtccacta ttaaagaacg tggactccaa cgtcaaaggg cgaaaaaccg 180
tctatcaggg cgatggccca ctacgtgaac catcacccta atcaagtttt ttggggtcga 240
ggtgccgtaa agcactaaat cggaacccta aagggatgcc ccgatttaga gcttgacggg 300
gaaagccggc gaacgtggcg agaaaggaag ggaagaaagc gaaaggagcg ggcgctaggg 360
cgctggcaag tgtagcggtc acgctgcgcg taaccaccac acccgccgcg cttaatgcgc 420
cgctacaggg cgcgtttaat taaacggatt agaagccgcc gagcgggtga cagccctccg 480
aaggaagact ctcctccgtg cgtcctcgtc ttcaccggtc gcgttcctga aacgcagatg 540
tgcctcgcgc cgcactgctc cgaacaataa agattctaca atactagctt ttatggttat 600
gaagaggaaa aattggcagt aacctggccc cacaaacctt caaatgaacg aatcaaatta 660
acaaccatag gatgataatg cgattagttt tttagcctta tttctggggt aattaatcag 720
cgaagcgatg atttttgatc tattaacaga tatataaatg caaaaactgc ataaccactt 780
taactaatac tttcaacatt ttcggtttgt attacttctt attcaaatgt aataaaagta 840
tcaacaaaaa attgttaata tacctctata ctttaacgtc aaggagaaaa aaccccggat 900
cggactacta gcctagggcg caacgcaatt aatgtgagtt agctcactca ttaggcaccc 960
caggctttac actttatgct tccggctcgt atgttgtgtg gaattgtgag cggataacaa 1020

tttcacacta aggagggttt aaacatgcgg tttagtacga cactggcgac agcagcaaca 1080
gcacttttct tcacagcaag tcaggtaagc gcgagctccg gatcctcgag cgcggccgct 1140
tatccatacg acgtaccaga ctacgcagga ggtcatcacc atcatcacca tgtcgacgga 1200
tctggaggag gtgaggagaa gtcccggctg ttggagaagg agaaccgtga actggaaaag 1260
atcattgctg agaaagagga gcgtgtctct gaactgcgcc atcaactcca gtctgtagga 1320
ggttgttaat aagtcgacta atgaccgcgg atcatgtaat tagttatgtc acgcttacat 1380
tcacgccctc cccccacatc cgctctaacc gaaaaggaag gagttagaca acctgaagtc 1440
taggtcccta tttatttttt tatagttatg ttagtattaa gaacgttatt tatatttcaa 1500
atttttcttt ttttctgta cagacgcgtg tacgcatgta acattatact gaaaaccttg 1560
cttgagaagg ttttgggacg ctcgaaggct ttaatttgca agctgcgcgc gggtcctttt 1620
catcacgtgc tataaaaata attataattt aaatttttta atataaatat ataaattaaa 1680
aatagaaagt aaaaaaagaa attaaagaaa aaatagtttt tgttttccga agatgtaaaa 1740
gactctaggg ggatcgccaa caaatactac cttttatctt gctcttcctg ctctcaggta 1800
ttaatgccga attgtttcat cttgtctgtg tagaagacca cacacgaaaa tcctgtgatt 1860
ttacatttta cttatcgtta atcgaatgta tatctattta atctgctttt cttgtctaat 1920
aaatatatat gtaaagtacg ctttttgttg aaatttttta aacctttgtt tatttttttt 1980
tcttcattcc gtaactcttc taccttcttt atttactttc taaaatccaa atacaaaaca 2040
taaaaataaa taaacacaga gtaaattccc aaattattcc atcattaaaa gatacgaggc 2100
gcgtgtaagt tacaggcaag cgatccgtcc taagaaacca ttattatcat gacattaacc 2160
tataaaaata ggcgtatcac gaggcccttt cgtcttcaag aaattcggtc gaaaaaagaa 2220
aaggagaggg ccaagaggga gggcattggt gactattgag cacgtgagta tacgtgatta 2280
agcacacaaa ggcagcttgg agtatgtctg ttattaattt cacaggtagt tctggtccat 2340
tggtgaaagt ttgcggcttg cagagcacag aggccgcaga atgtgctcta gattccgatg 2400
ctgacttgct gggtattata tgtgtgccca atagaaagag aacaattgac ccggttattg 2460
caaggaaaat ttcaagtctt gtaaaagcat ataaaaatag ttcaggcact ccgaaatact 2520
tggttggcgt gtttcgtaat caacctaagg aggatgtttt ggctctggtc aatgattacg 2580
gcattgatat cgtccaactg cacggagatg agtcgtggca agaataccaa gagttcctcg 2640
gtttgccagt tattaaaaga ctcgtatttc caaaagactg caacatacta ctcagtgcag 2700
cttcacagaa acctcattcg tttattccct tgtttgattc agaagcaggt gggacaggtg 2760
aacttttgga ttggaactcg atttctgact gggttggaag gcaagagagc cccgagagct 2820
tacattttat gttagctggt ggactgacgc cagaaaatgt tggtgatgcg cttagattaa 2880
atggcgttat tggtgttgat gtaagcggag gtgtggagac aaatggtgta aaagactcta 2940
acaaaatagc aaatttcgtc aaaaatgcta agaaataggt tattactgag tagtatttat 3000
ttaagtattg tttgtgcact tgccccgaat ttcttatgat ttatgatttt tattattaaa 3060
taagttataa aaaaaataag tgtatacaaa ttttaaagtg actcttaggt tttaaaacga 3120
aaattcttat tcttgagtaa ctctttcctg taggtcaggt tgctttctca ggtatagcat 3180
gaggtcgctc acatgtgagc aaaaggccag caaaaggcca ggaaccgtaa aaaggccgcg 3240
ttgctggcgt ttttccatag gctccgcccc cctgacgagc atcacaaaaa tcgacgctca 3300
agtcagaggt ggcgaaaccc gacaggacta taaagatacc aggcgtttcc ccctggaagc 3360
tccctcgtgc gctctcctgt tccgaccctg ccgcttaccg gatacctgtc cgcctttctc 3420
ccttcgggaa gcgtggcgct ttctcatagc tcacgctgta ggtatctcag ttcggtgtag 3480
gtcgttcgct ccaagctggg ctgtgtgcac gaaccccccg ttcagcccga ccgctgcgcc 3540
ttatccggta actatcgtct tgagtccaac ccggtaagac acgacttatc gccactggca 3600
gcagccactg gtaacaggat tagcagagcg aggtatgtag gcggtgctac agagttcttg 3660
aagtggtggc ctaactacgg ctacactaga aggacagtat ttggtatctg cgctctgctg 3720
aagccagtta ccttcggaaa aagagttggt agctcttgat ccggcaaaca aaccaccgct 3780
ggtagcggtg gtttttttgt ttgcaagcag cagattacgc gcagaaaaaa aggatctcaa 3840
gaagatcctt tgatcttttc tacggggtct gacgctcagt ggaacgaaaa ctcacgttaa 3900
gggattttgg tcatgagatt atcaaaaagg atcttcacct agatcctttt aaattaaaaa 3960
tgaagtttta aatcaatcta aagtatatat gagtaaactt ggtctgacag ttaccaatgc 4020
ttaatcagtg aggcacctat ctcagcgatc tgtctatttc gttcatccat agttgcctga 4080
ctccccgtcg tgtagataac tacgatacgg gagggcttac catctggccc cagtgctgca 4140
atgataccgc gagacccacg ctcaccggct ccagatttat cagcaataaa ccagccagcc 4200
ggaagggccg agcgcagaag tggtcctgca actttatccg cctccatcca gtctattaat 4260
tgttgccggg aagctagagt aagtagttcg ccagttaata gtttgcgcaa cgttgttgcc 4320
attgctacag gcatcgtggt gtcacgctcg tcgtttggta tggcttcatt cagctccggt 4380
tcccaacgat caaggcgagt tacatgatcc cccatgttgt gcaaaaaagc ggttagctcc 4440
ttcggtcctc cgatcgttgt cagaagtaag ttggccgcag tgttatcact catggttatg 4500
gcagcactgc ataattctct tactgtcatg ccatccgtaa gatgcttttc tgtgactggt 4560
gagtactcaa ccaagtcatt ctgagaatag tgtatgcggc gaccgagttg ctcttgcccg 4620
gcgtcaatac gggataatac cgcgccacat agcagaactt taaaagtgct catcattgga 4680
aaacgttctt cggggcgaaa actctcaagg atcttaccgc tgttgagatc cagttcgatg 4740
taacccactc gtgcacccaa ctgatcttca gcatctttta ctttcaccag cgtttctggg 4800
tgagcaaaaa caggaaggca aaatgccgca aaaaagggaa taagggcgac acggaaatgt 4860
tgaatactca tactcttcct ttttcaatat tattgaagca tttatcaggg ttattgtctc 4920
atgagcggat acatatttga atgtatttag aaaaataaac aaataggggt tccgcgcaca 4980
tttccccgaa aagtgccacc t 5001

<210> 11
<211> 5724
<212> DNA
<213> Artificial Sequence

<220>
<223> Vector pMAT5

<400> 11
   gacgtcaatt cgcgttaaat ttttgttaaa tcagctcatt ttttaaccaa taggccgaaa 60
   tccccaaaat cccttataaa tcaaaagaat agaccgagat agggttgagt gttgttccag 120
   tttggaacaa gagtccacta ttaaagaacg tggactccaa cgtcaaaggg cgaaaaaccg 180
   tctatcaggg cgatggccca ctacgtgaac catcacccta atcaagtttt ttggggtcga 240
   ggtgccgtaa agcactaaat cggaacccta aagggatgcc ccgatttaga gcttgacggg 300
   gaaagccggc gaacgtggcg agaaaggaag ggaagaaagc gaaaggagcg ggcgctaggg 360
   cgctggcaag tgtagcggtc acgctgcgcg taaccaccac acccgccgcg cttaatgcgc 420
   cgctacaggg cgcgtttaat taaacggatt agaagccgcc gagcgggtga cagccctccg 480
   aaggaagact ctcctccgtg cgtcctcgtc ttcaccggtc gcgttcctga aacgcagatg 540
   tgcctcgcgc cgcactgctc cgaacaataa agattctaca atactagctt ttatggttat 600
   gaagaggaaa aattggcagt aacctggccc cacaaacctt caaatgaacg aatcaaatta 660
   acaaccatag gatgataatg cgattagttt tttagcctta tttctggggt aattaatcag 720
   cgaagcgatg atttttgatc tattaacaga tatataaatg caaaaactgc ataaccactt 780
   taactaatac tttcaacatt ttcggtttgt attacttctt attcaaatgt aataaaagta 840
   tcaacaaaaa attgttaata tacctctata ctttaacgtc aaggagaaaa aaccccggat 900
   cggactacta gcctagggcg caacgcaatt aatgtgagtt agctcactca ttaggcaccc 960
   caggctttac actttatgct tccggctcgt atgttgtgtg gaattgtgag cggataacaa 1020
   tttcacacta aggagggttt aaacatgcgg tttagtacga cactggcgac agcagcaaca 1080
   gcacttttct tcacagcaag tcaggtaagc gcgagctccg aggtgcagct ggtgcagagc 1140
   ggcggcggcg tggtgcagcc gggcggcagc ctgcgtctga gctgcgccgc gagcggctac 1200
   accttcacca actacggcat gaactggatt cgtcaggccc ccgggaaggg cctggagtgg 1260
   gtgggctgga tcaacaccta caccggcgag ccgacctacg cagctgactt caagcgtcgt 1320
   gtcaccttca gcctcgacac cagcaagagc acggcgtacc tgcaactgaa cagcctgagg 1380
   gccgaggaca ctgcagttta ctactgcgcg aaatacccgt actactacgg tcgtagccac 1440
   tggtacttcg acgtctgggg ccaagggacc cttgtcaccg tctcgagcgg cggtggcggt 1500
   tctggtggtg gtggctctgg tggcggcgga tccgatatcg tgatgaccca gagcccgagc 1560
   accctgagcg cgagtccggg tgagcgcgcg accatcacct gcagtgcgag ccagagcatc 1620
   agcacctacc tggcgtggta tcagcagaaa ccaggtcaag cgccgcaagt gctgatctac 1680
   gctgcgagca acctggcgtc cggagtgccg aaccgtttca gcggtagccg tagcgggacc 1740
   gatttcaccc tgaccatcag cagcttgcag ccggaagact tcgcggtgta ctactgccag 1800
   cagtactaca gcaccccgtg gaccttcggt ggtggtacca aagtggaaat caaagcggcc 1860
   gcttatccat acgacgtacc agactacgca ggaggtcatc accatcatca ccatgtcgac 1920
   ggatctggag gaggtgagga gaagtcccgg ctgttggaga aggagaaccg tgaactggaa 1980
   aagatcattg ctgagaaaga ggagcgtgtc tctgaactgc gccatcaact ccagtctgta 2040
   ggaggttgtt aataagtcga ctaatgaccg cggatcatgt aattagttat gtcacgctta 2100
   cattcacgcc ctccccccac atccgctcta accgaaaagg aaggagttag acaacctgaa 2160
   gtctaggtcc ctatttattt ttttatagtt atgttagtat taagaacgtt atttatattt 2220
   caaatttttc ttttttttct gtacagacgc gtgtacgcat gtaacattat actgaaaacc 2280
   ttgcttgaga aggttttggg acgctcgaag gctttaattt gcaagctgcg cgcgggtcct 2340
   tttcatcacg tgctataaaa ataattataa tttaaatttt ttaatataaa tatataaatt 2400
   aaaaatagaa agtaaaaaaa gaaattaaag aaaaaatagt ttttgttttc cgaagatgta 2460
   aaagactcta gggggatcgc caacaaatac taccttttat cttgctcttc ctgctctcag 2520
   gtattaatgc cgaattgttt catcttgtct gtgtagaaga ccacacacga aaatcctgtg 2580
   attttacatt ttacttatcg ttaatcgaat gtatatctat ttaatctgct tttcttgtct 2640
   aataaatata tatgtaaagt acgctttttg ttgaaatttt ttaaaccttt gtttattttt 2700
   ttttcttcat tccgtaactc ttctaccttc tttatttact ttctaaaatc caaatacaaa 2760
   acataaaaat aaataaacac agagtaaatt cccaaattat tccatcatta aaagatacga 2820
   ggcgcgtgta agttacaggc aagcgatccg tcctaagaaa ccattattat catgacatta 2880
   acctataaaa ataggcgtat cacgaggccc tttcgtcttc aagaaattcg gtcgaaaaaa 2940
   gaaaaggaga gggccaagag ggagggcatt ggtgactatt gagcacgtga gtatacgtga 3000
   ttaagcacac aaaggcagct tggagtatgt ctgttattaa tttcacaggt agttctggtc 3060
   cattggtgaa agtttgcggc ttgcagagca cagaggccgc agaatgtgct ctagattccg 3120
   atgctgactt gctgggtatt atatgtgtgc ccaatagaaa gagaacaatt gacccggtta 3180
   ttgcaaggaa aatttcaagt cttgtaaaag catataaaaa tagttcaggc actccgaaat 3240
   acttggttgg cgtgtttcgt aatcaaccta aggaggatgt tttggctctg gtcaatgatt 3300
   acggcattga tatcgtccaa ctgcacggag atgagtcgtg gcaagaatac caagagttcc 3360
   tcggtttgcc agttattaaa agactcgtat ttccaaaaga ctgcaacata ctactcagtg 3420
   cagcttcaca gaaacctcat tcgtttattc ccttgtttga ttcagaagca ggtgggacag 3480
   gtgaactttt ggattggaac tcgatttctg actgggttgg aaggcaagag agccccgaga 3540
   gcttacattt tatgttagct ggtggactga cgccagaaaa tgttggtgat gcgcttagat 3600
   taaatggcgt tattggtgtt gatgtaagcg gaggtgtgga gacaaatggt gtaaaagact 3660
   ctaacaaaat agcaaatttc gtcaaaaatg ctaagaaata ggttattact gagtagtatt 3720
   tatttaagta ttgtttgtgc acttgccccg aatttcttat gatttatgat ttttattatt 3780
   aaataagtta taaaaaaaat aagtgtatac aaattttaaa gtgactctta ggttttaaaa 3840
   cgaaaattct tattcttgag taactctttc ctgtaggtca ggttgctttc tcaggtatag 3900
   catgaggtcg ctcacatgtg agcaaaaggc cagcaaaagg ccaggaaccg taaaaaggcc 3960
   gcgttgctgg cgtttttcca taggctccgc ccccctgacg agcatcacaa aaatcgacgc 4020
   tcaagtcaga ggtggcgaaa cccgacagga ctataaagat accaggcgtt tccccctgga 4080
   agctccctcg tgcgctctcc tgttccgacc ctgccgctta ccggatacct gtccgccttt 4140
   ctcccttcgg gaagcgtggc gctttctcat agctcacgct gtaggtatct cagttcggtg 4200
   taggtcgttc gctccaagct gggctgtgtg cacgaacccc ccgttcagcc cgaccgctgc 4260
   gccttatccg gtaactatcg tcttgagtcc aacccggtaa gacacgactt atcgccactg 4320
   gcagcagcca ctggtaacag gattagcaga gcgaggtatg taggcggtgc tacagagttc 4380
   ttgaagtggt ggcctaacta cggctacact agaaggacag tatttggtat ctgcgctctg 4440
   ctgaagccag ttaccttcgg aaaaagagtt ggtagctctt gatccggcaa acaaaccacc 4500
   gctggtagcg gtggtttttt tgtttgcaag cagcagatta cgcgcagaaa aaaaggatct 4560
   caagaagatc ctttgatctt ttctacgggg tctgacgctc agtggaacga aaactcacgt 4620
   taagggattt tggtcatgag attatcaaaa aggatcttca cctagatcct tttaaattaa 4680
   aaatgaagtt ttaaatcaat ctaaagtata tatgagtaaa cttggtctga cagttaccaa 4740
   tgcttaatca gtgaggcacc tatctcagcg atctgtctat ttcgttcatc catagttgcc 4800
   tgactccccg tcgtgtagat aactacgata cgggagggct taccatctgg ccccagtgct 4860
   gcaatgatac cgcgagaccc acgctcaccg gctccagatt tatcagcaat aaaccagcca 4920
   gccggaaggg ccgagcgcag aagtggtcct gcaactttat ccgcctccat ccagtctatt 4980
   aattgttgcc gggaagctag agtaagtagt tcgccagtta atagtttgcg caacgttgtt 5040
   gccattgcta caggcatcgt ggtgtcacgc tcgtcgtttg gtatggcttc attcagctcc 5100
   ggttcccaac gatcaaggcg agttacatga tcccccatgt tgtgcaaaaa agcggttagc 5160
   tccttcggtc ctccgatcgt tgtcagaagt aagttggccg cagtgttatc actcatggtt 5220
   atggcagcac tgcataattc tcttactgtc atgccatccg taagatgctt ttctgtgact 5280
   ggtgagtact caaccaagtc attctgagaa tagtgtatgc ggcgaccgag ttgctcttgc 5340
   ccggcgtcaa tacgggataa taccgcgcca catagcagaa ctttaaaagt gctcatcatt 5400
   ggaaaacgtt cttcggggcg aaaactctca aggatcttac cgctgttgag atccagttcg 5460
   atgtaaccca ctcgtgcacc caactgatct tcagcatctt ttactttcac cagcgtttct 5520
   gggtgagcaa aaacaggaag gcaaaatgcc gcaaaaaagg gaataagggc gacacggaaa 5580
   tgttgaatac tcatactctt cctttttcaa tattattgaa gcatttatca gggttattgt 5640
   ctcatgagcg gatacatatt tgaatgtatt tagaaaaata aacaaatagg ggttccgcgc 5700
   acatttcccc gaaaagtgcc acct 5724

<210> 12
<211> 8847
<212> DNA
<213> Artificial Sequence

<220>
<223> Vector pMAT3

<400> 12
   gacgtccact caaccctatc tcggtctatt cttttgattt ataagggatt ttgccgattt 60
   cggcctattg gttaaaaaat gagctgattt aacaaaaatt taacgcgaat tttaacaaaa 120
   tattaacgtt tacaatttcc tgatgcggta ttttctcctt acgcatctgt gcggtatttc 180
   acaccgcata gggtaataac tgatataatt aaattgaagc tctaatttgt gagtttagta 240
   tacatgcatt tacttataat acagtttttt agttttgctg gccgcatctt ctcaaatatg 300
   cttcccagcc tgcttttctg taacgttcac cctctacctt agcatccctt ccctttgcaa 360
   atagtcctct tccaacaata ataatgtcag atcctgtaga gaccacatca tccacggttc 420
   tatactgttg acccaatgcg tctcccttgt catctaaacc cacaccgggt gtcataatca 480
   accaatcgta accttcatct cttccaccca tgtctctttg agcaataaag ccgataacaa 540
   aatctttgtc gctcttcgca atgtcaacag tacccttagt atattctcca gtagataggg 600
   agcccttgca tgacaattct gctaacatca aaaggcctct aggttccttt gttacttctt 660
   ctgccgcctg cttcaaaccg ctaacaatac ctgggcccac cacaccgtgt gcattcgtaa 720
   tgtctgccca ttctgctatt ctgtatacac ccgcagagta ctgcaatttg actgtattac 780
   caatgtcagc aaattttctg tcttcgaaga gtaaaaaatt gtacttggcg gataatgcct 840
   ttagcggctt aactgtgccc tccatggaaa aatcagtcaa gatatccaca tgagttttta 900
   gtaaacaaat tttgggacct aatgcttcaa ctaactccag taattccttg gtggtacgaa 960
   catccaatga agcacacaag tttgtttgct tttcgtgcat gatattaaat agcttggcag 1020
   caacaggact aggatgagta gcagcacgtt ccttatatgt agctttcgac atgatttatc 1080
   ttcgtttcct gcaggttttt gttctgtgca gttgggttaa gaatactggg caatttcatg 1140
   tttcttcaac actacatatg cgtatatata ccaatctaag tctgtgctcc ttccttcgtt 1200
   cttccttctg ttcggagatt accgaatcaa aaaaatttca aagaaaccga aatcaaaaaa 1260
   aagaataaaa aaaaaatgat gaattgaatt gaaaagctgt ggtatggtgc actctcagta 1320
   caatctgctc tgatgccgca tagttaagcc agccccgaca cccgccaaca cccgctgacg 1380
   cgccctgacg ggcttgtctg ctcccggcat ccgcttacag acaagctgtg accgtctccg 1440
   ggagctgcat gtgtcagagg ttttcaccgt catcaccgaa acgcgcgagg ctagccccac 1500
   acaccatagc ttcaaaatgt ttctactcct tttttactct tccagatttt ctcggactcc 1560
   gcgcatcgcc gtaccacttc aaaacaccca agcacagcat actaaatttt ccctctttct 1620
   tcctctaggg tgtcgttaat tacccgtact aaaggtttgg aaaagaaaaa agagaccgcc 1680
   tcgtttcttt ttcttcgtcg aaaaaggcaa taaaaatttt tatcacgttt ctttttcttg 1740
   aaattttttt ttttagtttt tttctctttc agtgacctcc attgatattt aagttaataa 1800
   acggtcttca atttctcaag tttcagtttc atttttcttg ttctattaca acttttttta 1860
   cttcttgttc attagaaaga aagcatagca atctaatcta aggggcggtg ttgacaatta 1920
   atcatcggca tagtatatcg gcatagtata atacgacaag gtgaggaact aaaccatggc 1980
   caagttgacc agtgccgttc cggtgctcac cgcgcgcgat gtcgccggag cggtcgagtt 2040
   ctggaccgac cggctcgggt tctcccggga cttcgtggag gacgacttcg ccggtgtggt 2100
   ccgggacgac gtgaccctgt tcatcagcgc ggtccaggac caggtggtgc cggacaacac 2160
   cctggcctgg gtgtgggtgc gcggcctgga cgagctgtac gccgagtggt cggaggtcgt 2220
   gtccacgaac ttccgggacg cctccgggcc ggccatgacc gagatcggcg agcagccgtg 2280
   ggggcgggag ttcgccctgc gcgacccggc cggcaactgc gtgcacttcg tggccgagga 2340
   gcaggactga cacgtccgac ggcggcccac gggtcccagg cctcggagat ccgtccccct 2400
   tttcctttgt cgatatcatg taattagtta tgtcacgctt acattcacgc cctcccccca 2460
   catccgctct aaccgaaaag gaaggagtta gacaacctga agtctaggtc cctatttatt 2520
   tttttatagt tatgttagta ttaagaacgt tatttatatt tcaaattttt cttttttttc 2580
   tgtacagacg cgtgtacgca tgtaacatta tactgaaaac cttgcttgag aaggttttgg 2640
   gacgctcgaa ggctttaatt tgcaagctga attcacggat tagaagccgc cgagcgggtg 2700
   acagccctcc gaaggaagac tctcctccgt gcgtcctcgt cttcaccggt cgcgttcctg 2760
   aaacgcagat gtgcctcgcg ccgcactgct ccgaacaata aagattctac aatactagct 2820
   tttatggtta tgaagaggaa aaattggcag taacctggcc ccacaaacct tcaaatgaac 2880
   gaatcaaatt aacaaccata ggatgataat gcgattagtt ttttagcctt atttctgggg 2940
   taattaatca gcgaagcgat gatttttgat ctattaacag atatataaat gcaaaaactg 3000
   cataaccact ttaactaata ctttcaacat tttcggtttg tattacttct tattcaaatg 3060
   taataaaagt atcaacaaaa aattgttaat atacctctat actttaacgt caaggagaaa 3120
   aaaccccgga tcggactact agcagctgta atacgactca ctatagggaa tattaagcta 3180
   attctacttc atacattttc aattaagttt aaaccatgac aatgcctcat cgctatatgt 3240
   ttttggcagt ctttacactt ctggcactaa ctagtgtggc ctcaggagcc acttctagat 3300
   tggaaggttt gcaatctgaa aaccacagat tgagaatgaa gattactgaa ttggacaagg 3360
   acttggaaga agttactatg caattgcaag acgttggtgg ttgtgcggcc gctgaacaaa 3420
   agttgatttc tgaagaagac ttgagctccg gtggtggttc tggtggtggt tccggttctg 3480
   gtggtggtgg ttccggtggt ggttccggat ccggtttgcc aactgatgag accatcattg 3540
   tcatcagaac accaacaaca gccactactg ccatgactac aactcagcca tggaacgaca 3600
   cttttacctc tacttctacc gaattgacca cagtcaccgg taccaatggt ttgccaactg 3660
   atgagaccat cattgtcatc agaacaccaa caacagccac tactgccatg actacaactc 3720
   agccatggaa cgacactttt acctctacat ccactgaaat caccaccgtc accggtacca 3780
   atggtttgcc aactgatgag accatcattg tcatcagaac accaacaaca gccactactg 3840
   ccatgactac acctcagcca tggaacgaca cttttacctc tacatccact gaaatgacca 3900
   ccgtcaccgg taccaacggt ttgccaactg atgaaaccat cattgtcatc agaacaccaa 3960
   caacagccac tactgccata actacaactg agccatggaa cagcactttt acctctacat 4020
   ccactgaaat gaccaccgtc accggtacca acggtttgcc aactgatgaa accatcattg 4080
   tcatcagaac accaacaaca gccactactg ccataactac aactcagcca tggaacgaca 4140
   cttttacctc tacatccact gaaatgacca ccgtcaccgg taccaacggt ttgccaactg 4200
   atgaaaccat cattgtcatc agaacaccaa caacagccac tactgccatg actacaactc 4260
   agccatggaa cgacactttt acctctacat ccactgaaat caccaccgtc accggtacca 4320
   ccggtttgcc aactgatgag accatcattg tcatcagaac accaacaaca gccactactg 4380
   ccatgactac aactcagcca tggaacgaca cttttacctc tacatccact gaaatgacca 4440
   ccgtcaccgg taccaacggc gttccaactg acgaaaccgt cattgtcatc agaactccaa 4500
   ctagtgaagg tctaatcagc accaccactg aaccatggac tggtactttc acctctacat 4560
   ccactgagat gaccaccgtc accggtacta acggtcaacc aactgacgaa accgtgattg 4620
   ttatcagaac tccaaccagt gaaggtttgg ttacaaccac cactgaacca tggactggta 4680
   cttttacttc tacatctact gaaatgacca ccattactgg aaccaacggc gttccaactg 4740
   acgaaaccgt cattgtcatc agaactccaa ccagtgaagg tctaatcagc accaccactg 4800
   aaccatggac tggtactttt acttctacat ctactgaaat gaccaccatt actggaacca 4860
   atggtcaacc aactgacgaa accgttattg ttatcagaac tccaactagt gaaggtctaa 4920
   tcagcactac aacggaacca tggaccggta ctttcacttc tacatctact gaaatgacgc 4980
   acgtcaccgg taccaacggc gttccaactg acgaaaccgt cattgtcatc agaactccaa 5040
   ccagtgaagg tctaatcagc accaccactg aaccatggac tggcactttc acttcgactt 5100
   ccactgaggt taccaccatc actggaacca acggtcaacc aactgacgaa actgtgattg 5160
   ttatcagaac tccaaccagt gaaggtctaa tcagcaccac cactgaacca tggactggta 5220
   ctttcacttc tacatctact gaaatgacca ccgtcaccgg tactaacggt caaccaactg 5280
   acgaaaccgt gattgttatc agaactccaa ccagtgaagg tttggttaca accaccactg 5340
   aaccatggac tggtactttt acttcgactt ccactgaaat gtctactgtc actggaacca 5400
   atggcttgcc aactgatgaa actgtcattg ttgtcaaaac tccaactact gccatctcat 5460
   ccagtttgtc atcatcatct tcaggacaaa tcaccagctc tatcacgtct tcgcgtccaa 5520
   ttattacccc attctatcct agcaatggaa cttctgtgat ttcttcctca gtaatttctt 5580
   cctcagtcac ttcttctcta ttcacttctt ctccagtcat ttcttcctca gtcatttctt 5640
   cttctacaac aacctccact tctatatttt ctgaatcatc taaatcatcc gtcattccaa 5700
   ccagtagttc cacctctggt tcttctgaga gcgaaacgag ttcagctggt tctgtctctt 5760
   cttcctcttt tatctcttct gaatcatcaa aatctcctac atattcttct tcatcattac 5820
   cacttgttac cagtgcgaca acaagccagg aaactgcttc ttcattacca cctgctacca 5880
   ctacaaaaac gagcgaacaa accactttgg ttaccgtgac atcctgcgag tctcatgtgt 5940
   gcactgaatc catctcccct gcgattgttt ccacagctac tgttactgtt agcggcgtca 6000
   caacagagta taccacatgg tgccctattt ctactacaga gacaacaaag caaaccaaag 6060
   ggacaacaga gcaaaccaca gaaacaacaa aacaaaccac ggtagttaca atttcttctt 6120
   gtgaatctga cgtatgctct aagactgctt ctccagccat tgtatctaca agcactgcta 6180
   ctattaacgg cgttactaca gaatacacaa catggtgtcc tatttccacc acagaatcga 6240
   ggcaacaaac aacgctagtt actgttactt cctgcgaatc tggtgtgtgt tccgaaactg 6300
   cttcacctgc cattgtttcg acggccacgg ctactgtgaa tgatgttgtt acggtctatc 6360
   ctacatggag gccacagact gcgaatgaag agtctgtcag ctctaaaatg aacagtgcta 6420
   ccggtgagac aacaaccaat actttagctg ctgaaacgac taccaatact gtagctgctg 6480
   agacgattac caatactgga gctgctgaga cgaaaacagt agtcacctct tcgctttcaa 6540
   gatctaatca cgctgaaaca cagacggctt ccgcgaccga tgtgattggt cacagcagta 6600
   gtgttgtttc tgtatccgaa actggcaaca ccaagagtct aacaagttcc gggttgagta 6660
   ctatgtcgca acagcctcgt agcacaccag caagcagcat ggtaggatat agtacagctt 6720
   ctttagaaat ttcaacgtat gctggcagtg ccaacagctt actggccggt agtggtttaa 6780
   gtgtcttcat tgcgtcctta ttgctggcaa ttatttaaaa gcttgttatt actgagtagt 6840
   atttatttaa gtattgtttg tgcacttgcc ccgaatttct tatgatttat gatttttatt 6900
   attaaataag ttataaaaaa aataagtgta tacaaatttt aaagtgactc ttaggtttta 6960
   aaacgaaaat tcttattctt gagtaactct ttcctgtagg tcaggttgct ttctcaggta 7020
   tagcatgagg tcgctcacat gtgagcaaaa ggccagcaaa aggccaggaa ccgtaaaaag 7080
   gccgcgttgc tggcgttttt ccataggctc cgcccccctg acgagcatca caaaaatcga 7140
   cgctcaagtc agaggtggcg aaacccgaca ggactataaa gataccaggc gtttccccct 7200
   ggaagctccc tcgtgcgctc tcctgttccg accctgccgc ttaccggata cctgtccgcc 7260
   tttctccctt cgggaagcgt ggcgctttct catagctcac gctgtaggta tctcagttcg 7320
   gtgtaggtcg ttcgctccaa gctgggctgt gtgcacgaac cccccgttca gcccgaccgc 7380
   tgcgccttat ccggtaacta tcgtcttgag tccaacccgg taagacacga cttatcgcca 7440
   ctggcagcag ccactggtaa caggattagc agagcgaggt atgtaggcgg tgctacagag 7500
   ttcttgaagt ggtggcctaa ctacggctac actagaagga cagtatttgg tatctgcgct 7560
   ctgctgaagc cagttacctt cggaaaaaga gttggtagct cttgatccgg caaacaaacc 7620
   accgctggta gcggtggttt ttttgtttgc aagcagcaga ttacgcgcag aaaaaaagga 7680
   tctcaagaag atcctttgat cttttctacg gggtctgacg ctcagtggaa cgaaaactca 7740
   cgttaaggga ttttggtcat gagattatca aaaaggatct tcacctagat ccttttaaat 7800
   taaaaatgaa gttttaaatc aatctaaagt atatatgagt aaacttggtc tgacagttac 7860
   caatgcttaa tcagtgaggc acctatctca gcgatctgtc tatttcgttc atccatagtt 7920
   gcctgactcc ccgtcgtgta gataactacg atacgggagg gcttaccatc tggccccagt 7980
   gctgcaatga taccgcgaga cccacgctca ccggctccag atttatcagc aataaaccag 8040
   ccagccggaa gggccgagcg cagaagtggt cctgcaactt tatccgcctc catccagtct 8100
   attaattgtt gccgggaagc tagagtaagt agttcgccag ttaatagttt gcgcaacgtt 8160
   gttgccattg ctacaggcat cgtggtgtca cgctcgtcgt ttggtatggc ttcattcagc 8220
   tccggttccc aacgatcaag gcgagttaca tgatccccca tgttgtgcaa aaaagcggtt 8280
   agctccttcg gtcctccgat cgttgtcaga agtaagttgg ccgcagtgtt atcactcatg 8340
   gttatggcag cactgcataa ttctcttact gtcatgccat ccgtaagatg cttttctgtg 8400
   actggtgagt actcaaccaa gtcattctga gaatagtgta tgcggcgacc gagttgctct 8460
   tgcccggcgt caatacggga taataccgcg ccacatagca gaactttaaa agtgctcatc 8520
   attggaaaac gttcttcggg gcgaaaactc tcaaggatct taccgctgtt gagatccagt 8580
   tcgatgtaac ccactcgtgc acccaactga tcttcagcat cttttacttt caccagcgtt 8640
   tctgggtgag caaaaacagg aaggcaaaat gccgcaaaaa agggaataag ggcgacacgg 8700
   aaatgttgaa tactcatact cttccttttt caatattatt gaagcattta tcagggttat 8760
   tgtctcatga gcggatacat atttgaatgt atttagaaaa ataaacaaat aggggttccg 8820
   cgcacatttc cccgaaaagt gccacct 8847

<210> 13
<211> 5749

<212> DNA
<213> Artificial Sequence

<220>
<223> Helper Vector pMAT17

<400> 13
gacgtccact caaccctatc tcggtctatt cttttgattt ataagggatt ttgccgattt 60
cggcctattg gttaaaaaat gagctgattt aacaaaaatt taacgcgaat tttaacaaaa 120
tattaacgtt tacaatttcc tgatgcggta ttttctcctt acgcatctgt gcggtatttc 180
acaccgcata gggtaataac tgatataatt aaattgaagc tctaatttgt gagtttagta 240
tacatgcatt tacttataat acagtttttt agttttgctg gccgcatctt ctcaaatatg 300
cttcccagcc tgcttttctg taacgttcac cctctacctt agcatccctt ccctttgcaa 360
atagtcctct tccaacaata ataatgtcag atcctgtaga gaccacatca tccacggttc 420
tatactgttg acccaatgcg tctcccttgt catctaaacc cacaccgggt gtcataatca 480
accaatcgta accttcatct cttccaccca tgtctctttg agcaataaag ccgataacaa 540
aatctttgtc gctcttcgca atgtcaacag tacccttagt atattctcca gtagataggg 600
agcccttgca tgacaattct gctaacatca aaaggcctct aggttccttt gttacttctt 660
ctgccgcctg cttcaaaccg ctaacaatac ctgggcccac cacaccgtgt gcattcgtaa 720
tgtctgccca ttctgctatt ctgtatacac ccgcagagta ctgcaatttg actgtattac 780
caatgtcagc aaattttctg tcttcgaaga gtaaaaaatt gtacttggcg gataatgcct 840
ttagcggctt aactgtgccc tccatggaaa aatcagtcaa gatatccaca tgagttttta 900
gtaaacaaat tttgggacct aatgcttcaa ctaactccag taattccttg gtggtacgaa 960
catccaatga agcacacaag tttgtttgct tttcgtgcat gatattaaat agcttggcag 1020
caacaggact aggatgagta gcagcacgtt ccttatatgt agctttcgac atgatttatc 1080
ttcgtttcct gcaggttttt gttctgtgca gttgggttaa gaatactggg caatttcatg 1140
tttcttcaac actacatatg cgtatatata ccaatctaag tctgtgctcc ttccttcgtt 1200
cttccttctg ttcggagatt accgaatcaa aaaaatttca aggaaaccga aatcaaaaaa 1260
aagaataaaa aaaaaatgat gaattgaatt gaaaagctgt ggtatggtgc actctcagta 1320
caatctgctc tgatgccgca tagttaagcc agccccgaca cccgccaaca cccgctgacg 1380
cgccctgacg ggcttgtctg ctcccggcat ccgcttacag acaagctgtg accgtctccg 1440
ggagctgcat gtgtcagagg ttttcaccgt catcaccgaa acgcgcgagg ctagccccac 1500
acaccatagc ttcaaaatgt ttctactcct tttttactct tccagatttt ctcggactcc 1560
gcgcatcgcc gtaccacttc aaaacaccca agcacagcat actaaatttt ccctctttct 1620
tcctctaggg tgtcgttaat tacccgtact aaaggtttgg aaaagaaaaa agagaccgcc 1680
tcgtttcttt ttcttcgtcg aaaaaggcaa taaaaatttt tatcacgttt ctttttcttg 1740
aaattttttt ttttagtttt tttctctttc agtgacctcc attgatattt aagttaataa 1800
acggtcttca atttctcaag tttcagtttc atttttcttg ttctattaca acttttttta 1860
cttcttgttc attagaaaga aagcatagca atctaatcta aggggcggtg ttgacaatta 1920
atcatcggca tagtatatcg gcatagtata atacgacaag gtgaggaact aaaccatggc 1980
caagttgacc agtgccgttc cggtgctcac cgcgcgcgat gtcgccggag cggtcgagtt 2040
ctggaccgac cggctcgggt tctcccggga cttcgtggag gacgacttcg ccggtgtggt 2100
ccgggacgac gtgaccctgt tcatcagcgc ggtccaggac caggtggtgc cggacaacac 2160
cctggcctgg gtgtgggtgc gcggcctgga cgagctgtac gccgagtggt cggaggtcgt 2220
gtccacgaac ttccgggacg cctccgggcc ggccatgacc gagatcggcg agcagccgtg 2280
ggggcgggag ttcgccctgc gcgacccggc cggcaactgc gtgcacttcg tggccgagga 2340
gcaggactga cacgtccgac ggcggcccac gggtcccagg cctcggagat ccgtccccct 2400
tttcctttgt cgatatcatg taattagtta tgtcacgctt acattcacgc cctcccccca 2460
catccgctct aaccgaaaag gaaggagtta gacaacctga agtctaggtc cctatttatt 2520
tttttatagt tatgttagta ttaagaacgt tatttatatt tcaaattttt cttttttttc 2580
tgtacagacg cgtgtacgca tgtaacatta tactgaaaac cttgcttgag aaggttttgg 2640
gacgctcgaa ggctttaatt tgcaagctga attcacggat tagaagccgc cgagcgggtg 2700
acagccctcc gaaggaagac tctcctccgt gcgtcctcgt cttcaccggt cgcgttcctg 2760
aaacgcagat gtgcctcgcg ccgcactgct ccgaacaata aagattctac aatactagct 2820
tttatggtta tgaagaggaa aaattggcag taacctggcc ccacaaacct tcaaatgaac 2880
gaatcaaatt aacaaccata ggatgataat gcgattagtt ttttagcctt atttctgggg 2940
taattaatca gcgaagcgat gatttttgat ctattaacag atatataaat gcaaaaactg 3000
cataaccact ttaactaata ctttcaacat tttcggtttg tattacttct tattcaaatg 3060
taataaaagt atcaacaaaa aattgttaat atacctctat actttaacgt caaggagaaa 3120
aaaccccgga tcggactact agcagctgta atacgactca ctatagggaa tattaagcta 3180
attctacttc atacattttc aattaagttt aaaccatgac aatgcctcat cgctatatgt 3240
tttggcagt ctttacactt ctggcactaa ctagtgtggc ctcaggagcc acttctagat 3300
tggaaggttt gcaatctgaa aaccacagat tgagaatgaa gattactgaa ttggacaagg 3360
acttggaaga agttactatg caattgcaag acgttggtgg ttgtgcggcc gctgaacaaa 3420
agttgatttc tgaagaagac ttgagctccg gtggtggttc tggtggtggt tccggttctg 3480
gtggtggtgg ttccggtggt ggttccggat cccaggaact gacaactata tgcgagcaaa 3540
tcccctcacc aactttagaa tcgacgccgt actctttgtc aacgactact atttggcca 3600
acgggaaggc aatgcaagga gtttttgaat attacaaatc agtaacgttt gtcagtaatt 3660

gcggttctca cccctcaaca actagcaaag gcagccccat aaacacacag tatgtttttt 3720
aagcttgtta ttactgagta gtatttattt aagtattgtt tgtgcacttg ccccgaattt 3780
cttatgattt atgattttta ttattaaata agttataaaa aaaataagtg tatacaaatt 3840
ttaaagtgac tcttaggttt taaaacgaaa attcttattc ttgagtaact ctttcctgta 3900
ggtcaggttg ctttctcagg tatagcatga ggtcgctcac atgtgagcaa aaggccagca 3960
aaaggccagg aaccgtaaaa aggccgcgtt gctggcgttt ttccataggc tccgcccccc 4020
tgacgagcat cacaaaaatc gacgctcaag tcagaggtgg cgaaacccga caggactata 4080
aagataccag gcgtttcccc ctggaagctc cctcgtgcgc tctcctgttc cgaccctgcc 4140
gcttaccgga tacctgtccg cctttctccc ttcgggaagc gtggcgcttt ctcatagctc 4200
acgctgtagg tatctcagtt cggtgtaggt cgttcgctcc aagctgggct gtgtgcacga 4260
accccccgtt cagcccgacc gctgcgcctt atccggtaac tatcgtcttg agtccaaccc 4320
ggtaagacac gacttatcgc cactggcagc agccactggt aacaggatta gcagagcgag 4380
gtatgtaggc ggtgctacag agttcttgaa gtggtggcct aactacggct acactagaag 4440
gacagtattt ggtatctgcg ctctgctgaa gccagttacc ttcggaaaaa gagttggtag 4500
ctcttgatcc ggcaaacaaa ccaccgctgg tagcggtggt ttttttgttt gcaagcagca 4560
gattacgcgc agaaaaaaag gatctcaaga agatcctttg atcttttcta cggggtctga 4620
cgctcagtgg aacgaaaact cacgttaagg gattttggtc atgagattat caaaaaggat 4680
cttcacctag atccttttaa attaaaaatg aagttttaaa tcaatctaaa gtatatatga 4740
gtaaacttgg tctgacagtt accaatgctt aatcagtgag gcacctatct cagcgatctg 4800
tctatttcgt tcatccatag ttgcctgact ccccgtcgtg tagataacta cgatacggga 4860
gggcttacca tctggcccca gtgctgcaat gataccgcga gacccacgct caccggctcc 4920
agatttatca gcaataaacc agccagccgg aagggccgag cgcagaagtg gtcctgcaac 4980
tttatccgcc tccatccagt ctattaattg ttgccgggaa gctagagtaa gtagttcgcc 5040
agttaatagt ttgcgcaacg ttgttgccat tgctacaggc atcgtggtgt cacgctcgtc 5100
gtttggtatg gcttcattca gctccggttc ccaacgatca aggcgagtta catgatcccc 5160
catgttgtgc aaaaaagcgg ttagctcctt cggtcctccg atcgttgtca gaagtaagtt 5220
ggccgcagtg ttatcactca tggttatggc agcactgcat aattctctta ctgtcatgcc 5280
atccgtaaga tgcttttctg tgactggtga gtactcaacc aagtcattct gagaatagtg 5340
tatgcggcga ccgagttgct cttgcccggc gtcaatacgg gataataccg cgccacatag 5400
cagaacttta aaagtgctca tcattggaaa acgttcttcg gggcgaaaac tctcaaggat 5460
cttaccgctg ttgagatcca gttcgatgta acccactcgt gcacccaact gatcttcagc 5520
atcttttact ttcaccagcg tttctgggtg agcaaaaaca ggaaggcaaa atgccgcaaa 5580
aaagggaata agggcgacac ggaaatgttg aatactcata ctcttccttt ttcaatatta 5640
ttgaagcatt tatcagggtt attgtctcat gagcggatac atatttgaat gtatttagaa 5700
aaataaacaa ataggggttc cgcgcacatt tccccgaaaa gtgccacct 5749

<210> 14
<211> 6793
<212> DNA
<213> Artificial Sequence

<220>
<223> Vector pMAT8

<400> 14
   gacgtccact caaccctatc tcggtctatt cttttgattt ataagggatt ttgccgattt 60
   cggcctattg gttaaaaaat gagctgattt aacaaaaatt taacgcgaat tttaacaaaa 120
   tattaacgtt tacaatttcc tgatgcggta ttttctcctt acgcatctgt gcggtatttc 180
   acaccgcata gggtaataac tgatataatt aaattgaagc tctaatttgt gagtttagta 240
   tacatgcatt tacttataat acagtttttt agttttgctg gccgcatctt ctcaaatatg 300
   cttcccagcc tgcttttctg taacgttcac cctctacctt agcatccctt ccctttgcaa 360
   atagtcctct tccaacaata ataatgtcag atcctgtaga gaccacatca tccacggttc 420
   tatactgttg acccaatgcg tctcccttgt catctaaacc cacaccgggt gtcataatca 480
   accaatcgta accttcatct cttccaccca tgtctctttg agcaataaag ccgataacaa 540
   aatctttgtc gctcttcgca atgtcaacag tacccttagt atattctcca gtagataggg 600
   agcccttgca tgacaattct gctaacatca aaaggcctct aggttccttt gttacttctt 660
   ctgccgcctg cttcaaaccg ctaacaatac ctgggcccac cacaccgtgt gcattcgtaa 720
   tgtctgccca ttctgctatt ctgtatacac ccgcagagta ctgcaatttg actgtattac 780
   caatgtcagc aaattttctg tcttcgaaga gtaaaaaatt gtacttggcg gataatgcct 840
   ttagcggctt aactgtgccc tccatggaaa aatcagtcaa gatatccaca tgagttttta 900
   gtaaacaaat tttgggacct aatgcttcaa ctaactccag taattccttg gtggtacgaa 960
   catccaatga agcacacaag tttgtttgct tttcgtgcat gatattaaat agcttggcag 1020
   caacaggact aggatgagta gcagcacgtt ccttatatgt agctttcgac atgatttatc 1080
   ttcgtttcct gcaggttttt gttctgtgca gttgggttaa gaatactggg caatttcatg 1140
   tttcttcaac actacatatg cgtatatata ccaatctaag tctgtgctcc ttccttcgtt 1200
   cttccttctg ttcggagatt accgaatcaa aaaaatttca aagaaaccga aatcaaaaaa 1260
   aagaataaaa aaaaaatgat gaattgaatt gaaaagctgt ggtatggtgc actctcagta 1320
   caatctgctc tgatgccgca tagttaagcc agccccgaca cccgccaaca cccgctgacg 1380
   cgccctgacg ggcttgtctg ctcccggcat ccgcttacag acaagctgtg accgtctccg 1440
   ggagctgcat gtgtcagagg ttttcaccgt catcaccgaa acgcgcgagg ctagccccac 1500
   acaccatagc ttcaaaatgt ttctactcct tttttactct tccagatttt ctcggactcc 1560
   gcgcatcgcc gtaccacttc aaaacaccca agcacagcat actaaatttt ccctctttct 1620
   tcctctaggg tgtcgttaat tacccgtact aaaggtttgg aaaagaaaaa agagaccgcc 1680
   tcgtttcttt ttcttcgtcg aaaaaggcaa taaaaatttt tatcacgttt ctttttcttg 1740
   aaattttttt ttttagtttt tttctctttc agtgacctcc attgatattt aagttaataa 1800
   acggtcttca atttctcaag tttcagtttc atttttcttg ttctattaca acttttttta 1860
   cttcttgttc attagaaaga aagcatagca atctaatcta aggggcggtg ttgacaatta 1920
   atcatcggca tagtatatcg gcatagtata atacgacaag gtgaggaact aaaccatggc 1980
   caagttgacc agtgccgttc cggtgctcac cgcgcgcgat gtcgccggag cggtcgagtt 2040
   ctggaccgac cggctcgggt tctcccggga cttcgtggag gacgacttcg ccggtgtggt 2100
   ccgggacgac gtgaccctgt tcatcagcgc ggtccaggac caggtggtgc cggacaacac 2160
   cctggcctgg gtgtgggtgc gcggcctgga cgagctgtac gccgagtggt cggaggtcgt 2220
   gtccacgaac ttccgggacg cctccgggcc ggccatgacc gagatcggcg agcagccgtg 2280
   ggggcgggag ttcgccctgc gcgacccggc cggcaactgc gtgcacttcg tggccgagga 2340
   gcaggactga cacgtccgac ggcggcccac gggtcccagg cctcggagat ccgtccccct 2400
   tttcctttgt cgatatcatg taattagtta tgtcacgctt acattcacgc cctcccccca 2460
   catccgctct aaccgaaaag gaaggagtta gacaacctga agtctaggtc cctatttatt 2520
   tttttatagt tatgttagta ttaagaacgt tatttatatt tcaaattttt cttttttttc 2580
   tgtacagacg cgtgtacgca tgtaacatta tactgaaaac cttgcttgag aaggttttgg 2640
   gacgctcgaa ggctttaatt tgcaagctga attcacggat tagaagccgc cgagcgggtg 2700
   acagccctcc gaaggaagac tctcctccgt gcgtcctcgt cttcaccggt cgcgttcctg 2760
   aaacgcagat gtgcctcgcg ccgcactgct ccgaacaata aagattctac aatactagct 2820
   tttatggtta tgaagaggaa aaattggcag taacctggcc ccacaaacct tcaaatgaac 2880
   gaatcaaatt aacaaccata ggatgataat gcgattagtt ttttagcctt atttctgggg 2940
   taattaatca gcgaagcgat gatttttgat ctattaacag atatataaat gcaaaaactg 3000
   cataaccact ttaactaata ctttcaacat tttcggtttg tattacttct tattcaaatg 3060
   taataaaagt atcaacaaaa aattgttaat atacctctat actttaacgt caaggagaaa 3120
   aaaccccgga tcggactact agcagctgta atacgactca ctatagggaa tattaagcta 3180
   attctacttc atacattttc aattaagttt aaaccatgac aatgcctcat cgctatatgt 3240
   ttttggcagt ctttacactt ctggcactaa ctagtgtggc ctcaggagcc acttctagat 3300
   tggaaggttt gcaatctgaa aaccacagat tgagaatgaa gattactgaa ttggacaagg 3360
   acttggaaga agttactatg caattgcaag acgttggtgg ttgtgcggcc gctgaacaaa 3420
   agttgatttc tgaagaagac ttgagctccg gtggtggttc tggtggtggt tccggttctg 3480
   gtggtggtgg ttccggtggt ggttccggat cctcaagttt gtcatcatca tcttcaggac 3540
   aaatcaccag ctctatcacg tcttcgcgtc caattattac cccattctat cctagcaatg 3600
   gaacttctgt gatttcttcc tcagtaattt cttcctcagt cacttcttct ctattcactt 3660
   cttctccagt catttcttcc tcagtcattt cttcttctac aacaacctcc acttctatat 3720
   tttctgaatc atctaaatca tccgtcattc caaccagtag ttccacctct ggttcttctg 3780
   agagcgaaac gagttcagct ggttctgtct cttcttcctc ttttatctct tctgaatcat 3840
   caaaatctcc tacatattct tcttcatcat taccacttgt taccagtgcg acaacaagcc 3900
   aggaaactgc ttcttcatta ccacctgcta ccactacaaa aacgagcgaa caaaccactt 3960
   tggttaccgt gacatcctgc gagtctcatg tgtgcactga atccatctcc cctgcgattg 4020
   tttccacagc tactgttact gttagcggcg tcacaacaga gtataccaca tggtgcccta 4080
   tttctactac agagacaaca aagcaaacca aagggacaac agagcaaacc acagaaacaa 4140
   caaaacaaac cacggtagtt acaatttctt cttgtgaatc tgacgtatgc tctaagactg 4200
   cttctccagc cattgtatct acaagcactg ctactattaa cggcgttact acagaataca 4260
   caacatggtg tcctatttcc accacagaat cgaggcaaca aacaacgcta gttactgtta 4320
   cttcctgcga atctggtgtg tgttccgaaa ctgcttcacc tgccattgtt tcgacggcca 4380
   cggctactgt gaatgatgtt gttacggtct atcctacatg gaggccacag actgcgaatg 4440
   aagagtctgt cagctctaaa atgaacagtg ctaccggtga gacaacaacc aatactttag 4500
   ctgctgaaac gactaccaat actgtagctg ctgagacgat taccaatact ggagctgctg 4560
   ccatttctca aatcactgac ggtcaaatcc aagctactac cactgctacc accgaagcta 4620
   ccaccactgc tgccccatct tccaccgttg aaactgtttc tccatccagc accgaaacta 4680
   tctctcaaca aactgaaaat ggtgctgcta aggccgctgt cggtatgggt gccggtgctc 4740
   tagctgctgc tgctatgttg ttataagctt gttattactg agtagtattt atttaagtat 4800
   tgtttgtgca cttgccccga atttcttatg atttatgatt tttattatta aataagttat 4860
   aaaaaaaata agtgtataca aattttaaag tgactcttag gttttaaaac gaaaattctt 4920
   attcttgagt aactctttcc tgtaggtcag gttgctttct caggtatagc atgaggtcgc 4980
   tcacatgtga gcaaaaggcc agcaaaaggc caggaaccgt aaaaaggccg cgttgctggc 5040
   gtttttccat aggctccgcc cccctgacga gcatcacaaa aatcgacgct caagtcagag 5100
   gtggcgaaac ccgacaggac tataaagata ccaggcgttt ccccctggaa gctccctcgt 5160
   gcgctctcct gttccgaccc tgccgcttac cggatacctg tccgcctttc tcccttcggg 5220
   aagcgtggcg ctttctcata gctcacgctg taggtatctc agttcggtgt aggtcgttcg 5280
   ctccaagctg ggctgtgtgc acgaaccccc cgttcagccc gaccgctgcg ccttatccgg 5340
   taactatcgt cttgagtcca acccggtaag acacgactta tcgccactgg cagcagccac 5400
   tggtaacagg attagcagag cgaggtatgt aggcggtgct acagagttct tgaagtggtg 5460
   gcctaactac ggctacacta gaaggacagt atttggtatc tgcgctctgc tgaagccagt 5520
   taccttcgga aaaagagttg gtagctcttg atccggcaaa caaaccaccg ctggtagcgg 5580
   tggttttttt gtttgcaagc agcagattac gcgcagaaaa aaaggatctc aagaagatcc 5640
   tttgatcttt tctacggggt ctgacgctca gtggaacgaa aactcacgtt aagggatttt 5700
   ggtcatgaga ttatcaaaaa ggatcttcac ctagatcctt ttaaattaaa aatgaagttt 5760
   taaatcaatc taaagtatat atgagtaaac ttggtctgac agttaccaat gcttaatcag 5820
   tgaggcacct atctcagcga tctgtctatt tcgttcatcc atagttgcct gactccccgt 5880
   cgtgtagata actacgatac gggagggctt accatctggc cccagtgctg caatgatacc 5940
   gcgagaccca cgctcaccgg ctccagattt atcagcaata aaccagccag ccggaagggc 6000
   cgagcgcaga agtggtcctg caactttatc cgcctccatc cagtctatta attgttgccg 6060
   ggaagctaga gtaagtagtt cgccagttaa tagtttgcgc aacgttgttg ccattgctac 6120
   aggcatcgtg gtgtcacgct cgtcgtttgg tatggcttca ttcagctccg gttcccaacg 6180
   atcaaggcga gttacatgat cccccatgtt gtgcaaaaaa gcggttagct ccttcggtcc 6240
   tccgatcgtt gtcagaagta agttggccgc agtgttatca ctcatggtta tggcagcact 6300
   gcataattct cttactgtca tgccatccgt aagatgcttt tctgtgactg gtgagtactc 6360
   aaccaagtca ttctgagaat agtgtatgcg gcgaccgagt tgctcttgcc cggcgtcaat 6420
   acgggataat accgcgccac atagcagaac tttaaaagtg ctcatcattg gaaaacgttc 6480
   ttcggggcga aaactctcaa ggatcttacc gctgttgaga tccagttcga tgtaacccac 6540
   tcgtgcaccc aactgatctt cagcatcttt tactttcacc agcgtttctg ggtgagcaaa 6600
   aacaggaagg caaaatgccg caaaaaaggg aataagggcg acacggaaat gttgaatact 6660
   catactcttc ctttttcaat attattgaag catttatcag ggttattgtc tcatgagcgg 6720
   atacatattt gaatgtattt agaaaaataa acaaataggg gttccgcgca catttccccg 6780
   aaaagtgcca cct 6793

<210> 15
<211> 5520
<212> DNA
<213> Artificial Sequence

<220>
<223> Vector pMAT4

<400> 15
gacgtcaatt cgcgttaaat ttttgttaaa tcagctcatt ttttaaccaa taggccgaaa 60
tccccaaaat cccttataaa tcaaaagaat agaccgagat agggttgagt gttgttccag 120
tttggaacaa gagtccacta ttaaagaacg tggactccaa cgtcaaaggg cgaaaaaccg 180
tctatcaggg cgatggccca ctacgtgaac catcacccta atcaagtttt ttggggtcga 240
ggtgccgtaa agcactaaat cggaacccta aagggatgcc ccgatttaga gcttgacggg 300
gaaagccggc gaacgtggcg agaaaggaag ggaagaaagc gaaaggagcg ggcgctaggg 360
cgctggcaag tgtagcggtc acgctgcgcg taaccaccac acccgccgcg cttaatgcgc 420
cgctacaggg cgcgtttaat taagcagcac catggcctga aataacctct gaaagaggaa 480
cttggttagg taccttctga ggcggaaaga accagctgtg gaatgtgtgt cagttagggt 540
gtggaaagtc cccaggctcc ccagcaggca gaagtatgca aagcatgcat ctcaattagt 600
cagcaaccag gtgtggaaag tccccaggct ccccagcagg cagaagtatg caaagcatgc 660
atctcaatta gtcagcaacc atagtcccgc ccctaactcc gcccatcccg cccctaactc 720
cgcccagttc cgcccattct ccgccccatg gctgactaat tttttttatt tatgcagagg 780
ccgaggccgc ctcggcctct gagctattcc agaagtagtg aggaggcttt tttggaggcc 840
taggcttttg caaaaagctt gattcttctg acacaacagt ctcgaactta aggctagagc 900
cacagatcta ctgggcaggt aagtatcaag gtgagttcta atccgtactt caatatagca 960
atgagcagtt aagcgtatta ctgaaagttc caaagagaag gtttttttag gctaagataa 1020
tggggctctt tacatttcca caacatataa gtaagattag atatggatat gtatatggat 1080
atgtatatgg tggtaatgcc atgtaatatg attattaaac ttctttgcgt ccatccaaaa 1140
aaaaagtaag aatttttgaa aattcgtttc tgataggcac ctattggtct tactgacatc 1200
cactttgcct ttctctccac aggtgtccac cggtgcgcaa cgcaattaat gtgagttagc 1260
tcactcatta ggcaccccag gctttacact ttatgcttcc ggctcgtatg ttgtgtggaa 1320
ttgtgagcgg ataacaattt cacactaagg agggtttaaa ccatgctgat cttctggaca 1380
atcacacttt tcctgctggg agcagccaaa ggatcctcga gcgcggccgc ttatccatac 1440
gacgtaccag actacgcagg aggtcatcac catcatcacc atgtcgacgg atctggagga 1500
ggtgaggaga agtcccggct gttggagaag gagaaccgtg aactggaaaa gatcattgct 1560
gagaaagagg agcgtgtctc tgaactgcgc catcaactcc agtctgtagg aggttgttaa 1620
taagtcgact aatgagaatt cgacatgata agatacattg atgagtttgg acaaaccaca 1680
actagaatgc agtgaaaaaa atgctttatt tgtgaaattt gtgatgctat tgctttattt 1740
gtaaccatta taagctgcaa taaacaagtt aacaacaaca attgcattca ttttatgttt 1800
caggttcagg gggagatgtg ggaggttttt taaagcaagt aaaacctcta caaatgtggt 1860

aaaccgcgga tcatgtaatt agttatgtca cgcttacatt cacgccctcc ccccacatcc 1920
gctctaaccg aaaaggaagg agttagacaa cctgaagtct aggtccctat ttattttttt 1980
atagttatgt tagtattaag aacgttattt atatttcaaa tttttctttt ttttctgtac 2040
agacgcgtgt acgcatgtaa cattatactg aaaaccttgc ttgagaaggt tttgggacgc 2100
tcgaaggctt taatttgcaa gctgcgcgcg ggtccttttc atcacgtgct ataaaaataa 2160
ttataattta aattttttaa tataaatata taaattaaaa atagaaagta aaaaaagaaa 2220
ttaaagaaaa aatagttttt gttttccgaa gatgtaaaag actctagggg gatcgccaac 2280
aaatactacc ttttatcttg ctcttcctgc tctcaggtat taatgccgaa ttgtttcatc 2340
ttgtctgtgt agaagaccac acacgaaaat cctgtgattt tacattttac ttatcgttaa 2400
tcgaatgtat atctatttaa tctgcttttc ttgtctaata aatatatatg taaagtacgc 2460
tttttgttga aattttttaa acctttgttt attttttttt cttcattccg taactcttct 2520
accttcttta tttactttct aaaatccaaa tacaaaacat aaaaataaat aaacacagag 2580
taaattccca aattattcca tcattaaaag atacgaggcg cgtgtaagtt acaggcaagc 2640
gatccgtcct aagaaaccat tattatcatg acattaacct ataaaaatag gcgtatcacg 2700
aggccctttc gtcttcaaga aattcggtcg aaaaaagaaa aggagagggc caagagggag 2760
ggcattggtg actattgagc acgtgagtat acgtgattaa gcacacaaag gcagcttgga 2820
gtatgtctgt tattaatttc acaggtagtt ctggtccatt ggtgaaagtt tgcggcttgc 2880
agagcacaga ggccgcagaa tgtgctctag attccgatgc tgacttgctg ggtattatat 2940
gtgtgcccaa tagaaagaga acaattgacc cggttattgc aaggaaaatt tcaagtcttg 3000
taaaagcata taaaaatagt tcaggcactc cgaaatactt ggttggcgtg tttcgtaatc 3060
aacctaagga ggatgttttg gctctggtca atgattacgg cattgatatc gtccaactgc 3120
acggagatga gtcgtggcaa gaataccaag agttcctcgg tttgccagtt attaaaagac 3180
tcgtatttcc aaaagactgc aacatactac tcagtgcagc ttcacagaaa cctcattcgt 3240
ttattccctt gtttgattca gaagcaggtg ggacaggtga acttttggat tggaactcga 3300
tttctgactg ggttggaagg caagagagcc ccgagagctt acattttatg ttagctggtg 3360
gactgacgcc agaaaatgtt ggtgatgcgc ttagattaaa tggcgttatt ggtgttgatg 3420
taagcggagg tgtggagaca aatggtgtaa aagactctaa caaaatagca aatttcgtca 3480
aaaatgctaa gaaataggtt attactgagt agtatttatt taagtattgt ttgtgcactt 3540
gccccgaatt tcttatgatt tatgattttt attattaaat aagttataaa aaaaataagt 3600
gtatacaaat tttaaagtga ctcttaggtt ttaaaacgaa aattcttatt cttgagtaac 3660
tctttcctgt aggtcaggtt gctttctcag gtatagcatg aggtcgctca catgtgagca 3720
aaaggccagc aaaaggccag gaaccgtaaa aaggccgcgt tgctggcgtt tttccatagg 3780
ctccgccccc ctgacgagca tcacaaaaat cgacgctcaa gtcagaggtg gcgaaacccg 3840
acaggactat aaagatacca ggcgtttccc cctggaagct ccctcgtgcg ctctcctgtt 3900
ccgaccctgc cgcttaccgg atacctgtcc gcctttctcc cttcgggaag cgtggcgctt 3960
tctcatagct cacgctgtag gtatctcagt tcggtgtagg tcgttcgctc caagctgggc 4020
tgtgtgcacg aaccccccgt tcagcccgac cgctgcgcct tatccggtaa ctatcgtctt 4080
gagtccaacc cggtaagaca cgacttatcg ccactggcag cagccactgg taacaggatt 4140
agcagagcga ggtatgtagg cggtgctaca gagttcttga agtggtggcc taactacggc 4200
tacactagaa ggacagtatt tggtatctgc gctctgctga agccagttac cttcggaaaa 4260
agagttggta gctcttgatc cggcaaacaa accaccgctg gtagcggtgg tttttttgtt 4320
tgcaagcagc agattacgcg cagaaaaaaa ggatctcaag aagatccttt gatcttttct 4380
acggggtctg acgctcagtg gaacgaaaac tcacgttaag ggattttggt catgagatta 4440
tcaaaaagga tcttcaccta gatcctttta aattaaaaat gaagttttaa atcaatctaa 4500
agtatatatg agtaaacttg gtctgacagt taccaatgct taatcagtga ggcacctatc 4560
tcagcgatct gtctatttcg ttcatccata gttgcctgac tccccgtcgt gtagataact 4620
acgatacggg agggcttacc atctggcccc agtgctgcaa tgataccgcg agacccacgc 4680
tcaccggctc cagatttatc agcaataaac cagccagccg gaagggccga gcgcagaagt 4740
ggtcctgcaa ctttatccgc ctccatccag tctattaatt gttgccggga agctagagta 4800
agtagttcgc cagttaatag tttgcgcaac gttgttgcca ttgctacagg catcgtggtg 4860
tcacgctcgt cgtttggtat ggcttcattc agctccggtt cccaacgatc aaggcgagtt 4920
acatgatccc ccatgttgtg caaaaaagcg gttagctcct tcggtcctcc gatcgttgtc 4980
agaagtaagt tggccgcagt gttatcactc atggttatgg cagcactgca taattctctt 5040
actgtcatgc catccgtaag atgcttttct gtgactggtg agtactcaac caagtcattc 5100
tgagaatagt gtatgcggcg accgagttgc tcttgcccgg cgtcaatacg ggataatacc 5160
gcgccacata gcagaacttt aaaagtgctc atcattggaa aacgttcttc ggggcgaaaa 5220
ctctcaagga tcttaccgct gttgagatcc agttcgatgt aacccactcg tgcacccaac 5280
tgatcttcag catcttttac tttcaccagc gtttctgggt gagcaaaaac aggaaggcaa 5340
aatgccgcaa aaaagggaat aagggcgaca cggaaatgtt gaatactcat actcttcctt 5400
tttcaatatt attgaagcat ttatcagggt tattgtctca tgagcggata catatttgaa 5460
tgtatttaga aaaataaaca aataggggtt ccgcgcacat ttccccgaaa agtgccacct 5520

<210> 16
<211> 6258
<212> DNA

<213> Artificial Sequence

<220>
<223> Vector pMAT6

<400> 16
gacgtcaatt cgcgttaaat ttttgttaaa tcagctcatt ttttaaccaa taggccgaaa 60
tccccaaaat cccttataaa tcaaaagaat agaccgagat agggttgagt gttgttccag 120
tttggaacaa gagtccacta ttaaagaacg tggactccaa cgtcaaaggg cgaaaaaccg 180
tctatcaggg cgatggccca ctacgtgaac catcacccta atcaagtttt ttggggtcga 240
ggtgccgtaa agcactaaat cggaacccta aagggatgcc ccgatttaga gcttgacggg 300
gaaagccggc gaacgtggcg agaaaggaag ggaagaaagc gaaaggagcg ggcgctaggg 360
cgctggcaag tgtagcggtc acgctgcgcg taaccaccac acccgccgcg cttaatgcgc 420
cgctacaggg cgcgtttaat taagcagcac catggcctga aataacctct gaaagaggaa 480
cttggttagg taccttctga ggcggaaaga accagctgtg gaatgtgtgt cagttagggt 540
gtggaaagtc cccaggctcc ccagcaggca gaagtatgca aagcatgcat ctcaattagt 600
cagcaaccag gtgtggaaag tccccaggct ccccagcagg cagaagtatg caaagcatgc 660
atctcaatta gtcagcaacc atagtcccgc ccctaactcc gcccatcccg cccctaactc 720
cgcccagttc cgcccattct ccgccccatg gctgactaat tttttttatt tatgcagagg 780
ccgaggccgc ctcggcctct gagctattcc agaagtagtg aggaggcttt tttggaggcc 840
taggcttttg caaaaagctt gattcttctg acacaacagt ctcgaactta aggctagagc 900
cacagatcta ctgggcaggt aagtatcaag gtgagttcta atccgtactt caatatagca 960
atgagcagtt aagcgtatta ctgaaagttc caaagagaag gtttttttag gctaagataa 1020
tggggctctt tacatttcca caacatataa gtaagattag atatggatat gtatatggat 1080
atgtatatgg tggtaatgcc atgtaatatg attattaaac ttctttgcgt ccatccaaaa 1140
aaaaagtaag aatttttgaa aattcgtttc tgataggcac ctattggtct tactgacatc 1200
cactttgcct ttctctccac aggtgtccac cggtgcgcaa cgcaattaat gtgagttagc 1260
tcactcatta ggcaccccag gctttacact ttatgcttcc ggctcgtatg ttgtgtggaa 1320
ttgtgagcgg ataacaattt cacactaagg agggtttaaa ccatgctgat cttctggaca 1380
atcacacttt tcctgctggg agcagccaaa ggatcctcga gctccgaggt gcagctggtg 1440
cagagcggcg gcggcgtggt gcagccgggc ggcagcctgc gtctgagctg cgccgcgagc 1500
ggctacacct tcaccaacta cggcatgaac tggattcgtc aggcccccgg gaagggcctg 1560
gagtgggtgg gctggatcaa cacctacacc ggcgagccga cctacgcagc tgacttcaag 1620
cgtcgtgtca ccttcagcct cgacaccagc aagagcacgg cgtacctgca actgaacagc 1680
ctgagggccg aggacactgc agtttactac tgcgcgaaat acccgtacta ctacggtcgt 1740
agccactggt acttcgacgt ctggggccaa gggacccttg tcaccgtctc gagcggcggt 1800
ggcggttctg gtggtggtgg ctctggtggc ggcggatccg atatcgtgat gacccagagc 1860
ccgagcaccc tgagcgcgag tccgggtgag cgcgcgacca tcacctgcag tgcgagccag 1920
agcatcagca cctacctggc gtggtatcag cagaaaccag gtcaagcgcc gcaagtgctg 1980
atctacgctg cgagcaacct ggcgtccgga gtgccgaacc gtttcagcgg tagccgtagc 2040
gggaccgatt tcaccctgac catcagcagc ttgcagccgg aagacttcgc ggtgtactac 2100
tgccagcagt actacagcac cccgtggacc ttcggtggtg gtaccaaagt ggaaatcaaa 2160
gcggccgctt atccatacga cgtaccagac tacgcaggag gtcatcacca tcatcaccat 2220
gtcgacggat ctggaggagg tgaggagaag tcccggctgt tggagaagga gaaccgtgaa 2280
ctggaaaaga tcattgctga gaaagaggag cgtgtctctg aactgcgcca tcaactccag 2340
tctgtaggag gttgttaata agtcgactaa tgagaattcg acatgataag atacattgat 2400
gagtttggac aaaccacaac tagaatgcag tgaaaaaaat gctttatttg tgaaatttgt 2460
gatgctattg ctttatttgt aaccattata agctgcaata aacaagttaa caacaacaat 2520
tgcattcatt ttatgtttca ggttcagggg gagatgtggg aggtttttta aagcaagtaa 2580
aacctctaca aatgtggtaa accgcggatc atgtaattag ttatgtcacg cttacattca 2640
cgccctcccc ccacatccgc tctaaccgaa aaggaaggag ttagacaacc tgaagtctag 2700
gtccctattt atttttttat agttatgtta gtattaagaa cgttatttat atttcaaatt 2760
tttctttttt ttctgtacag acgcgtgtac gcatgtaaca ttatactgaa aaccttgctt 2820
gagaaggttt tgggacgctc gaaggcttta atttgcaagc tgcgcgcggg tcctttcat 2880
cacgtgctat aaaaataatt ataatttaaa ttttttaata taaatatata aattaaaaat 2940
agaaagtaaa aaaagaaatt aaagaaaaaa tagtttttgt tttccgaaga tgtaaaagac 3000
tctaggggga tcgccaacaa atactacctt ttatcttgct cttcctgctc tcaggtatta 3060
atgccgaatt gtttcatctt gtctgtgtag aagaccacac acgaaaatcc tgtgatttta 3120
cattttactt atcgttaatc gaatgtatat ctatttaatc tgcttttctt gtctaataaa 3180
tatatatgta aagtacgctt tttgttgaaa ttttttaaac ctttgtttat ttttttttct 3240
tcattccgta actcttctac cttctttatt tactttctaa aatccaaata caaaacataa 3300
aaataaataa acacagagta aattcccaaa ttattccatc attaaaagat acgaggcgcg 3360
tgtaagttac aggcaagcga tccgtcctaa gaaaccatta ttatcatgac attaacctat 3420
aaaaataggc gtatcacgag gccctttcgt cttcaagaaa ttcggtcgaa aaaagaaaag 3480
gagagggcca agagggaggg cattggtgac tattgagcac gtgagtatac gtgattaagc 3540
acacaaaggc agcttggagt atgtctgtta ttaatttcac aggtagttct ggtccattgg 3600
tgaaagtttg cggcttgcag agcacagagg ccgcagaatg tgctctagat tccgatgctg 3660
acttgctggg tattatatgt gtgcccaata gaaagagaac aattgacccg gttattgcaa 3720

ggaaaatttc aagtcttgta aaagcatata aaaatagttc aggcactccg aaatacttgg 3780
ttggcgtgtt tcgtaatcaa cctaaggagg atgttttggc tctggtcaat gattacggca 3840
ttgatatcgt ccaactgcac ggagatgagt cgtggcaaga ataccaagag ttcctcggtt 3900
tgccagttat taaaagactc gtatttccaa aagactgcaa catactactc agtgcagctt 3960
cacagaaacc tcattcgttt attcccttgt ttgattcaga agcaggtggg acaggtgaac 4020
ttttggattg gaactcgatt tctgactggg ttggaaggca agagagcccc gagagcttac 4080
attttatgtt agctggtgga ctgacgccag aaaatgttgg tgatgcgctt agattaaatg 4140
gcgttattgg tgttgatgta agcggaggtg tggagacaaa tggtgtaaaa gactctaaca 4200
aaatagcaaa tttcgtcaaa aatgctaaga aataggttat tactgagtag tatttattta 4260
agtattgttt gtgcacttgc ccgaatttct ttatgattta tgatttttat tattaaataa 4320
gttataaaaa aaataagtgt atacaaattt taaagtgact cttaggtttt aaaacgaaaa 4380
ttcttattct tgagtaactc tttcctgtag gtcaggttgc tttctcaggt atagcatgag 4440
gtcgctcaca tgtgagcaaa aggccagcaa aaggccagga accgtaaaaa ggccgcgttg 4500
ctggcgtttt tccataggct ccgcccccct gacgagcatc acaaaaatcg acgctcaagt 4560
cagaggtggc gaaacccgac aggactataa agataccagg cgtttccccc tggaagctcc 4620
ctcgtgcgct ctcctgttcc gaccctgccg cttaccggat acctgtccgc ctttctccct 4680
tcgggaagcg tggcgctttc tcatagctca cgctgtaggt atctcagttc ggtgtaggtc 4740
gttcgctcca agctgggctg tgtgcacgaa ccccccgttc agcccgaccg ctgcgcctta 4800
tccggtaact atcgtcttga gtccaacccg gtaagacacg acttatcgcc actggcagca 4860
gccactggta acaggattag cagagcgagg tatgtaggcg gtgctacaga gttcttgaag 4920
tggtggccta actacggcta cactagaagg acagtatttg gtatctgcgc tctgctgaag 4980
ccagttacct tcggaaaaag agttggtagc tcttgatccg gcaaacaaac caccgctggt 5040
agcggtggtt tttttgtttg caagcagcag attacgcgca gaaaaaaagg atctcaagaa 5100
gatcctttga tcttttctac ggggtctgac gctcagtgga acgaaaactc acgttaaggg 5160
attttggtca tgagattatc aaaaaggatc ttcacctaga tccttttaaa ttaaaaatga 5220
agttttaaat caatctaaag tatatatgag taaacttggt ctgacagtta ccaatgctta 5280
atcagtgagg cacctatctc agcgatctgt ctatttcgtt catccatagt tgcctgactc 5340
cccgtcgtgt agataactac gatacgggag ggcttaccat ctggccccag tgctgcaatg 5400
ataccgcgag acccacgctc accggctcca gatttatcag caataaacca gccagccgga 5460
agggccgagc gcagaagtgg tcctgcaact ttatccgcct ccatccagtc tattaattgt 5520
tgccgggaag ctagagtaag tagttcgcca gttaatagtt tgcgcaacgt tgttgccatt 5580
gctacaggca tcgtggtgtc acgctcgtcg tttggtatgg cttcattcag ctccggttcc 5640
caacgatcaa ggcgagttac atgatccccc atgttgtgca aaaaagcggt tagctccttc 5700
ggtcctccga tcgttgtcag aagtaagttg gccgcagtgt tatcactcat ggttatggca 5760
gcactgcata attctcttac tgtcatgcca tccgtaagat gcttttctgt gactggtgag 5820
tactcaacca agtcattctg agaatagtgt atgcggcgac cgagttgctc ttgcccggcg 5880
tcaatacggg ataataccgc gccacatagc agaactttaa aagtgctcat cattggaaaa 5940
cgttcttcgg ggcgaaaact ctcaaggatc ttaccgctgt tgagatccag ttcgatgtaa 6000
cccactcgtg cacccaactg atcttcagca tcttttactt tcaccagcgt ttctgggtga 6060
gcaaaaacag gaaggcaaaa tgccgcaaaa aagggaataa gggcgacacg gaaatgttga 6120
atactcatac tcttcctttt tcaatattat tgaagcattt atcagggtta ttgtctcatg 6180
agcggataca tatttgaatg tatttagaaa aataaacaaa taggggttcc gcgcacattt 6240
ccccgaaaag tgccacct 6258

<210> 17
<211> 3818
<212> DNA
<213> Artificial Sequence

<220>
<223> Vector pMAL1

<400> 17
acatgttctt tcctgcgtta tcccctgatt ctgtggataa ccgtattacc gcctttgagt 60
gagctgatac cgctcgccgc agccgaacga ccgagcgcag cgagtcagtg agcgaggaag 120
cggaagagcg cccaatacgc aaaccgcctc tccccgcgcg ttggccgatt cattaatgca 180
gctggcacga caggtttccc gactggaaag cgggcagtga gcgcaacgca attaatgtga 240
gttagctcac tcattaggca ccccaggctt tacactttat gcttccggct cgtatgttgt 300
gtggaattgt gagcggataa caatttcaca caggaaacag ctatgaccat gattacgcca 360
agcgcgttta actttagtaa ggaggaatta aaaaatgaaa tacctgctgc cgaccgcagc 420
cgcgggtttg ctgttactgg cggcccagcc ggctatggcg atgaaagcta ctaaactggt 480
actgggcaac ccgtatgttg gctttgaaat gggttacgac tggttaggtc gtatgccgta 540
caaaggcagc gttgaaaacg gtgcatacaa agctcagggc gttcaactga ccgctaaact 600
gggttaccca atcactgacg acctggacat ctacactcgt ctgggtggca tggtatggcg 660
tgcagacact aaatccaacg tttatggtaa aaaccacgac accggcgttt ctccggtctt 720
cgctggcggt gttgagtacg cgatcactcc tgaaatcgct acccgtctgg aataccagtg 780

gacgaacaac atcggtgacg cacacaccat cggcactcgt ccggacggag gtacatcccg 840
cctggagggc ctacagtcag aaaaccatcg cctgcgaatg aagatcacag agctggataa 900
agacttggaa gaagtcacca tgcagctgca agacgttggc ggttgctaat gagcgcgctc 960
actggccgtc gttttacaac gtcgtgactg ggaaaaccct ggcgttaccc aacttaatcg 1020
ccttgcagca catccccctt tcgccagctg gcgtaatagc gaagaggccc gcaccgatcg 1080
cccttcccaa cagttgcgca gcctgaatgg cgaatgggac gcgccctgta gcggcgcatt 1140
aagcgcggcg ggtgtggtgg ttacgcgcag cgtgaccgct acacttgcca gcgccctagc 1200
gcccgctcct ttcgctttct tcccttcctt tctcgccacg ttcgccggct ttccccgtca 1260
agctctaaat cgggggctcc ctttagggtt ccgatttagt gctttacggc acctcgaccc 1320
caaaaaactt gattagggtg atggttcacg tagtgggcca tcgccctgat agacggtttt 1380
tcgccctttg acgttggagt ccacgttctt taatagtgga ctcttgttcc aaactggaac 1440
aacactcaac cctatctcgg tctattcttt tgatttataa gggattttgc cgatttcggc 1500
ctattggtta aaaaatgagc tgatttaaca aaaatttaac gcgaatttta acaaaatatt 1560
aacgcttaca atttaggtgg cacttttcgg ggaaatgtgc gcggaacccc tatttgttta 1620
tttttctaaa tacattcaaa tatgtatccg ctcatgagac aataaccctg ataaatgctt 1680
caataatatt gaaaaaggaa gagtatgagt attcaacatt tccgtgtcgc ccttattccc 1740
ttttttgcgg cattttgcct tcctgttttt gctcacccag aaacgctggt gaaagtaaaa 1800
gatgctgaag atcagttggg tgcacgagtg ggttacatcg aactggatct caacagcggt 1860
aagatccttg agagttttcg ccccgaagaa cgttttccaa tgatgagcac ttttcgaccg 1920
aataaatacc tgtgacggaa gatcacttcg cagaataaat aaatcctggt gtccctgttg 1980
ataccgggaa gccctgggcc aacttttggc gaaaatgaga cgttgatcgg cacgtaagag 2040
gttccaactt tcaccataat gaaataagat cactaccggg cgtatttttt gagttgtcga 2100
gattttcagg agctaaggaa gctaaaatgg agaaaaaaat cactggatat accaccgttg 2160
atatatccca atggcatcgt aaagaacatt ttgaggcatt tcagtcagtt gctcaatgta 2220
cctataacca gaccgttcag ctggatatta cggccttttt aaagaccgta aagaaaaata 2280
agcacaagtt ttatccggcc tttattcaca ttcttgcccg cctgatgaat gctcatccgg 2340
aattacgtat ggcaatgaaa gacggtgagc tggtgatatg ggatagtgtt cacccttgtt 2400
acaccgtttt ccatgagcaa actgaaacgt tttcatcgct ctggagtgaa taccacgacg 2460
atttccggca gtttctacac atatattcgc aagatgtggc gtgttacggt gaaaacctgg 2520
cctatttccc taaagggttt attgagaata tgtttttcgt ctcagccaat ccctgggtga 2580
gtttcaccag ttttgattta aacgtggcca atatggacaa cttcttcgcc cccgttttca 2640
ccatgggcaa atattatacg caaggcgaca aggtgctgat gccgctggcg attcaggttc 2700
atcatgccgt ttgtgatggc ttccatgtcg gcagaatgct taatgaatta caacagtact 2760
gcgatgagtg gcagggcggg gcgtaatttt tttaaggcag ttattggtgc ccttaaacgc 2820
ctggttgcta cgcctgaata agtgataata agcggatgaa tggcagaaat tcgaaagcaa 2880
attcgacccg gtcgtcggtt cagggcaggg tcgttaaata gccgcttatg tctattgctg 2940
gtttaccggt ttattgacta ccggaagcag tgtgaccgtg tgcttctcaa atgcctgagg 3000
ccagtttgct caggctctcc ccgtggaggt aataattgac gatatgatcc tttttttctg 3060
atcaaaaagg atctaggtga agatcctttt tgataatctc atgaccaaaa tcccttaacg 3120
tgagttttcg ttccactgag cgtcagaccc cgtagaaaag atcaaaggat cttcttgaga 3180
tccttttttt ctgcgcgtaa tctgctgctt gcaaacaaaa aaaccaccgc taccagcggt 3240
ggtttgtttg ccggatcaag agctaccaac tctttttccg aaggtaactg gcttcagcag 3300
agcgcagata ccaaatactg tccttctagt gtagccgtag ttaggccacc acttcaagaa 3360
ctctgtagca ccgcctacat acctcgctct gctaatcctg ttaccagtgg ctgctgccag 3420
tggcgataag tcgtgtctta ccgggttgga ctcaagacga tagttaccgg ataaggcgca 3480
gcggtcgggc tgaacggggg gttcgtgcac acagcccagc ttggagcgaa cgacctacac 3540
cgaactgaga tacctacagc gtgagctatg agaaagcgcc acgcttcccg aagggagaaa 3600
ggcggacagg tatccggtaa gcggcagggt cggaacagga gagcgcacga gggagcttcc 3660
agggggaaac gcctggtatc tttatagtcc tgtcgggttt cgccacctct gacttgagcg 3720
tcgatttttg tgatgctcgt caggggggcg gagcctatgg aaaaacgcca gcaacgcggc 3780
ctttttacgg ttcctggcct tttgctggcc ttttgctc 3818

<210> 18
<211> 3878
<212> DNA
<213> Artificial Sequence

<220>
<223> Vector pMAL2

<400> 18
acatgttctt tcctgcgtta tcccctgatt ctgtggataa ccgtattacc gcctttgagt 60
gagctgatac cgctcgccgc agccgaacga ccgagcgcag cgagtcagtg agcgaggaag 120
cggaagagcg cccaatacgc aaaccgcctc tccccgcgcg ttggccgatt cattaatgca 180
gctggcacga caggtttccc gactggaaag cgggcagtga gcgcaacgca attaatgtga 240
gttagctcac tcattaggca ccccaggctt tacactttat gcttccggct cgtatgttgt 300
gtggaattgt gagcggataa caatttcaca caggaaacag ctatgaccat gattacgcca 360
agcgcgttta actttagtaa ggagggttta aacaatgaaa aagtctttag tcctcaaagc 420

ctccgtagcc gttgctaccc tcgttccgat gctaagcttc gctatgaaag ctactaaact 480
ggtactgggc aacccgtatg ttggctttga aatgggttac gactggttag gtcgtatgcc 540
gtacaaaggc agcgttgaaa acggtgcata caaagctcag ggcgttcaac tgaccgctaa 600
actgggttac ccaatcactg acgacctgga catctacact cgtctgggtg gcatggtatg 660
gcgtgcagac actaaatcca acgtttatgg taaaaaccac gacaccggcg tttctccggt 720
cttcgctggc ggtgttgagt acgcgatcac tcctgaaatc gctacccgtc tggaatacca 780
gtggacgaac aacatcggtg acgcacacac catcggcact cgtccggacg gaggtggcgg 840
atccggtgag cagaagctga tcagcgagga ggacctgagc tccggtggag gcacatcccg 900
cctggagggc ctacagtcag aaaaccatcg cctgcgaatg aagatcacag agctggataa 960
agacttggaa gaagtcacca tgcagctgca agacgttggc ggttgctaat gagcgcgctc 1020
actggccgtc gttttacaac gtcgtgactg ggaaaaccct ggcgttaccc aacttaatcg 1080
ccttgcagca catccccctt tcgccagctg gcgtaatagc gaagaggccc gcaccgatcg 1140
cccttcccaa cagttgcgca gcctgaatgg cgaatgggac gcgccctgta gcggcgcatt 1200
aagcgcggcg ggtgtggtgg ttacgcgcag cgtgaccgct acacttgcca gcgccctagc 1260
gcccgctcct ttcgctttct tcccttcctt tctcgccacg ttcgccggct ttccccgtca 1320
agctctaaat cgggggctcc ctttagggtt ccgatttagt gctttacggc acctcgaccc 1380
caaaaaactt gattagggtg atggttcacg tagtgggcca tcgccctgat agacggtttt 1440
tcgccctttg acgttggagt ccacgttctt taatagtgga ctcttgttcc aaactggaac 1500
aacactcaac cctatctcgg tctattcttt tgatttataa gggattttgc cgatttcggc 1560
ctattggtta aaaaatgagc tgatttaaca aaaatttaac gcgaatttta acaaaatatt 1620
aacgcttaca atttaggtgg cacttttcgg ggaaatgtgc gcggaacccc tatttgttta 1680
tttttctaaa tacattcaaa tatgtatccg ctcatgagac aataaccctg ataaatgctt 1740
caataatatt gaaaaaggaa gagtatgagt attcaacatt tccgtgtcgc ccttattccc 1800
ttttttgcgg cattttgcct tcctgttttt gctcacccag aaacgctggt gaaagtaaaa 1860
gatgctgaag atcagttggg tgcacgagtg ggttacatcg aactggatct caacagcggt 1920
aagatccttg agagttttcg ccccgaagaa cgttttccaa tgatgagcac ttttcgaccg 1980
aataaatacc tgtgacggaa gatcacttcg cagaataaat aaatcctggt gtccctgttg 2040
ataccgggaa gccctgggcc aacttttggc gaaaatgaga cgttgatcgg cacgtaagag 2100
gttccaactt tcaccataat gaaataagat cactaccggg cgtatttttt gagttgtcga 2160
gattttcagg agctaaggaa gctaaaatgg agaaaaaaat cactggatat accaccgttg 2220
atatatccca atggcatcgt aaagaacatt ttgaggcatt tcagtcagtt gctcaatgta 2280
cctataacca gaccgttcag ctggatatta cggccttttt aaagaccgta aagaaaaata 2340
agcacaagtt ttatccggcc tttattcaca ttcttgcccg cctgatgaat gctcatccgg 2400
aattacgtat ggcaatgaaa gacggtgagc tggtgatatg ggatagtgtt cacccttgtt 2460
acaccgtttt ccatgagcaa actgaaacgt tttcatcgct ctggagtgaa taccacgacg 2520
atttccggca gtttctacac atatattcgc aagatgtggc gtgttacggt gaaaacctgg 2580
cctatttccc taaagggttt attgagaata tgtttttcgt ctcagccaat ccctgggtga 2640
gtttcaccag ttttgattta aacgtggcca atatggacaa cttcttcgcc cccgttttca 2700
ccatgggcaa atattatacg caaggcgaca aggtgctgat gccgctggcg attcaggttc 2760
atcatgccgt ttgtgatggc ttccatgtcg gcagaatgct taatgaatta caacagtact 2820
gcgatgagtg gcagggcggg gcgtaatttt tttaaggcag ttattggtgc ccttaaacgc 2880
ctggttgcta cgcctgaata agtgataata agcggatgaa tggcagaaat tcgaaagcaa 2940
attcgacccg gtcgtcggtt cagggcaggg tcgttaaata gccgcttatg tctattgctg 3000
gtttaccggt ttattgacta ccggaagcag tgtgaccgtg tgcttctcaa atgcctgagg 3060
ccagtttgct caggctctcc ccgtggaggt aataattgac gatatgatcc tttttttctg 3120
atcaaaaagg atctaggtga agatcctttt tgataatctc atgaccaaaa tcccttaacg 3180
tgagttttcg ttccactgag cgtcagaccc cgtagaaaag atcaaaggat cttcttgaga 3240
tccttttttt ctgcgcgtaa tctgctgctt gcaaacaaaa aaaccaccgc taccagcggt 3300
ggtttgtttg ccggatcaag agctaccaac tctttttccg aaggtaactg gcttcagcag 3360
agcgcagata ccaaatactg tccttctagt gtagccgtag ttaggccacc acttcaagaa 3420
ctctgtagca ccgcctacat acctcgctct gctaatcctg ttaccagtgg ctgctgccag 3480
tggcgataag tcgtgtctta ccgggttgga ctcaagacga tagttaccgg ataaggcgca 3540
gcggtcgggc tgaacggggg gttcgtgcac acagcccagc ttggagcgaa cgacctacac 3600
cgaactgaga tacctacagc gtgagctatg agaaagcgcc acgcttcccg aagggagaaa 3660
ggcggacagg tatccggtaa gcggcagggt cggaacagga gagcgcacga gggagcttcc 3720
agggggaaac gcctggtatc tttatagtcc tgtcgggttt cgccacctct gacttgagcg 3780
tcgatttttg tgatgctcgt caggggggcg gagcctatgg aaaaacgcca gcaacgcggc 3840
ctttttacgg ttcctggcct tttgctggcc ttttgctc 3878

<210> 19
<211> 38
<212> PRT
<213> Artificial Sequence

<220>
<223> GR1

<400> 19

Glu Glu Lys Ser Arg Leu Leu Glu Lys Glu Asn Arg Glu Leu Glu Lys
1               5                   10                  15
Ile Ile Ala Glu Lys Glu Glu Arg Val Ser Glu Leu Arg His Gln Leu
            20                  25                  30
Gln Ser Val Gly Gly Cys
        35

<210> 20
<211> 38
<212> PRT
<213> Artificial Sequence

<220>
<223> GR2

<400> 20
Thr Ser Arg Leu Glu Gly Leu Gln Ser Glu Asn His Arg Leu Arg Met
1               5                   10                  15
Lys Ile Thr Glu Leu Asp Lys Asp Leu Glu Glu Val Thr Met Gln Leu
            20                  25                  30
Gln Asp Val Gly Gly Cys
        35